# EUROPEAN PATENT SPECIFICATION

(11) **EP 1 644 321 B1**
(45) Date of publication and mention of the grant of the patent: **09.02.2011**
(21) Application number: 04739820.1
(22) Date of filing: 11.06.2004
(51) Int. Cl.: C07C 235/52, C07D 333/08, C07D 209/04, C07D 241/40, C07D 213/02, A61K 31/166, A61K 31/381, A61K 31/404, A61K 31/498, A61K 31/435, A61P 5/00

(54) **NEW BENZAMIDES AS PPATY MODULATORS**
NEUE BENZAMIDE ALS PPATY-MODULATOREN
NOUVEAUX BENZAMIDES EN TANT QUE MODULATEURS DE PPATY

(30) Priority: 13.06.2003 ES 200301461
(43) Date of publication of application: 12.04.2006
(73) Proprietor: LABORATORIOS S.A.L.V.A.T., S.A., 08950 Esplugues de Llobregat (Barcelona) (ES)
(72) Inventor: FERNANDEZ SERRAT, Anna, F-06800 Cagnes sur Mer (FR); SERRA COMAS, Carmen, E-08902 L'Hospitalet De Llobregat (ES); BALSA LOPEZ, Dolors, E-08912 Badalona (ES); LLEBARIA SOLDEVILA, Amadeu, E-08195 Sant Cugat Del Vallès (ES); FARRERONS GALLEMI, Carles, E-08034 Mataro (ES); MIQUEL BONO, Ignacio José, E-08902 L'Hospitalet de Llobregat (ES); CATENA RUIZ, Juan Lorenzo, E-08901 L'Hospitale De Llobregat (ES); LAGUNAS ARNAL, Carmen, E-08903 L'Hospitalet de Llobregat (ES); CORDOMI MONTOYA, Arnau, E-08032 Barcelona (ES); SALCEDO ROCA, Carolina, E-08757 Corbera (ES); TOLEDO MESA, Natividad, E-08410 Vilanova Del Vallès (ES); MARRERO GONZALEZ, Pedro, E-08028 Barcelona (ES); HARO BAUTISTA, Diego, E-08980 Sant Feliu de Llobregat (ES); FERNANDEZ GARCIA, Andrés, E-08034 Barcelona (ES)
(74) Representative: Rambelli, Paolo
(86) International application number: PCT/EP2004/006330
(87) International publication number: WO 2004/110983

(56) References cited:
- WO-A-00/55118
- WO-A-97/27847

## Description

The present invention relates to new benzamides acting as PPARγ and PPARγ /PPARδ modulators, as well as to processes and intermediates useful for their preparation, and to pharmaceutical compositions containing them.

### BACKGROUND ART

Peroxisome proliferator activated receptors (PPARs) belong to the superfamily of transcription factors known as nuclear receptors. This family includes steroid, retinoid and thyroid hormone receptors. Three sub-types of PPARs have been identified in humans, rodents and *Xenopus.* They are PPARα, PPARβ/δ and PPARγ, each encoded by a different gene and showing different tissue distribution.

The gene encoding for PPARγ is transcribed in humans in three different mRNA isoforms (PPARγ1, PPARγ2 and PPARγ3) through different splicing and promoter usage (Fajas et al., J. Biol. Chem. 1997, 272, 18779-18789). The PPARγ1 isoform shows a wide tissular distribution, while PPARγ2 and PPARγ3 are confined to certain tissues: PPARγ2 is expressed only in adipose tissue and PPARγ3 in adipose tissue as well as in macrophages (Fajas et al., FEBS Lett. 1998, 438, 55-60).

Differences detected in tissue distribution as well as in the activation profile of the PPARγ isoforms suggest they are involved in a variety of physiological functions playing a central role in homeostasis and lipid metabolism (Vamecq et al., Lancet 1999, 354, 141-148). These functions include, for example, lipidic transport in plasma and catabolism of fatty acids, regulation of insulin sensitivity and blood glucose levels, differentiation of macrophages that form atherosclerotic plaques, inflammatory response, carcinogenesis, hyperplasia, and adipocyte differentiation, the latter being the most verified function of the PPARγ (Grimaldi, Prog. Lipid Res. 2001, 40, 269-281, Schiller et al., J. Biol. Chem. 2001, 276, 14133-14137). Thus, the discovery of these transcription factors has provided new pharmacological targets for the development of useful therapeutic agents for the prevention and treatment of metabolic diseases such as diabetes, obesity and dyslipidaemia.

Non-insulin dependent diabetes mellitus (NIDDM) or type 2 diabetes is characterized by an insulin resistance in peripheral tissues, including muscle, liver, and adipose tissue. Glitazones, selective PPARγ agonist compounds, are drugs that reduce insulin resistance and lower blood glucose levels. Currently two products belonging to this family, rosiglitazone and pioglitazone, have been approved for the treatment of type 2 diabetes in humans.

A great effort has been made in recent years to design new drugs that improve the side effect profile of the first glitazones, show a greater affinity as a PPARγ ligands, and increase their potency in type 2 diabetes. This rational design has yielded structurally diverse compounds that show great potency and selectivity. Among them is interesting to highlight the 2-alkoxyphenylpropionic type derivatives ragaglitazar (1, EP 1049684) and tesaglitazar (2, EP 1084103). These compounds are currently in phase III and II of clinical development, respectively.

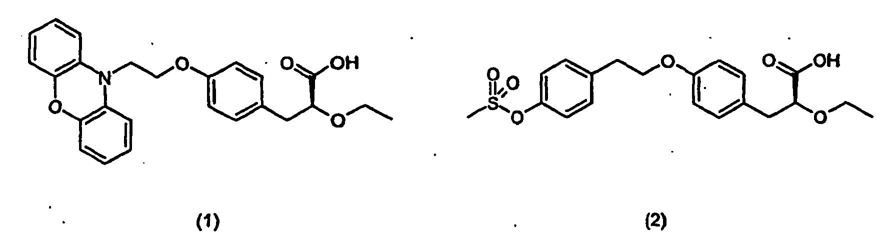

The use of compounds totally or partially blocking PPARγ activity is useful for the inhibition of adipocyte differentiation, which constitutes an effective treatment for obesity.

PPARδ activation has been shown to lead to increased levels of HDL cholesterol in db/db mice (Leibowitz et al, FEBS Lett. 2000, 473, 333-336), and in diabetic-obese rhesus monkeys, while lowering the levels of LDL, triglycerides, and insulin (Oliver et al, Proc Nat Acad Sci USA, 2001, 98, 5306-5311). The involvement of PPARδ in fatty acid oxidation in muscles was further substained in PPARα knock-out mice (Muoio et al., J. Biol. Chem. 2002, 277, 26089-26097). A number of PPARδ compounds have been reported to be useful in the treatment of hyperglycemia, hyperlipidemia and hypercholesterolemia (e.g. WO 02/59098, WO 01/603, WO 01/25181, WO 02/14291, WO 01/79197, WO 99/4815, WO 97/28149, WO 98/27974, WO 97/28115, WO 97/27857, WO 97/28137, WO 97/27847). Taken together, these observations suggest that PPARδ activation is useful in the treatment and prevention of cardiovascular diseases and conditions including atherosclerosis, hypertriglyceremia and mixed dyslipidemia (WO 01/00603) In vitro studies investigating the pharmacological modulation of PPARδ suggest that this kind of ligands may prove to be efficacious drugs for decreasing cardiovascular disease associated with metabolic syndrome, a condition comprised of a cluster of risk factors that also includes insulin resistance, obesity and hypertension (Mukjerheer, Drug News Perspect. 2002, 15, 261-267).

Pro-differentiation and lipid accumulation effects have been reported in rodent and cultured human keratinocytes, as well as protection against cell death upon PPARδ activation (Tan et al., Genes Dev. 2001, 15, 3263-3277; Schmuth et al., J. Invest. Dermatol. 2004, 122, 971-983). Modulators of these activities could be useful for treating a variety of skin disorders.

In addition, PPARδ has been implicated as a direct target in colorectal carcinogenesis in mice. All the evidences suggest that PPARδ expression may promote tumour growth and, thus, may be also a potential target for the treatment of colorectal cancer (e.g. Park et al., Prod Nat Acad Sci USA, 2001, 98, 2598-2603). While PPARγ is acknowledged as a master regulator of adipogenesis, PPARδ may also play a role in adipocyte differentiation, as demonstrated by *in vitro* and in PPARδ-deficient animals, promoting PPARγ gene expression, which upon specific ligand activation promotes adipogenesis. Thus a non-selective PPARγ/δ antagonist would be also a potential drug for obesity (Shearer et al., Curr. Med. Chem. 2003, 10, 267-280).

This indicate that research for compounds displaying various degrees of PPARγ and PPARδ modulation should lead to the discovery of drugs that have great potential in the treatment of diseases such as type-2 diabetes, dyslipidemia, syndrome X, *cardiovascular* diseases (including atherosclerosis), hypercholesteremia, colon cancer, skin disorders (including psoriasis, and wound healing, Tan et al., Expert Opin. Ther. Targets, 2004, 8, 39), and bone diseases (Pei et al., J. Clin. Invest., 2004, 113, 805-806).

Consequently, it is of great interest to provide new therapeutic agents that selectively modulate PPARγ, and PPARγ / PPARδ.

Kundu and collaborators have described benzamides (3), (4) and (5) as N-α-glucosidase inhibitors (Comb. Chem. High. 2002, 5, 545-550). WO 02/096426 disclose compounds (6) and (7) as intermediates for compounds which are matrix metalloproteinase inhibitors. Finally, WO 04/014844 disclose compounds (8) and (9) as factor IX modulators. These compounds are structurally close to those of this invention, but were described for different uses.

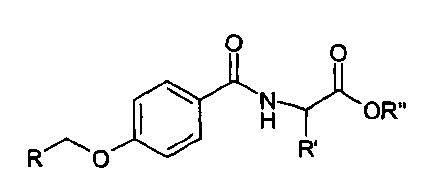

| | **R** | **R'** | **R"** |
|---|---|---|---|
| (3) | Phenyl- | Benzyl- | -H |
| (4) | 4-Methoxyphenyl- | Benzyl- | -H |
| (5) | 4-Bromophenyl- | Benzyl- | -H |
| (6) | 2- Methylquinolin-4-yl- | Cyclopentyl- | -methyl |
| (7) | 2-Methylquinolin-4-yl- | Tetrahydropyran-4-yl- | -methyl |
| (8) | Phenyl- | Biphenyl-4-ylmethyl | -H |
| (9) | Phenyl- | 4'-Trifluoromethoxybiph enyl-4-methyl- | -H |

### SUMMARY OF THE INVENTION

One aspect of the present invention relates to the provision of new compounds of formula (I), 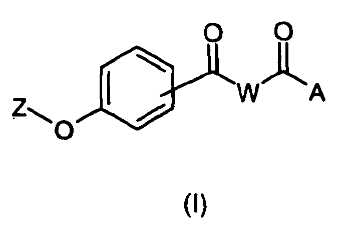 its stereoisomers and mixtures thereof, its polymorphs and mixtures thereof, and the pharmaceutically acceptable solvates and addition salts of all of them, wherein the central benzene ring may be substituted in meta- or *para-*position and,
-A is a radical selected from the group consisting of -OR1, -NR2OR1 and -NR2R3; wherein R1, R2 and R3 independently represent -H or -(C₁-C₄)-alkyl;
-W- is a biradical selected from the group: -NH-CH(E)-, and -N(D)-CH₂-CH₂-; wherein E is a radical of the -G-I-J-K type and D is a radical of the -G-I'-J-K type where:
   -G - is a bond or a -(CH₂)₁₋₄- biradical;
   -I - is a biradical of a cycle selected from the following groups:
      a) cyclopropane, cyclobutane, cyclopentane, cyclohexane and cyclohexene, all optionally substituted by one or several radicals independently selected from -OH, oxo (=O), -CHO, -SH, -NO₂, -CN, -F, -Cl, -Br, (C₁-C₄)-alkanoyl, (C₁-C₄) -alkoxycarbonyl, (C₁-C₄)-alkanoyloxy, (C₁-C₄) -alkylsulfinyl, (C₁-C₄)-alkylsulfanyl, (C₁-C₄) -alkylsulfonyl, (C₁-C₄)-alkyloxy-SO₂-, (C₁-C₄) -alkyl-SO₂O-, -NR2R3,-CONR2R3, (C₁-C₄)-alkyl optionally substituted by one or several -OH or -F, and (C₁-C₄)-alkoxyl optionally substituted by one or several -OH or -F;
      b) a five- or six-membered aromatic heterocycle containing from one to three heteroatoms selected from O, S and N, this heterocycle being optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄) -alkylsulfinyl, (C₁-C₄) alkylsulfanyl, (C₁-C₄) -alkylsulfonyl, (C₁-C₄)-alkyloxy-SO₂-, (C₁-C₄) -alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
      c) benzene or benzene substituted by one or several radicals independently selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F; and
      d) a bicyclic system consisting of a benzene fused with a five- or six-membered ring optionally containing from one to three heteroatoms selected from O, S and N, this bicyclic system being optionally substituted by one or several radicals independently selected from -OH, oxo (=O), -CHO, -SH, -NO₂, -CN, -F, -Cl, -Br, (C₁-C₄) -alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
   -J- is a bond or a biradical selected from the following groups:
      a) -(CH₂)₁₋₄-alkylidene;
      b) -O-, and
      c) -O-(C₁-C₄)-alkyl;
   -K is a radical selected from the following groups:
      a) -H;
      b) (C₁-C₄)-alkyl;
      c) a radical from a cycle selected from the following: cyclopropane, cyclobutane, cyclopentane, cyclohexane and cyclohexene, all of them optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
      d) a radical from a five- or six-membered heterocycle containing from one to three heteroatoms selected from O, S and N, being this heterocycle optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F; and
      e) phenyl or phenyl optionally substituted by one or several radicals independently selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
   -I'- is a biradical of a cycle selected from the following groups:
      a) cyclopropane, cyclobutane, cyclopentane, cyclohexane and cyclohexene, all optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
      b) a five- or six-membered aromatic heterocycle containing from one to three heteroatoms selected from O, S and N, being this heterocycle optionally substituted by one or several radicals independently selected from -OH, OXO (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄) -alkoxyl optionally substituted by one or several -OH or -F;
      c) benzene substituted by one or several radicals independently selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted-by one or several -OH or -F; and
      d) a bicyclic system consisting of a benzene fused with a five- or six-membered ring optionally containing from one to three heteroatoms selected from O, S and N, being this bicyclic system optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, (C₁-C₄)-alkoxyl optionally substituted by one or several -OH or -F;
   -Z is a radical selected from the following groups:
      a) -Q-I-J-T wherein
         -Q- is a biradical -(CH₂)₁₋₃-;
         -I- is as defined above;
         -J- is as defined above; and
         -T is a radical selected from the following groups: .
         a.a) -H;
         a.b) (C₁-C₄)-alkyl;
         a.c) a radical from a cycle selected from the following: cyclopropane, cyclobutane, cyclopentane, cyclohexane and cyclohexene, all of them optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
         a.d) a radical from a five- or six-membered heterocycle containing from one to three heteroatoms selected from O, S and N, this heterocycle being optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C4)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-S020-, -NR2R3, -CONR2R3, (C₁-C₄)-alkyl optionally substituted by one or several -OH or -F, and (C₁-C₄)-alkoxyl optionally substituted by one or several -OH or -F;
         a.e) phenyl or phenyl optionally substituted by one or several radicals independently selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O- , -NR2R3, -CONR2R3, (C₁-C₄)-alkyl optionally substituted by one or several -OH or -F, and (C₁-C₄)-alkoxyl optionally substituted by one or several -OH or -F; and
         a.f) a radical from a bicyclic system consisting of a benzene fused with a five-or six-membered ring optionally containing from one to three heteroatoms selected from O, S and N, being this bicyclic system optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
      b) -(CH₂)ₛ-X-P-I-J-T wherein
         s is 2 or 3;
         -X- is selected from the group consisting of -O-, -S-, -SO-, -SO₂- and -NR4-, being R4 a radical selected from the group:
         b.a) -H;
         b.b) (C₁-C₁₀)-alkyl;
         b.c) cycloalkyl, cycloalkyl-CO-, cycloalkyl-(C₁-C₃)-alkyl and cycloalkyl-(C₁-C₃)-alkanoyl, wherein the cycloalkyl is a five- or six-membered ring optionally substituted by one or several radicals selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and -(C₁-C₄)-alkoxyl optionally substituted by one or several OH or F;
         b.d) phenyl, phenyl-CO-, phenyl- (C₁-C₃)-alkyl and phenyl-(C₁-C₃)-alkanoyl, being this aromatic ring optionally substituted by one or several radicals selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F; and
         b.e) a heterocycle, heterocycle-CO, heterocycle-(C₁-C₃)-alkyl and heterocycle-(C₁-C₃)-alkanoyl, wherein the heterocycle is a five- or six-membered ring containing from one to three heteroatoms selected from O, S and N, being this heterocycle optionally substituted by one or several radicals selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
         -P- is a bond or a - (CH₂)₁₋₄- biradical;
         -I- is as defined above;
         -J- is as defined above; and
         -T is a radical as defined above;
      c) -(CH₂)ᵤ-CO-NRS-P-I-J-T wherein
         u is 1 or 2;
         -R5 is a radical selected from the group:
         c.a) -H;
         c.b) (C₁-C₁₀)-alkyl;
         c.c) cycloalkyl and cycloalkyl-(C₁-C₃)-alkyl, wherein the cycloalkyl is a five- or six-membered ring optionally substituted by one or several radicals selected from -OH, oxo (=O), -CHO, -SH, -NO₂, -CN, -F, -Cl, -Br, ( C₁-C4)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
         c.d) phenyl and phenyl-(C₁-C₃)-alkyl, being this aromatic ring optionally substituted by one or several radicals selected from -OH, -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C₁-C₄)-alkylsulfanyl, (C₁-C₉)-alkylsulfonyl, (C₁-C₄)-alkyloxy-SO₂-, (C₁-C₄)-alkyl-SO₂O-, -NR2R3 , -CONR2R3, (C₁-C₄)-alkyl optionally substituted by one or several -OH or -F, and (C₁-C₄)-alkoxyl optionally substituted by one or several -OH or -F; and
         c.e) a heterocycle and heterocycle-(C₁-C₃)-alkyl, wherein the heterocycle is a five- or six-membered ring containing from one to three heteroatoms selected from O, S and N, being this heterocyclo optionally substituted by one or several radicals selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄)-alkanoyloxy, (C₁-C₄)-alkylsulfinyl, (C1-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or severalseveral -OH or -F, and (C₁-C₄)-alkoxyl optionally substituted by one or several -OH or -F;
         -P- is as defined above;
         -I- is as defined above;
         -J- is as defined above; and
         -T is as defined above
with the proviso that compound of formula (I) is neither of 2-(4-benzyloxybenzoylamino)-3-phenylpropionic acid, 2-[4-(4-methoxybenzyloxy)benzoylamino]-3-phenylpropionic acid, 2-[4-(4-bromobenzyloxy)benzoylamino]-3-phenylpropionic acid, cyclopentyl-[4-(2-methylquinolin-4-ylmethoxy)benzoylamino]acetic acid methyl ester, [4-(2-methylquinolin-4-ylmethoxy)benzoylamino](tetrahydropyran-4-yl)acetic acid methyl ester or 2-(4-benzyloxybenzoylamino)-3-biphenyl-4-ylpropionic acid or 2-(4-benzyloxybenzoylamino)-3-(4'-trifluoromethoxybiphenyl-4-yl)propionic acid.

In a particular embodiment of this aspect of the invention, in the compounds of formula (I), -W- is -NH-CH(E)-. In another particular embodiment -W- is -NH-CH(E)-, and -Z is a radical of the -Q-I-J-T type. In another particular embodiment -W- is -NH-CH(E)-, and -Z is a radical of the -(CH₂)ₛ-X-P-I-J-T type. In another particular embodiment -W- is -NH-CH(E)-, and -Z is a radical of the -(CH₂)ₛ-O-P-I-J-T type. In another particular embodiment -W- is -NH-CH(E)-, and -Z is a radical of the -(CH₂)₂-NR4-P-I-J-T type. In another particular embodiment -W- is -N(E)-CH₂-CH₂-. In another particular embodiment -W- is -N(E)-CH₂-CH₂-, and -Z is a radical of the -Q-I-J-T type. In another particular embodiment -W- is -N(B)-CH₂-CH₂-, and -Z is a radical of the -(CH₂)ₛ-X-P-I-J-T type. In another particular embodiment -W-is -N(E)-CH₂-CH₂-, and -Z is a radical of the -(CH₂)ₛ-O-P-I-J-T type. In another particular embodiment -W-is -N(E)-CH₂-CH₂-, and -Z is a radical of the -(CH₂)₂-NR4-P-I-J-T type. In another particular embodiment -A is a radical of the -OR1 type.

Preferred compounds of the present invention include:
(2*S*)-3-(4-benzyloxyphenyl)-2-[4-(4-butoxybenzyloxy)benzoylamino]propionic acid methyl ester;
(2*S*)-3-(4-benzyloxyphenyl)-2-[4-(3-bromobenzyloxyl)benzoylamino]propionic acid methyl ester;
(2*S*)-3-(4-benzyloxyphenyl)-2-[4-(2-chlorobenzyloxy)benzoylamino]propionic acid methyl ester;
(2*S*)-3-(4-benzyloxyphenyl)-2-[4-(2-fluorobenzyloxy)benzoylamino]propionic acid methyl ester;
(2*S*)-3-(4-benzyloxyphenyl)-2-[4-(3-methylbenzyloxy)benzoylamino]propionic acid methyl ester;
(2*S*)-3-(4-benzyloxyphenyl)-2-[4-(3-trifluoromethylbenzyloxy)benzoylamino]propionic acid methyl ester;
(2*S*)-3-(4-benzyloxyphenyl)-2-[4-(2-methoxybenzyloxy)benzoylamino]propionic acid methyl ester;
(2*S*)-3-(4-benzyloxyphenyl)-2-[4-(2-methylbenzyloxy)benzoylamino]propionic acid methyl ester;
(2*S*)-3-(4-benzyloxyphenyl)-2-[4-(2-trifluoromethylbenzyloxy)benzoylamino]propionic acid methyl ester;
(2*S*)-3-(4-benzyloxyphenyl)-2-[4-(2-otolylethoxy)benzoylamino]propionic acid methyl ester;
(2*S*)-3-(4-benzyloxyphenyl)-2-{4-[3-(4-propoxyphenoxy)propoxy]benzoylamino}propionic acid methyl ester;
(2*S*)-3-(4-benzyloxyphenyl)-2-[4-(3-methoxybenzyloxy)benzoylamino]propionic acid methyl ester;
(2*S*)-3-(4-benzyloxyphenyl)-2-[4-(2-ethoxybenzyloxy)benzoylamino]propionic acid methyl ester;
(2*S*)-3-(4-benzyloxyphenyl)-2-[4-(4-butylbenzyloxy)benzoylamino]propionic acid methyl ester;
(2*S*)-2-[4-(4-butylbenzyloxy)benzoylamino]-3-cyclohexylpropionic acid methyl ester;
(2*S*)-2-{4-[2-(3-methylquinoxalin-2yloxy)ethoxy]benzoylamino}-3-phenylpropionic acid methyl ester;
(2*S*)-3-(4-benzyloxyphenyl)-2-[4-(2-pyridin-2-ylethoxy)benzoylamino]propionic acid methyl ester;
(2*S*)-3-(4-benzyloxyphenyl)-2-{4-[2-(3-methylquinoxalin-2-yloxy)ethoxy]benzoylamino}propionic acid methyl ester;
(2*S*)-3-(4-benzyloxyphenyl)-2-{4-[2-(pyridin-2-yloxy)ethoxy]benzoylamino}propionic acid methyl ester;
(2*S*)-3-(4-benzyloxyphenyl)-2-{4-[2-(quinolin-8-yloxy)ethoxy]benzoylamino}propionic acid methyl ester;
(2*S*)-3-(4-benzyloxyphenyl)-2-{4-[2-(quinolin-7-yloxy)ethoxy]benzoylamino}propionic acid methyl ester;
(2*S*)-3-(4-benzyloxyphenyl)-2-{4-[2-(quinolin-2-yloxy)ethoxy]benzoylamino}propionic acid methyl ester;
(2*S*)-3-(4-benzyloxyphenyl)-2-{4-[3-(3-methylquinoxalin-2-yloxy)propoxy]benzoylamino}propionic acid methyl ester;
(2*S*)-3-(4-bromophenyl)-2-{4-[2-(3-methylquinoxalin-2-yloxy)ethoxy]benzoylamino}propionic acid methyl ester;
(2*S*)-3-(4-fluorophenyl)-2-{4-[2-(3-methylquinoxalin-2-yloxy)ethoxy]benzoylamino}propionic acid methyl ester;
(2*S*)-3-(4-benzyloxyphenyl)-2-{4-[2-(3-methylquinoxalin-2-yloxy)ethoxy]benzoylamino}propionic acid ethyl ester;
(2*S*)-3-(4-benzyloxyphenyl)-2-{4-[2-(3-methylquinoxalin-2-yloxy)ethoxy]benzoylamino}propionic acid isopropyl ester;
(2*S*)-3-(4-benzyloxyphenyl)-2-{4-[2-(3-methylquinoxalin-2-yloxy)ethoxylbenzoylamino}propionic acid propyl ester;
(2*S*)-2-(4-benzyloxybenzoylamino)-3-(4-benzyloxyphenyl)propionic acid;
(2*S*)-2-[4-(3-benzyloxybenzyloxy)benzoylamino]-3-(4-benzyloxyphenyl)propionic acid;
3-{(3-benzyloxybenzyl)-[4-(2-dibenzylaminoethoxy)benzoyl]amino}propionic acid;
3-((3-benzyloxybenzyl)-{3-[2-(3-methylquinoxalin-2-yloxy)ethoxy]benzoyl}amino)propionic acid;
3-{(3-benzyloxybenzyl)-[4-(3-benzyloxybenzyloxy)benzoyl]amino}propionic acid;
2-[4-(4-benzyloxybenzyloxy)benzoylamino]-3-(4-benzyloxyphenyl)propionic acid;
(2*S*)-2-[3-(4-benzyloxybenzyloxy)benzoylamino]-3-(4-benzyloxyphenyl)propionic acid;
3-(4-benzyloxyphenyl)-2-[3-(biphenyl-4-ylmethoxy)benzoylamino]propionic acid;
2-[4-(3-benzyloxybenzyloxy)benzoylamino]-3-(4-bromophenyl)propionic acid;
3-(4-benzyloxyphenyl)-2-[4-(4-butylbenzyloxy)benzoylamino]propionic acid;
2-[4-(4-butylbenzyloxy)benzoylamino]-3-cyclohexylpropionic acid;
{(3-benzyloxybenzyl)-[4-(4-butylbenzyloxy)benzoyl]amino}acetic acid;
3-{(3-benzyloxybenzyl)-[4-(4-butylbenzyloxy)benzoyl]amino}propionic acid;
3-(4-benzyloxyphenyl)-2-[4-(2-bromobenzyloxy)benzoylamino]propionic acid;
3-(4-benzyloxyphenyl)-2-[4-(2-chlorobenzyloxy)benzoylamino]propionic acid;
3-(4-benzyloxyphenyl)-2-[4-(2-methylbenzyloxy)benzoylamino]propionic acid;
3-(4-benzyloxyphenyl)-2-[4-(3-trifluoromethylbenzyloxy)benzoylamino]propionic acid; and
3-(4-benzyloxyphenyl)-2-[4-(2-trifluoromethylbenzyloxy)benzoylamino]propionic acid.

Throughout the description and claims, the terms (C₁-C₄)-alkyl, (C₁-C₁₀)-alkyl, (C₁-C₄)-alkoxyl, (C₁-C₄)-alkanoyl, (C₁-C₄)-alkoxycarbonyl and (C₁-C₄)-alkanoyloxy shall be construed as straight or branched.

Some of the compounds of formula (I) of the present invention may have one or several chiral centres. The present invention includes each one of the possible stereoisomers and mixtures thereof, particularly racemic mixtures thereof. A single enantiomer may be prepared by any of the commonly used processes, for example, by chromatographic separation of the racemic mixture on a stationary chiral phase, by resolution of the racemic mixture by fractional crystallisation techniques of the diastereomeric salts thereof, by chiral synthesis, by enzymatic resolution or by biotransformation.

Pharmaceutically acceptable salts include, among others, addition salts of inorganic acids such as hydrochloric, hydrobromic, nitric, sulphuric and phosphoric, as well as addition salts of organic acids such as acetic, benzenesulphonic, benzoic, camphorsulphonic, mandelic, methanesulphonic, oxalic, succinic, fumaric, tartaric, and maleic. Likewise, an acid proton in compounds of formula (I) may be substituted by a metallic ion, for example, an alkaline metal ion, an alkaline-earth metal ion or an aluminium ion; or may be coordinated with an organic or inorganic base. An acceptable organic base includes diethylamine and triethylamine. An acceptable inorganic base includes aluminium hydroxide, calcium hydroxide, potassium hydroxide, sodium carbonate, and sodium hydroxide. There may be more than one cation or anion depending on the number of functions with charge and on the valency of cations and anions.

Some of the compounds of formula (I) of the present invention may exist in unsolvated as well as solvated forms such as, for example, hydrates. The present invention encompasses all such above-mentioned forms which are pharmaceutically active. Some of the compounds of general formula (I) may exhibit polymorphism, encompassing the present invention all the possible polymorphic forms, and mixtures thereof.

Compounds of general structure (I) may be prepared following various processes perfectly known by any skill person in the field of organic synthesis. Compounds of the present invention may be synthesized using the methods described below, as well as other processes known in the field of organic synthesis. Preferred methods include, but are not limited to, the general processes shown in the attached schemes.

### Method A

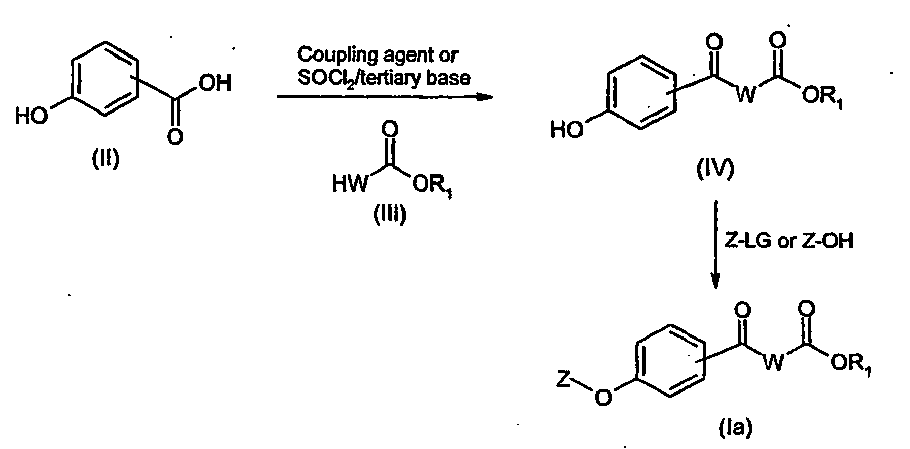

According to a first method (Method A), the phenolic acid (II) is treated with the amine derivative (III) in the presence of a suitable coupling agent, for example the combination of 1- (3-dimethylaminopropyl)-3-ethylcarbodiimide (EDC) and 1-hydroxybenzotriazole (HOBT), or with thionyl chloride in the presence of a tertiary base such as triethylamine (Elmore, Amino Acids Pep. Proteins 2001, 32, 107-162). Final compounds (Ia) are obtained by Williamson etherification by displacement of a leaving group (LG) bonded to a type -Z radical with the phenol (IV) (using for example NaH, K₂CO₃ or Cs₂CO₃ as a base in a solvent such as DMF or acetone; (Bal-Tembe et al., Bioorg. Med. Chem. 1997, 5, 1381-1388; Cantello et al., J. Med. Chem. 1994, 37, 3977-3985, Solar et al., J .Org. Chem. 1966, 31, 1996-1997; EP .875510), or by Mitsunobu reaction between (IV) and a Z-OH type alcohol in the presence of, for example, diethyl azodicarboxylate (DEAD) and triphenylphosphine in tetrahydrofurane as a solvent (Mitsunobu, Synthesis 1981, 1; Hughes, Org. Redact. 1992, 42, 335).

### Method B

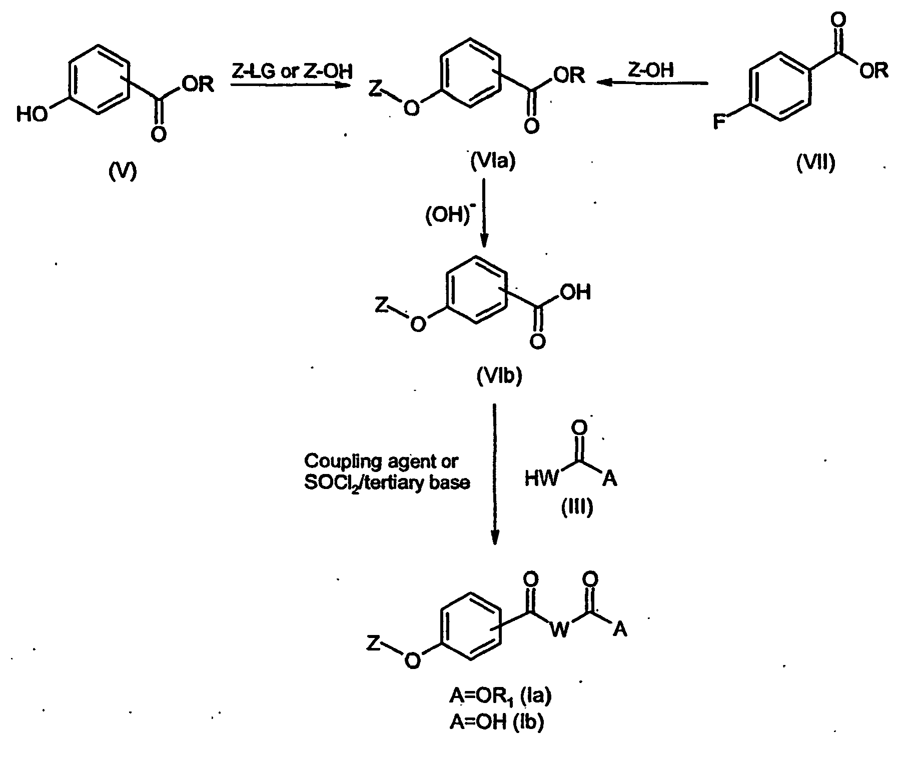

An alternative strategy (Method B) involves prior alkylation of the phenolic esters (V). After basic hydrolysis of the resulting ester, the final compounds (I) are synthesized by reaction with the amine derivative (III). Alternatively, and only in the specific case of *para* substitution of the aromatic ring, the phenolic ether (VI) may be formed by aromatic nucleophilic substitution starting from the fluorinated compound (VII).

When -Z is a radical of the -(CH₂)ₛ-X-P-I-J-T type where X is O or S, for the alkylation of the phenol another alternative procedure may be followed (Method C).

### Method C

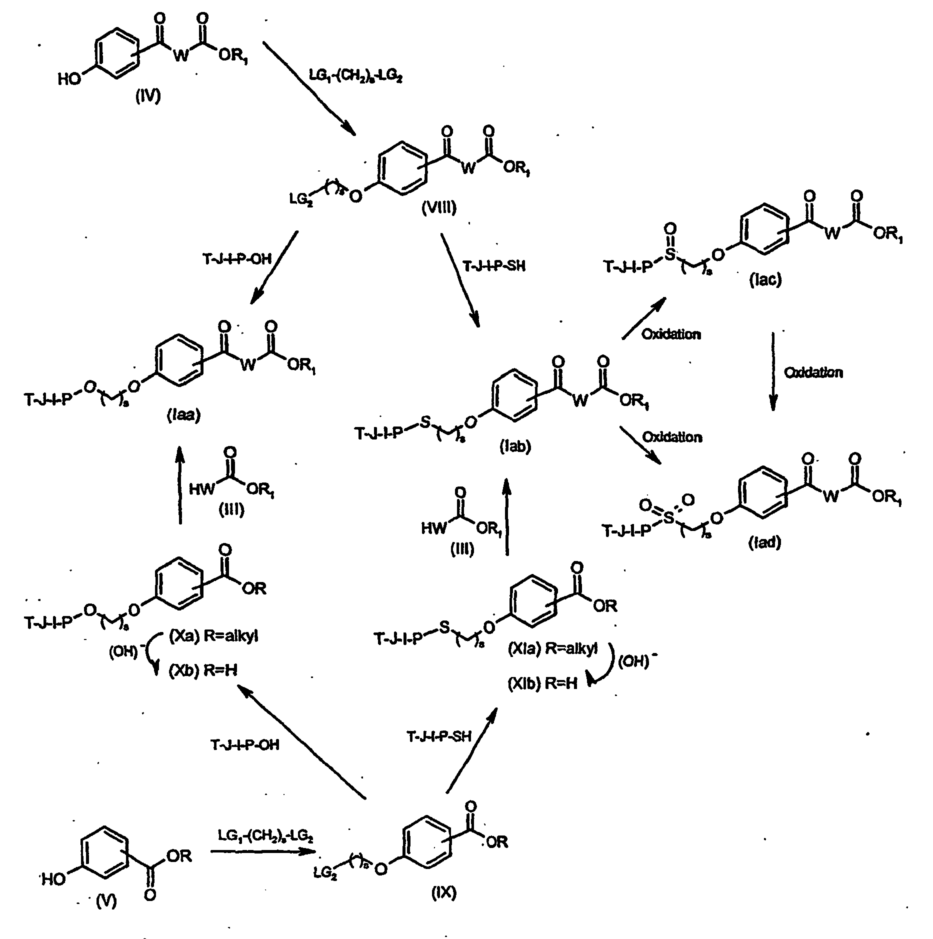

The phenol (IV) or (V) is treated with the suitable doubly functionalised alkylidene derivative (EP 875510) and, then, a nucleophilic substitution reaction with the desired alcohol or thiol is carried out to obtain the compounds (Iaa), and (Iab) or the esters (Xa) and (XIa), depending on the initial phenol. The hydrolysis of the esters (Xa) and (XIa) and their subsequent reaction with the amine derivative (III) also leads to the compounds (Iaa) and (Iab). The derivatives of the sulphoxide (Iac) and sulphone (Iad) types are obtained by oxidation of the corresponding thioether (Iab) in the presence of oxidizing agents such as, for example, hydrogen peroxide or *m*-chloroperbenzoic acid.

### Method D

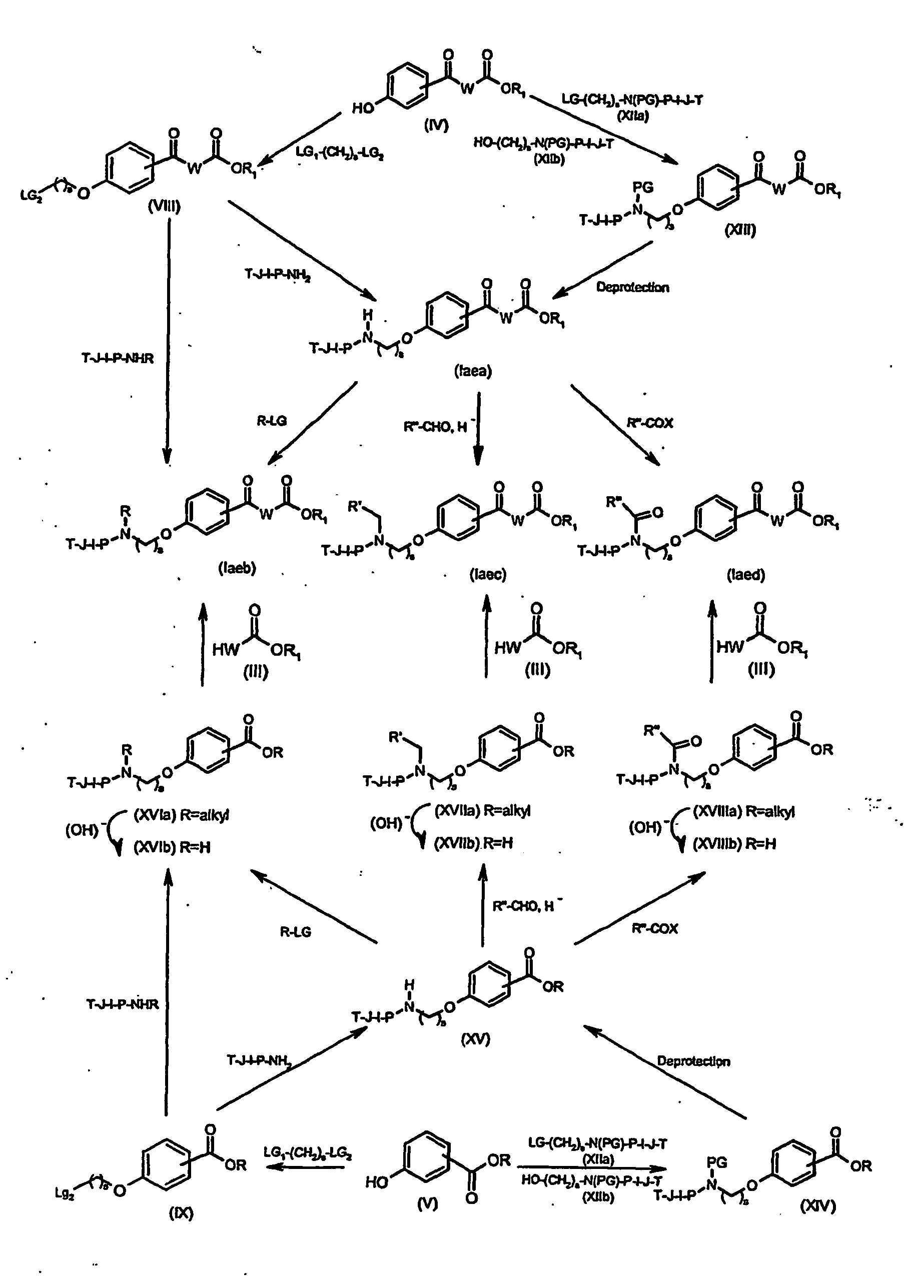

When -Z is a radical of the -(CH₂)ₛ-NR4-P-I-J-T type, the amines (Iae) may be obtained starting from the phenols (IV) or (V) following two alternative alkylation routes in every case (Method D). In the case of phenol (IV), the reaction with a doubly functionalised alkylidene derivative and, then, the nucleophilic substitution with the desired amines; or the etherification with the protected amine compound (XII) (as a trifluoroacetamide derivative, for example), and subsequent functionalisation of the amine released after its deprotection (in a basic medium, or with NaBH₄ (Harland and Hodge Synthesis 1984, 941-943)) yields the desired compounds. The tertiary amines of the (Iaeb) and (Iaec) types are obtained by the treatment of the compound (Iaea) with alkylating agents or by reductive alkylation, respectively. The amides (Iaed) are synthesized by acylation of the compound (Iaea) with the corresponding acid derivative in the presence of a tertiary amine, or by treating the secondary amine with an acid in the presence of a coupling agent such as, for example the combination of EDC and HOBT (Elmore, Amino Acids Pep. Proteins 2001, 32, 107-162). In the case of the phenol (V), the amines (Iae) are obtained by reaction of their precursor acids (XVIb), (XVIIb) and (XVIIIb) with the amine derivatives (III) following the methods outlined above. These acids, in turn, are obtained from the phenol (V) following an analogous process to that used in the case of the phenol (IV).

The Z-OH or Z-LG type compounds are products that have already been described. Some of them are commercially available or may be prepared following methods analogous to those used to synthesize others that are already known, such as those that are explained in detail in the following documents: EP 03062228; WO 97/31907; WO 01/00603; Daoud et al., J. Indian Chem. Soc. 1989, 66, 316-318 and Aquino, J. Med. Chem. 1996, 39, 562-569, some of them summarised in Scheme 1.

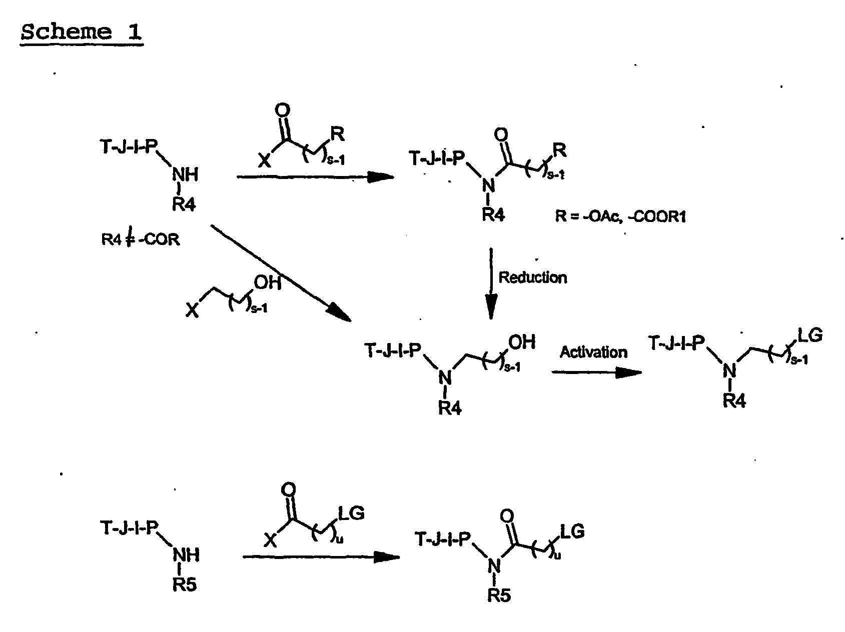

Some of the compounds (III) are commercially available products, particularly when they are α-amino acids. Others have already been described or may be synthesized following various routes, most of which have been described (March, Advanced Organic Chemistry, 1991, Ed. John Wiley & Sons; Juaristi, Enantioselective Synthesis of β-Amino Acids, 1997, Ed. Wiley-VDH).

An approach for the preparation of the α-amino acids (W is -NH-CH(E)-) is the Sorensen synthesis (Mori, Tetrahedron 1985, 2369-2377; Scheme 2), wherein dialkyl acyl-amide-malonate is alkylated in a basic medium and, after subsequent hydrolysis and decarboxylation, the desired α-amino acids (IIIa) are obtained.

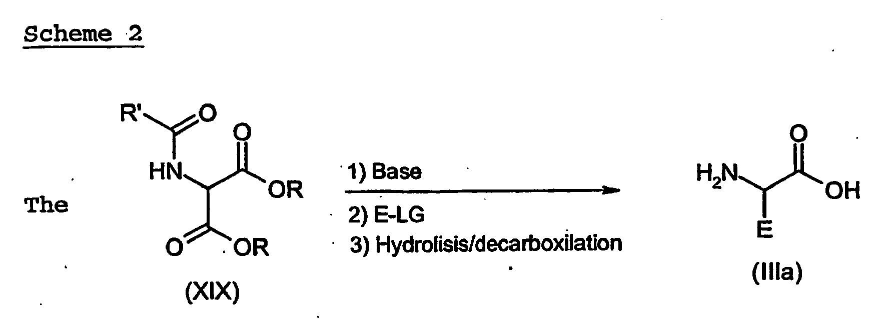

*N-*Substituted glycines and β-alanines (W is -N(E)-CH₂- or -N(D)-CH₂-CH₂-) may be synthesized by the methods shown bellow, either by reductive amination of the corresponding glycine or alanine with the suitable aldehyde (Scheme 3) using reducing agents such as NaBH₄, NaBH₃CN or NaBH(AcO)₃, or by nucleophilic substitution of the esters (XX) or (XXI) with the suitable amine (Scheme 4).

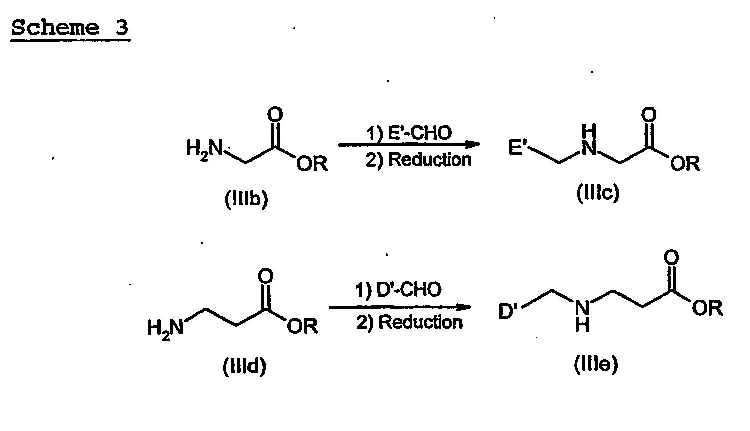

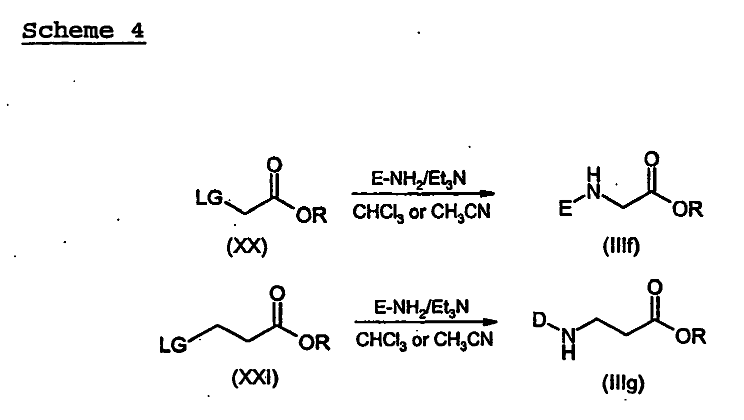

An alternative process for the synthesis of β-alanines (III) would be the addition of the corresponding amine to the α,β-unsaturated ester of interest (Scheme 5)

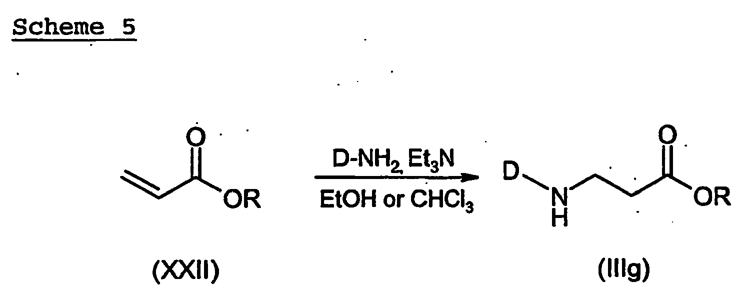

Conversion of a compound of formula (I) into a different one involves transforming the -CO-A group into a different group. The modifications considered are: the hydrolysis of the -COOR1 substituent, wherein -R1 represents a -(C₁-C₄)-alkyl moiety, to yield the corresponding carboxylic acid; the esterification of the carboxylic acids (Ib) with the R1OH alcohols; and, lastly, the amination of the -COOR1 group to obtain the corresponding amides. The hydrolysis methods used are the usual ones, for example, using an alkaline hydroxide in aqueous methanol. The amination and esterification processes are those commonly used (Scheme 6).

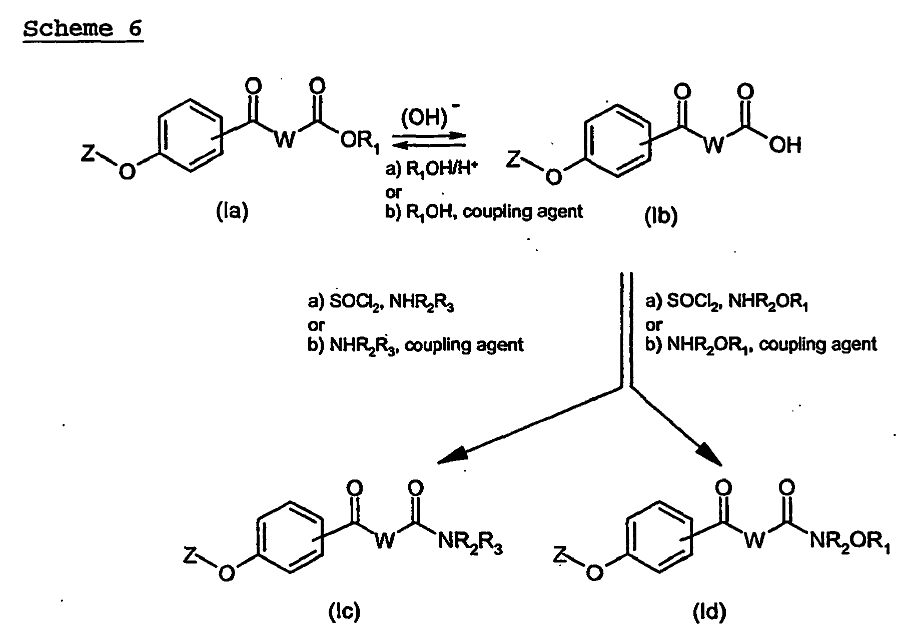

The compounds of the present invention are ligands of the PPARγ and PPARδ. Therefore, they are expectedly useful for the prophylactic and/or curative treatment of a condition mediated by PPARγ or PPARγ / PPARδ in an animal including a human. Thus, an aspect of the present invention relates to the use of these compounds for the preparation of a medicament for the prophylactic and/or curative treatment of a condition associated with metabolic diseases, particularly non-insulin-dependent diabetes mellitus, obesity, hypercholesterolaemia, and other lipid-mediated pathologies, cardiovascular diseases associated with metabolic syndrome, inflammation and inflammatory processes in general, such as rheumatoid arthritis, atherosclerosis, psoriasis, and intestinal inflammatory disease, bone diseases, particularly osteoporosis, cancer, skin wound healing, and cutaneous disorders associated with an anomalous differentiation of epidermic cells, particularly the formation of keloids. Therefore, this aspect of the invention is related to a method for the prophylactic and/or curative treatment of an animal, including a human, suffering from the above-mentioned pathologies, which comprises administering a therapeutically effective amount of a formula (I) compound.

Another aspect of the invention relates to pharmaceutical compositions comprising a therapeutically effective amount of the compound (I), as the active ingredient, together with appropiate amounts of pharmaceutically acceptable excipients. Preferably, the compound is administered orally, parenterally or topically.

Throughout the description and claims the word "comprise" and variations of the word, such as "comprising", is not intended to exclude other additives, components, elements or steps. The disclosures in the abstract accompanying this application and in the application from which priority is claimed, are incorporated herein as reference.

Additional objects, advantages and novel features of the invention will be set forth in part in the description, and in part will become apparent to those skilled in the art upon examination of the description or may be learned by practice of the invention. The present invention will be further illustrated by the following examples. The examples are given by way of illustration only and are not to be construed as limiting.

### EXAMPLES

¹H-NMR spectra of the compounds have been recorded using a VARIAN GEMINI-200 MHz and a VARIAN UNITY-300 MHz equipment and chemical shifts are expressed as ppm (δ) from the internal reference TMS. Mass spectra have been obtained with an Agilent 1100 VL mass spectrometer. The nomenclature of the different compounds used in the present document is based on the software AUTONOM (Automatic Nomenclature) from the Beilstein Institute, which uses the IUPAC systematic nomenclature.

### INTERMEDIATES (IV)

### METHOD A:

To a solution of 1 eq of the aminic derivative (III), 1 eq of the acid (II), 1.3 eq of HOBT, and 1.3 eq of EDC in tetrahydrofurane, the solution being 0.2 M in the aminic derivative, 2 eq of triethylamine were added. The reaction mixture was stirred at room temperature for 18h, and then water and dichloromethane were added. The organic layer was separated, and the aqueous layer was extracted once with dichloromethane. The organic layers were combined, dried over anhydrous sodium sulfate, and filtered. The solvent was distilled off under reduced pressure and the obtained residue was purified by column chromatography.

### METHOD B:

To a solution 0.1 M of 1 eq of the aminic derivative (III) in anhydrous dichloromethane, 2 eq of triethylamine, and 1.2 eq of the corresponding acid chloride, were added. The reaction' mixture was either refluxed with stirring (secondary amines) or stirred at room temperature (primary amines) for 18h, then treated with water, twice with sodium bicarbonate and, finally, with a brine. The solvent was distilled off under reduced pressure and the obtained residue was purified by column chromatography.

### METHOD C:

To a solution of 1 eq of the aminic derivative (III), and 1 eq of the corresponding acid chloride in ethyl acetate, the solution being 0.05 M in the aminic derivative, Amberlyst 21 (200 mg/mmol acid chloride) was added. The reaction mixture was either refluxed with stirring (secondary amines) or stirred at room temperature (primary amines). Then, the resin was filtered, and the solvent was distilled off under reduced pressure. The obtained residue was purified by column chromatography.

**TABLE 1**

| **INTERM** | |
|---|---|
| IV.1 | (2*S*)-3-(4-Benzyloxyphenyl)-2-(4-hydroxybenzoylami no)propionic acid methyl ester; ¹H-NMR: 7.52 (d, 2H), 7.31-7.20 (m, 5H), 6.97 (d, 2H), 6.81 (d, 2H), 6.74 (d, 2H), 4.94 (s,2H), 4.86 (m, 1H), 3.66 (s, 3H), 3.06 (m, 2H) |
| IV.2 | (2*S*)-3-(4-Benzyloxyphenyl)-2-(3-hydroxybenzoylami no)propionic acid methyl ester; MS: 406 |
| IV.3 | (2*S*)-3-Cyclohexyl-2-(4-hydroxybenzoylamino)propio nic acid methyl ester; MS: 306 |

### INTERMEDIATES (VIa)

### METHOD D:

A suspension of 1 eq of phenol (V), 3 eq of anhydrous potassium carbonate, and 1.3 eq of the Z-LG derivative in ethyl acetate, the suspension being aproximately 0.5 M in the phenol (V), was refluxed for 18h. Then, the suspension was allowed to cool down and the white solid was filtered. The solvent was distilled off under reduced pressure, and the obtained residue was purified by column chromatography.

### METHOD E:

A suspension of 1 eq of phenol (V), 3 eq of cesium carbonate, 1.3 eq of the Z-LG derivative, and a catalythic amount of potassium iodide in anhydrous, dimethylformamide (DMF), the suspension being 0.1 M in the phenol (V), was heatet at 80°C for 18h. Then, the suspension was allowed to cool down at room temperature, and then water and ethyl acetate were added. The organic layer was washed three times with brine, then dried over anhydrous sodium sulfate and filtered. The solvent was distilled off under reduced pressure, and the obtained residue was purified by column chromatography.

### METHOD F:

To a solution 0.1 M of 1 eq of phenol (V) in anhydrous DMF, containing a catalythic amount of potassium iodide, 1.1 eq of 60% sodium hydride in paraffin were added. The suspension was stirred at room temperature for 10 minutes and then 1.1 eq of the Z-LG derivative were added. The resulting solution was stirred at 80°C for 18h, and then allowed to cool down to room temperature. After treating with water and ethyl acetate, the organic layer was washed three times with brine, then dried over anhydrous sodium sulfate and filtered. The solvent was distilled off under reduced pressure, and the obtained residue was purified by column chromatography.

### METHOD G:

To a solution of 1 eq of phenol (V), 2.2 eq of the Z-OH derivative, and 2.2 eq of triphenylphosphine in tetrahydrofurane, the solution being 0.2 M in the phenol, 2.2 eq of DEAD were added under inert atmosphere. The reaction mixture was stirred at room temperature for 18h. Then, the solvent was distilled off under reduced pressure, and the obtained residue was purified by column chromatography.

### METHOD H:

To a solution 0.01 M of 1 eq of the Z-OH derivative in anhydrous DMF, 1.1 eq of 60% sodium hydride in paraffine were added slowly with stirring until bubbling was finished. Then, 1.2 eq of 4-fluorobenzoic acid methyl ester were added, and the mixture was heated art 80°C for 20h. The resulting solution was carefully poured over water/ice, and the mixture formed was extracted four times with ethyl acetate. The organic extracts were washed five times with brine, then dried over anhydrous magnesium sulfate and filtered. The solvent was distilled off under reduced pressure, and the obtained residue was purified by column chromatography.

**TABLE 2**

| INTERM | |
|---|---|
| VIa.1 | 4-[2-(Dibenzylamino)ethoxy]benzoic methyl ester; ¹H-NMR: 8.00 (d, 2H), 7.45-7.20 (m, 10H), 6.85 (d, 2H), 4.05 (t, 2H), 3.90 (s, 3H), 3.75 (s, 4H), 2.91 (t, 2H) |
| VIa.2 | 4-[2-(2-Phenyloxazol-4-yl-5-methyl)ethoxy]benzoic acid methyl ester; ¹H-NMR: 7.99-7.85 (m, 4H), 7.44-7.38 (m, 3H), 6.88 (d, 2H), 4.23 (t, 2H), 3.85 (s, 3H), 2.98 (t, 2H), 2.36 (s, 3H) |
| VIa.3 | 3-[2-Dibenzylamino)ethoxy] benzoic acid methyl ester; ¹H-NMR: 7.65-7.00 (m, 14H), 4.09 (t, 2H), 3.92 (s, 3H), 3.74 (s, 4H), 2.93 (t, 2H) |
| VIa.4 | 3-(4-Butylbenzyloxy)benzoic acid methyl ester; MS: 299 |
| VIa.5 | 4-(2-Pyridin-2-ylethoxy)benzoic acid methyl ester; MS: 258 |
| VIa.6 | 4-(2-Naphthalen-2-ylethoxy)benzoic acid methyl ester; MS: 307 |
| VIa.7 | 4-(4-Butylbenzyloxy)benzoic acid methyl ester; MS: 299 |
| VIa.8 | 3-(4-Benzyloxybenzyloxy)benzoic acid methyl ester; MS: 349 |
| VIa.9 | 4-(3-Benzyloxybenzyloxy)benzoic acid methyl ester; MS: 349 |
| VIa.10 | 3-(3-Benzyloxybenzyloxy)benzoic acid methyl ester; MS: 349 |

### INTERMEDIATES (VIb)

### METHOD J:

To a solution 0.1 M of 1 eq of intermediate (VIa) in a mixture of 3:1 tetrahydrofurane:methanol, between 1.5 and 10 eq of lithium hydroxide 1 M in water were added. The resulting mixture was stirred at room temperature for 18h, then treated with HCl 1 N until pH=5-6, and extracted twice with ethyl acetate. The organic layers were dried over anhydrous sodium sulfate and filtered. The solvent was distilled off under reduced pressure, and the obtained residue was purified by column chromatography.

### METHOD K:

To a solution 0.05 M of 1 eq of intermediate (VIa) in methanol, 5 eq of potassium hydroxide 1.4 M were added. The resulting solution was stirred at room temperature for 18h, then treated with HCl 1 N until acid pH, and extracted with ethyl acetate twice. The organic extracts were dried over anhydrous sodium sulfate and filtered. The solvent was distilled off under reduced pressure, and the obtained residue was purified by column chromatography.

**TABLE 3**

| INTERM | |
|---|---|
| VIb.1 | 4- [2-(Dibenzylamino)ethoxy]benzoic acid; ¹H-NMR: 7.98 (d, 2H), 7.47-7.27 (m, 10H), 6.91 (d, 2H), 4.14 (t, 2H), 3.78 (s, 4H), 2.95 (t, 2H) |
| VIb.2 | 4-[2-(2-Phenyloxazol-4-yl-5-methyl)ethoxy]benzoic acid; ¹H-NMR: 7.95-7.83 (m, 4H), 7.50-7.46 (m, 3H), 7.01 (d, 2H), 4.28 (t, 2H), 2.95 (t, 2H), 2.35 (s, 3H) |
| VIb. 3 | 3-[2-(Dibenzylamino)ethoxyl benzoic acid; ¹H-NMR: 7.54-7.00 (m, 14H), 4.40 (t, 2H), 4.31 (s, 4H), 3.39 (t, 2H) |
| VIb.4 | 4-(3-Benzyloxybenzyloxy)benzoic acid; MS: 335 |
| VIb.5 | 3-(3-Benzyloxybenzyloxy)benzoic acid; MS: 335 |
| VIb.6 | 4-(2-Pyridin-2-ylethoxy)benzoic acid; MS: 244 |
| VIb.7 | 3-(4-Butylbenzyloxy)benzoic acid; MS: 285 |
| VIb.8 | 4-(2-Naphthalen-2-ylethoxy)benzoic acid; MS: 293 |
| VIb.9 | 4-(4-Butylbenzyloxy)benzoic acid; MS: 285 |
| VIb.10 | 3-(4-Benzyloxybenzyloxy)benzoic acid; ¹H-NMR: 7.60 (br s, 2H), 7.43-7.22 (m, 8H), 7.10 (dd, 1H), 6.94 (d, 2H), 5.02 (s, 2H), 4.97 (s, 2H) |

### INTERMEDIATES (VIII)

### METHOD L:

To a suspension of 1 eq of phenol (IV), and 2 eq of cesium carbonate in anhydrous DMF, the suspension being 0.4 M in the phenol, 100 eq of LG₁-(CH₂)ₛ-LG₂ were added. The reaction mixture was heated at 90°C for 18h, and then treated with water and 1,2-dichloroethane. The organic layer was washed three times with brine, then dried over anhydrous sodium sulfate and filtered. The solvent was distilled off under reduced pressure, and the obtained residue was purified by column chromatography.

**TABLE 4**

| INTERM | |
|---|---|
| VIII.1 | (2*S*)-3-(4-Benzyloxyphenyl)-2-[3-(2-chloroethoxy)b enzoylamino] propionic acid methyl ester; ¹H-NMR: 7.71 (d, 2H), 7.50-7.33 (m, 5H), 7.06 (d, 2H), 6.93-6.89 (m, 4H), 6.58 (d, 1H), 5.07-5.01 (m, 3H), 4.25 (t, 2H), 3.82 (t, 2H), 3.76 (s, 3H), 3.20 (m, 2H) |
| VIII.2 | (2*S*)-3-(4-Benzyloxyphenyl)-2-[3-(2-chloroethoxy)b enzoylamino]propionic acid methyl ester; ¹H-NMR: 7.50-7.25 (m, 8H), 7.10-7.00 (m, 3H), 6.91 (d, 2H), 6.57 (d, 1H), 5.10-5.00 (m, 3H), 4.27 (t, 2H), 3.83 (t, 2H), 3.78 (s, 3H), 3.24 (dd, 1H), 3.16 (dd, 1H) |

### INTERMEDIATE (Xa), (XVIa) and (XIa)

The following compounds were synthesized according to any of methods D, E or F, starting from intermediates (IX).

**TABLE 5**

| INTERM | |
|---|---|
| Xa.1 | 4-[2-(3-Methylquinoxalin-2-yloxy)ethoxy]benzoic acid methyl ester; ¹H-NMR: 8.02 (d, 2H), 7.95 (d, 1H), 7.88 (d, 1H), 7.65-7.48 (m, 2H), 7.00 (d, 2H), 4.87 (t, 2H), 4.48 /t, 2H), 3.89 (s,3H), 2.64 (s, 3H) |
| Xa.2 | 3-[2-(3-Methylquinoxalin-2-yloxy)ethoxy]benzoic acid methyl ester; MS: 339 |
| XVIa.1 | 3-[2-(3-Methyl-2-oxo-2*H*-quinoxalin-1-yl)ethoxy]be nzoic acid methyl ester; MS: 339 |
| XVIa.2 | 4-[2-(3-Methyl-2-oxo-2*H*-quinoxalin-1-yl)ethoxy]be nzoic acid methyl ester; MS: 339 |
| XIa .1 | 3-[2-(Thiophen-2-ylsulfanyl)ethoxy]benzoic acid methyl ester; MS: 295 |

### INTERMEDIATE (Xb), (XVIb) and (XIb)

The following compounds were synthesized according to any of methods J or K, starting from intermediates (Xa), (XVIa) or (XIa).

**TABLE 6**

| INTERM | |
|---|---|
| Xb.1 | 4-[2-(3-Methylquinoxalin-2-yloxy)ethoxy]benzoic acid; MS: 325 |
| Xb.2 | 3-[2-(3-Methylquinoxalin-2-yloxy)ethoxy]benzoic acid; MS: 325 |
| XVIb.1 | 3-[2-(3-Methyl-2-oxo-2H-quinoxalin-1-yl)ethoxy]be nzoic acid; MS: 325 |
| XVIb.2 | 4 - [2- (3-Methyl-2-oxo-2H-quinoxalin-1-yl) ethoxy] be nzoic acid; MS: 325 |
| XIb.1 | 3-[2-(Thiophen-2-ylsulfanyl)ethoxy]benzoic acid; MS: 281 |

### INTERMEDIATE (XIV)

The following compounds were synthesized according to any of methods D to G, starting from phenol (V) and amines (XIIa) or (XIIb).

**TABLE 7**

| INTERM | |
|---|---|
| XIV.1 | 3-{2-[Benzyl-(2,2,2-trifluoroacetyl)amino]ethoxy} benzoic acid methyl ester; MS: 382 |
| XIV.2 | 4-{2-[Benzyl-(2,2,2-trifluoroacetyl)amino]ethoxy} benzoic acid methyl ester; MS: 382 |

### INTERMEDIATE (XV)

The following compounds were synthesized either according to any of methods D to F, starting either from intermediate (IX), and the corresponding amines, or according to method M, from intermediate (XIV).

### METHOD M:

To a solution 0.1 M of 1 eq of intermediate (XIV) (PG = trifluoroacetyl) in a mixture of tetrahydrofurane:methanol (3:1), 5 eq of lithium hydroxide 1 M in water were added. The solution was stirred until complete dissolution, then diluted with a mixture of water/ethyl acetate, and then acidified to pH=5 with HC1 1 N. The organic layer was dried over anhydrous sodium sulfate and filtered. The solvent was distilled off under reduced pressure, and the obtained residue was disolved in methanol 0.1 M and treated with 3.2 eq of thionyl chloride. The solution was refluxed for 18h, and then allowed to cool down to room temperature. The solvent was distilled off under reduced pressure and the residual solid was broken up with hexane.

**TABLE 8**

| INTERM | |
|---|---|
| XV.1 | 4-(2-Benzylaminoethoxy)behzoic acid methyl ester; ¹H-NMR_{:} 7.96 (d, 2H), 7.34-7.21 (m, 5H), 6.89 (d, 2H), 4.12 (t, 2H), 3.87 (s, 2H), 3.85 (s, 3H), 3.10 (t, 2H); MS: 286 |
| XV. 2 | 3-(2-Benzylaminoethoxy)benzoic acid methyl ester; MS: 286 |

### INTERMEDIATE (XVIIIa)

The following compounds were synthesized according to any of methods A to C, starting from intermediate (XV) and the corresponding acids.

**TABLE 9**

| INTERM | |
|---|---|
| XVIIIa.1 | 4-[2-(*N*-Benzyl-*N*-benzoylamino)ethoxy]benzoic acid methyl ester; ¹H-NMR: 7.99 (d, 2H) , 7.43-6.77 (m, 12H), 4.91-4.70 (m, 2H), 4.35-3.65 (m, 4H), 3.90 (s, 3H) |
| XVIIIa.2 | 4-{2-[*N*-Benzyl-*N*-(pyridin-3-ylcarbonyl)amino] et hoxy}benzoic acid methyl ester; ¹H-NMR: 8. 80-6.85 (m, 13H), 4.91-4.69 (m, 2H), 4.36-3.60 (m, 4H), 3.86 (s, 3H) |

### INTERMEDIATE (XVIIIb)

The following compounds were synthesized according to methods J or K, starting from intermediate (XVIIIa).

**TABLE 10**

| INTERM | |
|---|---|
| XVIII b.1 | 4-[2-(*N*-Benzyl-*N*-benzoylamino)ethoxy]benzoic; ¹H-NMR: 8.06 (d, 2H), 7.41-6.81 (m, 12H), 4.94-4.71 (m, 2H), 4.37-3.67 (m, 4H) |
| XVIII b.2 | 4-{2-[*N*-Benzyl-*N*-(pyridin-3-ylcarbonyl)amino]ethox y}benzoic acids ¹H-NMR: 8.75-6.85 (m, 13H), 4.91-4.72 (m, 2H), 4.36-3.60 (m, 4H) |

### EXAMPLE (Ia):

The compounds of formula (Ia) shown in Table 11 were synthesized according to any of methods D to G, starting from intermediate (IV):

**TABLE 11**

| Ex. | |
|---|---|
| 1 | (2*S*)-3-(4-Benzyloxyphenyl)-2-[4-(3-phenylallyloxy)benzoylamino]propionic acid methyl ester; ¹H-NMR: 7.73 (d, 2H), 7.43-7.25 (m, 10H), 7.07 (d, 2H), 6.98 (d, 2H), 6.92 (d, 2H), 6.76 (d, 1H), 6.57 (d, 1H), 6.42 (dt, 1H), 5.10-5.00 (m, 3H), 4.75 (dd, 2H), 3.77 (s, 3H), 3.28-3.16 (m, 2H) |
| 2 | (2*S*)-3-(4-Benzyloxyphenyl)-2-[4-(4-phenoxybenzyloxy)benzoylamino]propionic acid methyl ester; ¹H-NMR: 7.71 (d, 2H), 7.42-6.88 (m, 20H), 6.48 (d, 1H), 5.07-5.04 (m, 5H), 3.77 (s, 3H), 3.25-3.10 (m, 2H) |
| 3 | (2*S*)-3-(4-Benzyloxyphenyl)-2-[4-(biphenyl-4-ylmethoxy)benzoylamino]propionic acid methyl ester; ¹H-NMR: 7.72 (d, 2H), 7.65-7.26 (m, 14H), 7.07-7.00 (m,4H), 6.90 (d, 2H), 6.48 (d, 1H), 5.16 (s, 2H), 5.10-5.00 (m, 3H), 3.77 (s, 3H), 3.30-3.10 (m, 2H) |
| 4 | (2*S*)-3-(4-Benzyloxyphenyl)-2-[4-(3-phenoxybehzyloxy) benzoylamino]propionic acid methyl ester; ¹H-NMR: 7.70 (d, 2H), 7.43-6.92 (m, 20H), 6.48 (d, 1H), 5.08-5.04 (m, 5H), 3.77 (s, 3H), 3.30-3.10 (m, 2H) |
| 5 | (2*S*)-2-[4-(3-Benzyloxybenzyloxy)benzoylamino]-3-(4-benzyloxyphenyl) propionic acid methyl ester; ¹H-NMR: 7.70 (d, 2H), 7.43-6.92 (m, 20H), 6.48 (d, 1H), 5.09 (s, 2H), 5.08 (s, 2H), 5.06-5.02 (m, 3H), 3.77 (s, 3H), 3.27-3.14 (m, 2H) |
| 6 | (2*S*)-3-(4-Benzyloxyphenyl)-2-(4-phenethyloxybenzoylamino)propionic acid methyl ester; ¹H-NMR: 7.69 (d, 2H), 7.42-7.30 (m, 10H), 7.04 (d, 2H), 6.92-6.88 (m, 4H), 6.45 (d, 1H), 5.06-5.00 (m, 3H), 4.22 (t, 2H), 3.77 (s, 3H), 3.27-3.10 (m, 4H) |
| 7 | (2*S*)-3-(4-Benzyloxyphenyl)-2-[4-(3-phenylpropoxy)benzoylamino]propionic acid methyl ester; ¹H-NMR: 7.70 (d, 2H), 7.43-7.22 (m, 10H), 7.06 (d, 2H), 6.93-6.89 (m, 4H), 6.51 (d, 1H), 5.10-5.02 (m, 3H), 4.00 (t, 2H), 3.77 (s, 3H), 3.25-3.15 (m, 2H), 2.83 (t, 2H), 2.12 (m, 2H) |
| 8 | (2*S*)-2-[4-(4-Benzyloxybenzyloxy)benzoylamino]-3-(4-benzyloxyphenyl)propionic acid methyl ester. ¹H-NMR: 7.70 (d, 2H), 7.46-7.35 (m, 13H), 7.06-6.97.(m, 7H), 6.89 (d, 2H), 6.49 (d, 1H), 5.09-5.00 (m, 7H), 3.76 (s, 3H), 3.26-3.11 (m, 2H) |
| 9 | (2*S*)-3-(4-Benzyloxyphenyl)-2-[4-(biphenyl-2-ylmethoxy)benzoylamino]propionic acid methyl ester; MS: 572 |
| 10 | (2*S*)-3-(4-Benzyloxyphenyl)-2-[3-(3-phenylallyloxy)benzoylamino]propionic acid methyl ester; MS: 522 |
| 11 | (2*S*)-2-[3-(4-Benzyloxybenzyloxy)benzoylamino]-3-(4-benzyloxyphenyl)propionic acid methyl ester; ¹H-NMR: 7.47-7.26 (m, 15H), 7.13-7.00 (m, 5H), 6.93 (d, 2H), 6.61 (d, 1H), 5.09-5.00 (m, 7H), 3.78 (s, 3H), 3.29-3.15 (m, 2H) |
| 12 | (2*S*)-3-(4-Benzyloxyphenyl)-2-[3-(biphenyl-4-ylmethoxy)benzoylamino]propionic acid methyl ester; MS: 572 |
| 13 | (2*S*)-3-(4-Benzyloxyphenyl)-2-[3-(3-phenoxybenzyloxy)benzoylamino]propionic acid methyl ester; MS: 588 |
| 14 | (2*S*)-3-(4-Benzyloxyphenyl)-2-[3-(3-phenylpropoxy)benzoylamino]propionic acid methyl ester; MS: 524 |
| 15 | (2*S*)-3-(4-Benzyloxyphenyl)-2-[4-(4-butoxybenzyloxy)benzoylamino]propionic acid methyl ester; MS: 568 |
| 16 | (2*S*)-2-[4-(4-Butoxybenzyloxy)benzoylamino]-3-cyclohexylpropionic acid methyl ester; MS: 468 |
| 17 | (2*S*)-3-(4-Benzyloxyphenyl)-2-[4-(thiophen-3-ylmethoxy)benzoylamino]propionic acid methyl ester; MS : 488 |
| 18 | (2*S*)-3-(4-Benzyloxyphenyl)-2-[4-(2-bromobenzyloxy)benzoylamino]propionic acid methyl ester; MS: 575 |
| 19 | (2*S*)-3-(4-Benzyloxyphenyl)-2-[4-(3-bromobenzyloxy)benzoylamino]propionic acid methyl ester; MS: 575 |
| 20 | (2*S*)-3-(4-Benzyloxyphenyl)-2-[4-(2-chlorobenzyloxy)benzoylamino]propionic acid methyl ester; MS: 530 |
| 21 | (2*S*)-3-(4-Benzyloxyphenyl)-2-[4-(3-chlorobenzyloxy)benzoylamino]propionic acid methyl ester; MS: 530 |
| 22 | (2*S*)-3-(4-Benzyloxyphenyl)-2-[4-(2-fluorobenzyloxy)benzoylamino]propionic acid methyl ester; MS: 514 |
| 23 | (2*S*)-3-(4-Benzyloxyphenyl)-2-[4-(3-methylbenzyloxy)benzoylamino]propionic acid methyl ester; MS: 496 |
| 24 | (2*S*)-3-(4-Benzyloxyphenyl)-2-[4-(3-trifluoromethy lbenzyloxy)-benzoylamino] propionic acid methyl ester; MS: 564 |
| 25 | (2*S*)-3-(4-Benzyloxyphenyl)-2-[4-(2-methoxybenzyloxy)benzoylamino]propionic acid methyl ester; MS: 526 |
| 26 | (2*S*)-2-[4-(3-Bromobenzyloxy)benzoylamino]-3-cyclohexylpropionic acid methyl ester; MS: 475 |
| 27 | (2*S*)-3-(4-Benzyloxyphenyl)-2-[4-(2-methylbenzyloxy)benzoylamino]propionic acid methyl ester; MS: 510 |
| 28 | (2*S*)-3-(4-Benzyloxyphenyl)-2-[4-(2-trifluoromethylbenzyloxy)-benzoylamino] propionic acid methyl ester; MS: 564 |
| 29 | (2*S*)-3-(4-Benzyloxyphenyl)-2-[4-(2-o-tolylethoxy)benzoylaminolpropionic acid methyl ester; MS: 524 |
| 30 | (2*S*)-3-(4-Benzyloxyphenyl)-2-{4-[3-(4-propoxyphenoxy)propoxy] benzoylamino} propionic acid methyl ester; MS: 598 |
| 31 | (2*S*)-3-(4-Benzyloxyphenyl)-2-[4-(3-methoxybenzyloxy)benzoylamino] propionic acid methyl ester; MS: 526 |
| 32 | (2*S*)-3-(4-Benzyloxyphenyl)-2-[4-(2-ethoxybenzyloxy)benzoylamino]propionic acid methyl ester; MS: 540 |
| 33 | 3-(4-Benzyloxyphenyl)-2-(4-[(diphenylcarbamoyl)methoxy]-benzoylamino}propionic acid methyl ester; ¹H-NMR: 7.64 (d, 2H), 7.41-7.26 (m. 15H), 7.03 (d, 2H), 6.91-6.81 (m, 4H), 6.55 (d, 1H), 5.08-4.95 (m, 3H), 4.61 (s, 2H), 3.74 (s, 3H), 3.26-3.06 (m, 2H) |
| 34 | 3-(4-Benzyloxyphenyl)-2-{4-[(benzylphenylcarbamoyl)methoxy]-benzoylamino}propionic acid methyl ester; ¹H-NMR: 7.65 (d, 2H), 7.40-6.77 (m, 21H), 6.54 (d, 1H), 5.08-4.95 (m, 3H), 4.90 (s, 2H), 4.42 (s, 2H), 3.74 (s, 3H), 3.25-3.06 (m, 2H) |
| 35 | 3-(4-Benzyloxyphenyl)-2-{4-[(dibenzylcarbamoyl)methoxy]-benzoylamino}propionic acid methyl ester; ¹H-NMR: 7.69 (d, 2H), 7.42-7.16 (m, 15H), 7.05 (d, 2H), 6.90 (d, 4H), 6.53 (d, 1H), 5.09-4.97 (m, 3H), 4.83 (s, 2H), 4.62 (s, 2H), 4.51 (s, 2H), 3.76 (s, 3H), 3.29-3.10 (m, 2H) |
| 36 | 3-(4-Benzyloxyphenyl)-2-{4-[2-(10,11-dihydrodibenzo[b,f]azepin-5-yl)-2-oxoethoxy]benzoylamino}propionic acid methyl ester; ¹H-NMR: 7.65 (d,'2H), 7.41-6.81 (m, 19H), 6.49 (d, 1H), 5.09-4.97 (m, 3H), 4.80 (d, 1H), 4.45 (d, 1H), 3.75 (s, 3H), 3.45-3.17 (m, 4H), 2.93-2.81 (m, 2H) |
| 37 | 3-(4-Benzyloxyphenyl)-2-{4-[(diphenylcarbamoyl)methoxy]-benzoylamino}propionic acid; ¹H-NMR: 7.61 (d, 2H), 7.37-6.66 (m, 22H), 5.00-4.85 (m, 3H), 4.59 (s, 2H), 3.30-3.10 (m, 2H) |
| 38 | 3-(4-Benzyloxyphenyl)-2-(4-{[(3-methoxyphenyl)phenylcarbamoyl]methoxy}-benzoylamino)propionic acid methyl ester; MS: 645 |
| 39 | 3-(4-Benzyloxyphenyl)-2-{4-[(cyclohexylphenylcarbamoyl)methoxy]-benzoylamino}propionic acid methyl ester; MS: 621 |

The compounds of formula (Ia) shown in Table 12 were synthesized according to any of methods A to C, starting from intermediate (VIb):

**TABLE 12**

| Ex. | |
|---|---|
| 40 | {[4-(2-Dibenzylaminoethoxy)benzoyl]-(3-phenoxybenzyl)amino}acetic acid ethyl ester; ¹H-NMR: 7.42-6.75 (m, 23H), 4.80-4.65 (m, 2H), 4.30-3.99 (m, 6H), 3.72 (s, 4H), 2.90 (t, 2H), 1.27 (m, 3H) |
| 41 | {(3-Benzyloxybenzyl)-[3-(2-dibenzylaminoethoxy)benzoyl]amino}acetic acid ethyl ester; ¹H-NMR: 7.42-6.89 (m, 23H), 5.07-5.03 (m, 2H), 4.74-4.53 (m, 2H), 4.30-3.82 (m, 6H), 3.70 (s, 4H), 2.87 (t, 2H), 1.32-1.19 (m, 3H) |
| 42 | {[3-(2-Dibenzylaminoethoxy)benzoyl]-(3-phenoxybenzyl)amino}acetic acid ethyl ester; ¹H-NMR: 7.40-6.84 (m, 23H), 4.78-4.56 (m, 2H), 4.30-3.85 (m, 6H), 3.70 (s, 4H), 2.86 (t, 2H), 1.33-1.19 (m, 3H) |
| 43 | {[3-(2-Dibenzylaminoethoxy)benzoyl]-(4-phenoxybenzyl)amino}acetic acid ethyl ester; ¹H-NMR: 7.41-6.90 (m, 23H), 4.77-4.57 (m, 2H), 4.30-3.85 (m, 6H), 3.71 (s, 4H), 2.88 (t, 2H), 1.33-1.19 (m, 3H) |
| 44 | {(4-tert-Butylbenzyl)-[3-(2-dibenzylaminoethoxy)benzoyl]amino}acetic acid ethyl ester; ¹H-NMR: 7.41-6.80 (m, 18H), 4.78-4.57 (m, 2H), 4.25-3.42 (m, 10H), 2.87 (m, 2H), 1.32-1.19 (m, 12H) |
| 45 | {[3-(2-Dibenzylaminoethoxy)benzoyl]-(3-benzyloxybenzyl)amino}acetic acid ethyl ester; ¹H-NMR: 7.41-6.80 (m, 23H), 5.07-5.03 (m, 2H), 4.78-4.57 (m, 2H), 4.30-3.68 (m, 10H), 2.86 (m, 2H), 1.33-1.19 (m, 3H) |
| 46 | {(4-Benzyloxybenzyl)-[4-(2-dibenzylaminoethoxy)benzoyl]amino}acetic acid ethyl ester; ¹H-NMR: 7.47-7.23 (m, 19H), 6.97 (d, 2H), 6.79 (d, 2H), 5.08 (s, 2H), 4.71-4.62 (m, 2H), 4.22-3.99 (m, 6H), 3.72 (s, 4H), 2.89 (t, 2H), 1.27 (m, 3H) |
| 47 | 3-(5-Benzyloxy-1*H*-indol-3-yl)-2-[4-(2-dibenzylaminoethoxy)-benzoylamino]propionic acid methyl ester; ¹H-NMR: 8.01 (s, 1H), 7.64 (d, 2H), 7.41-7.22 (m, 14H), 7.00 (dd, 2H), 6.90 (dd, 1H), 6.74 (d, 2H), 6.64 (d, 1H), 5.17 (m, 1H), 4.85 (d, 1H), 4.62 (d, 1H), 3.94 (t, 2H), 3.74 (s, 3H), 3.70 (s, 4H), 3.45 (dd, 1H), 3.35 (dd, 1H), 2.86 (t, 2H) |
| 48 | 2-[4-(2-Dibenzylaminoethoxy)benzoylamino]-3-(1-methyl-1H-indol-3-yl)propionic acid methyl ester; ¹H-NMR: 7.60 (d, 2H), 7.53 (d, 1H), 7.41-7.21 (m, 12H), 7.08 (t, 1H), 6.85 (s, 1H), 6.77 (d, 2H), 6.56 (d, 1H), 5.12 (m, 1H), 4.03 (t, 2H), 3.75-3.72 (m, 10H), 3.43 (d, 2H), 2.90 (t, 2H) |
| 49 | 3-(5-Benzyloxy-1*H*-indol-3-yl)-2-[3-(2-dibenzylaminoethoxy)benzoylamino]-propionic acid methyl ester; ¹H-NMR: 7.95 (s, 1H), 7.41-7.19 (m, 14H), 7.04-6.88 (m, 4H), 6.69 (d, 1H), 5.16 (m, 1H), 4.89 (d, 1H), 4.71 (d, 1H), 3.98 (t, 2H), 3.74 (s, 3H), 3.69 (s, 4H), 3.45 (dd, 1H), 3.35 (dd, 1H), 2.86 (t, 2H) |
| 50 | {(3-Benzyloxybenzyl)-[4-(3-benzyloxybenzyloxy)benzoyl]amino}acetic acid ethyl ester; ¹H-NMR: 7.45-7.28 (m, 14H), 7.06-6.81 (m, 8H), 5.08 (s, 4H), 5.06 (s, 2H), 4.77-4.66 (m, 2H), 4.21 (m, 2H), 4.11-3.88 (m, 2H), 1.26 (m, 3H) |
| 51 | 3-{(3-Benzyloxybenzyl)-[4-(3-benzyloxybenzyloxy)benzoyl]amino}propionic acid ethyl ester; ¹H-NMR: 7.45-7.26 (m, 14H), 6.96-6.82 (m, 8H), 5.08 (s, 4H), 5.05 (s, 2H), 4.63 (br s, 2H), 4.12 (m, 2H), 3.66 (m, 2H), 2.67 (m, 2H), 1.26 (m, 3H) |
| 52 | 3-{(4-Benzyloxybenzyl)-[4-(3-benzyloxybenzyloxy)benzoyl]amino}propionic acid ethyl ester; ¹H-NMR: 7.46-7.27 (m, 13H), 7.13-6.93 (m, 9H), 5.08 (s, 2H), 5.07 (s, 2H), 5.06 (s, 2H), 4.60 (br s, 2H), 4.13 (m, 2H), 3.65 (m, 2H), 2.67 (m, 2H), 1.27 (m, 3H) |
| 53 | {(4-Benzyloxybenzyl)-[3-(3-benzyloxybenzyloxy)benzoyl] amino}acetic acid ethyl ester; ¹H-NMR: 7.42-7.28 (m, 13H), 7.11-6.95 (m, 9H), 5.07-4.99 (m, 6H), 4.75-4.53 (m, 2H), 4.23 (q, 2H) , 4.13-3.84 (m, 2H), 1.26 (m, 3H) |
| 54 | {(3-Benzyloxybenzyl)-[3-(3-benzyloxybenzyloxy)benzoyl]amino}acetic acid ethyl ester; ¹H-NMR: 7.46-7.26 (m, 13H), 7.11-6.80 (m, 9H), 5.09-4.94 (m, 6H), 4.79-4.57 (m, 2H), 4.23 (q, 2H), 4.14-3.84 (m, 2H), 1.26 (m, 3H) |
| 55 | 3-{(3-Benzyloxybenzyl)-[3-(3-benzyloxybenzyloxy)benzoyl]amino}propionic acid ethyl ester; ¹H-NMR: 7.45-7.25 (m, 13H), 7.02-6.90 (m, 7H), 6.78 (m, 2H), 5.07-4.94 (m, 6H), 4.75-4.52 (m, 2H), 4.15 (m, 2H), 3.71-3.50 (m, 2H), 2.72-2.39 (m, 2H), 1.26 (m, 3H) |
| 56 | 3-[(4-Benzyloxybenzoyl)-(3-benzyloxybenzyl)amino]propionic acid ethyl ester; MS: 524 |
| 57 | 3-{(4-Benzyloxybenzyl)-[3-(3-benzyloxybenzyloxy)benzoyl]amino}propionic acid ethyl ester; MS: 631 |
| 58 | (2*S*)-2-[3-(4-Butylbenzyloxy)benzoylamino]-3-cyclohexylpropionic acid methyl ester; ¹H-NMR: 7.45 (s, 1H), 7.35 (d, 4H), 7.20 (d, 2H), 7.12 (m, 1H), 6.47 (d, 1H), 5.06 (s, 2H), 4.87 (m, 1H), 3.76 (s, 1H), 2.62 (t, 2H), 1.78-0.90 (m, 20H) |
| 59 | 2-[4-(3-Benzyloxybenzyloxy)benzoylamino]-3-(4-bromophenyl)propionic acid methyl ester; ¹H-NMR: 7.69 (d, 2H), 7.45-7.20 (m, 8H), 7.06-6.93 (m, 7H), 6.50 (d, 1H), 5.09-5.03 (m, 5H), 3.77 (s, 3H), 3.25 (dd, 1H), 3.16 (dd, 1H) |
| 60 | 2-[4-(3-Benzyloxybenzyloxy)benzoylamino]-3-(4-fluorophenyl)propionic acid methyl ester; ¹H-NMR: 7.69 (d, 2H), 7.45-7.20 (m, 6H), 7.11-6.93 (m, 9H), 6.49 (d, 1H), 5.09-5.02 (m, 5H), 3.76 (s, 3H), 3.27 (dd, 1H), 3.18 (dd, 1H) |
| 61 | 3-(4-Benzyloxyphenyl)-2-[4-(2-naphthalen-2-yl-ethoxy)benzoylamino]propionic acid methyl ester; MS: 560 |
| 62 | 3-{[4-(2-Dibenzylaminoethoxy)benzoyl]-(3-phenoxybenzyl)amino}propionic acid methyl ester; ¹H-NMR: 7.40-7.23 (m, 16H), 7.13 (t, 1H), 7.01 (d, 2H), 6.90 (dd, 2H), 6.75 (d, 2H), 4.60 (br s, 2H), 4.01 (t, 2H), 3.71-3.62 (m, 9H), 2.88 (t, 2H), 2.67 (m, 2H) |
| 63 | 3-{[3-(2-Dibenzylaminoethoxy)benzoyl]naphthalen-2-ylmethylamino}propionic acid methyl ester; ¹H-NMR: 7.80 (m, 3H), 7.50-7.47 (m, 2H) , 7.32-7.22 (m, 13H), 7.00 (d, 1H), 6.93 (s, 1H), 6.84 (d, 1H), 4.93-4.70(m, 2H), 4.03-3.91 (t, 2H), 3.80-3.55 (m, 9H), 2.81-2.50 (m, 4H) |
| 64 | 3-{(4-tert-Butylbenzyl)-[3-(2-dibenzylaminoethoxy)benzoyl]amino}propionic acid methyl ester; ¹H-NMR: 7.39-7.20 (m, 14H), 7.10 (m, 1H), 6.95 (d, 1H), 6.91 (s, 1H), 6.85 (d, 1H), 4.72-4.50 (m, 2H), 3.97 (m, 2H), 3.70-3.55 (m, 9H), 2.86 (m, 2H), 2.71-2.48 (m, 2H), 1.30 (s, 9H) |
| 65 | 3-{(4-Bromobenzyl)-[3-(2-dibenzylaminoethoxy)benzoyl]amino}propionic acid methyl ester; ¹H-NMR: 7.47 (d, 2H), 7.39-7.20 (m, 12H), 7.05 (m, 1H), 6.93 (d, 1H), 6.86-6.80 (m, 2H), 4.68-4.50 (m, 2H), 3.97 (m, 2H), 3.70-3.55 (m, 9H), 2.87 (m, 2H), 2.72-2.46 (m, 2H) |
| 66 | 3-{[3-(2-Dibenzylaminoethoxy)benzoyl]-(3-phenoxybenzyl)amino}propionic acid methyl ester; ¹H-NMR: 7.45-7.22 (m, 16H), 7.11 (t, 1H), 7.00 (m, 2H), 6.90-6.84 (m, 4H), 4.72-4.49 (m, 2H), 3.96 (m, 2H), 3.70-3.60 (m, 9H), 2.87 (m, 2H), 2.72-2.45 (m, 2H) |
| 67 | 3-{[4-(2-Dibenzylaminoethoxy)benzoyl]-(4-phenoxybenzyl)amino}propionic acid methyl ester; ¹H-NMR: 7.40-7.14 (m, 17H), 7.02-6.97 (m, 4H), 6.78 (d, 2H), 4.61 (br s, 2H), 4.01 (t, 2H), 3.71-3.65 (m, 9H), 2.89 (t, 2H), 2.66 (m, 2H) |
| 68 | (2*S*)-2-[4-(4-Butylbenzyloxy)benzoylaminol-3-phenylpropionic acid methyl ester; MS: 446 |
| 69 | (2*S*)-3-(4-Benzyloxyphenyl)-2-[4-(4-butylbenzyloxy)benzoylamino]propionic acid methyl ester; MS: 552 |
| 70 | (2*S*)-2-[4-(4-Butylbenzyloxy)benzoylamino]-3-cyclohexylpropionic acid methyl ester; MS: 452 |
| 71 | {(3-Benzyloxybenzyl)-[4-(4-butylbenzyloxy)benzoyl]amino}acetic acid methyl ester; ¹H-NMR: 7.45-7.18 (m, 12H), 6.93-6.80 (m, 5H), 5.07 (s, 2H), 5.03 (s, 2H), 4.74-4.65 (m, 2H), 4.11-3.90 (m, 2H), 3.75 (s, 3H), 2.62 (t, 2H), 1.59 (m, 2H), 1.35 (m, 2H), 0.92 (t, 3H) |
| 72 | {(4-Benzyloxybenzyl)-[4-(4-butylbenzyloxy)benzoyl]amino}acetic acid methyl ester; ¹H-NMR: 7.50-7.12 (m, 13H), 6.95 (d, 4H), 5.07 (s, 2H), 5.03 (s, 2H), 4.70-4.62 (m, 2H), 4.11-3.92 (m, 2H), 3.74 (s, 3H), 2.61 (t, 2H), 1.59 (m, 2H), 1.35 (m, 2H), 0.92 (t, 3H) |
| 73 | 3-{(3-Benzyloxybenzyl)-[4-(4-butylbenzyloxy)benz yl]amino}propionic acid methyl ester; MS: 566 |
| 74 | 3-{(4-Benzyloxybenzyl)-[4-(4-butylbenzyloxy) benzoyl]amino}propionic acid methyl ester; ¹H-NMR: 7.45-7.13 (m, 13H), 6.95 (d, 4H), 5.06 (s, 2H), 5.03 (s, 2H), 4.58 (br s, 2H), 3.66-3.60 (m, 5H), 2.62 (m, 4H), 1.59 (m, 2H), 1.35 (m, 2H), 0.92 (t, 3H) |
| 75 | (2*S*)-2-[4-(3-Benzyloxybenzyloxy)benzoylaminol-3-cyclohexylpropionic acid methyl ester; ¹H-NMR: 7.72 (d, 2H), 7.44-7.28 (m, 6H), 7.06-6.92 (m, 5H), 6.40 (d, 1H), 5.09 (s, 2H), 5.07 (s, 2H), 4.87 (s, 1H), 3.76 (s, 1H), 1.77-0.95 (m, 13H) |
| 76 | (2*S*)-2-[3-(3-Benzyloxybenzyloxy) benzoylamino]-3-cyclohexylpropionic acid methyl ester; ¹H-NMR: 7.44-7.28 (m, 9H), 7.10-6.93 (m, 4H), 6.50 (d, 1H), 5.08 (s, 4H), 4.87 (s, 1H), 3.76 (s, 1H), 1.81-0.91 (m, 13H) |
| 77 | (2*S*)-3-(4-Benzyloxyphenyl)-2-[3-(4-butylbenzyloxy)benzoylamino]propionic acid methyl ester; MS: 552 |
| 78 | (2*S*)-2-[3-(4-Benzyloxybenzyloxy)benzoylamino]-3-phenylpropionic acid methyl ester; MS: 496 |
| 79 | (2*S*)-2-[3-(4-Benzyloxybenzyloxy)benzoylamino]-3-cyclohexylpropionic acid methyl ester; MS: 502 |
| 80 | {(3-Benzyloxybenzyl)-[3-(4-butylbenzyloxy)benzoyl]amino}acetic acid methyl ester; ¹H-NMR: 7.41-7.16 (m, 12H), 7.10-6.80 (m, 5H), 5.06-4.92 (m, 4H), 4.78-4.57 (m, 2H), 4.14-3.90 (m, 2H), 3.77-3.68 (m, 3H), 2.61 (t, 2H), 1.59 (m, 2H), 1.35 (m, 2H), 0.93 (t, 3H) |
| 81 | 3-{(3-Benzyloxybenzyl) - [3- (4-butylbenzyloxy)benzoyl]amino}propionic acid methyl ester; ¹H-NMR: 7.41-7.17 (m, 10H), 7.01-6.77 (m, 7H), 5.05-5.04 (m, 4H), 4.91-4.71 (m, 2H), 4.66-4.48 (m, 2H), 3.69-3.60 (m, 3H), 2.72 (m, 2H), 2.61 (t, 2H), 1.59 (m, 2H), 1.35 (m, 2H), 0.93 (t, 3H) |
| 82 | (2*S*)-[3-(4-Benzyloxybenzyloxy)benzoylamino]phenyl acetic acid methyl ester; MS: 482 |
| 83 | (2*S*)-2-{4-[2-(3-Methylquinoxalin-2-yloxy)ethoxy]benzoylamino}-3-phenylpropionic acid methyl ester; MS: 486 |
| 84 | (2*S*)-[3-(3-Benzyloxybenzyloxy)benzoylamino]phenyl acetic acid methyl ester; MS: 482 |
| 85 | (2*S*)-2-[3-(3-Benzyloxybenzyloxy)benzoylamino]-3-phenylpropionic acid methyl ester; MS: 496 |
| 86 | {(3-Benzyloxybenzyl)-3-4-benzyloxybenzyloxy)benzoyl]amino}acetic acid methyl ester; ¹H-NMR: 7.45-7.25 (m, 14H), 7.10-6.75 (m, 8H), 5.08-4.89 (m, 6H), 4.78-4.57 (m, 2H), 4.14-3.85 (m, 2H), 3.77-3.68 (m, 3H) |
| 87 | 3-{(3-Benzyloxybenzyl)-[3-(4-benzyloxybenzyloxy)benzoyl]amino}propionic acid methyl ester; ¹H-NMR: 7.45-7.24 (m, 14H), 7.00-6.77 (m, 8H), 5.08-4.88 (m, 6H), 4.71-4.51 (m, 2H), 3.69-3.50 (m, 5H), 2.72-2.39 (m, 2H) |
| 88 | (2*S*)-[4-(4-Butylbenzyloxy)benzoylamino]phenylacetic acid methyl ester; MS: 432 |
| 89 | (2*S*) -3- (4-Benzyloxyphenyl) -2- [4- (2-pyridin-2-yl-ethoxy)benzoylamino]propionic acid methyl ester; MS: 511 |
| 90 | {[4-(3-Benzyloxybenzyloxy)benzoyl]-(4-benzyloxyphenyl)amino}acetic acid ethyl ester; MS: 602 |
| 91 | {[4-(3-Benzyloxybenzyloxy)benzoyl]-(3-benzyloxyphenyl)amino}acetic acid ethyl ester; MS: 602 |
| 92 | 3-(4-Benzyloxyphenyl)-2-{3-[(benzylphenethylcarbamoyl)methoxy]benzoylamino}p ropionic acid methyl ester; ¹H-NMR: 7.41-7.23 (m, 16H), 7.16-7.05 (m, 5H), 6.98-6.98 (m, 2H), 6.60-6.55 (m,14H), 5.06-4.93 (m, 3H), 4.70 (d, 1H), 4.43 (d, 1H), 3.76 (s, 3H), 3.64-3.40 (m, 2H), 3.26-3.11 (m, 2H), 2.85 (t, 2H); MS: 657 |
| 93 | {3-[(Benzylphenylcarbamoyl)methoxybenzoylamino}thiophen-3-ylacetic acid methyl ester; ¹H-NMR: 7.40-7.31 (m, 5H), 7.29-7.15 (m, 6H), 7.15-7.09 (m, 1H), 7.09-6.95 (m, 4H), 5.88 (d, 1H),5.30 (s, 1H), 4.90 (s, 2H), 4.43 (s, 2H), 3.80 (s, 3H); MS: 515 |

The compounds of formula (Iaa), (Iab) and (Iae) shown in Table 13 were synthesized according to any of methods D to F, starting from intermediate (VIII) and the corresponding alcohols, thiols or amines:

**TABLE 13**

| Ex. | |
|---|---|
| 94 | (2*S*)-3-(4-Benzyloxyphenyl)-2-{4-[2-(3-bromophenoxy)ethoxy]-benzoylamino}propionic acid methyl ester; ¹H-NMR: 7.72 (d, 2H), 7.42-6.88 (m, 15H), 6.51 (d, 1H), 5.10-5.03 (m, 3H), 4.33 (s, 4H), 3.77 (s, 3H), 3.29-3.11 (m, 2H) |
| 95 | (2*S*)-3-(4-Benzyloxyphenyl)-2-{4-[2-(3-methylquinoxalin-2-yloxy)ethoxy]benzoylamino}propionic acid methyl ester; ¹H-NMR: 7.99-6.87 (m, 17H), 6.54 (d, 1H), 5.10-5.02 (m, 3H), 4.88 (t, 2H), 4.47 (t, 2H), 3.77 (s, 3H), 3.29-3.11 (m, 2H), 2.64 (s, 3H) |
| 96 | (2*S*)-3-(4-Benzyloxyphenyl)-2-{3-[2-(2,6-dimethylphenoxy)ethoxy]-benzoylamino}propionic acid methyl ester; ¹H-NMR: 7.44-7.36 (m, 9H), 7.14-6.90 (m, 7H), 6.57 (d, 1H), 5.07-5.03 (m, 3H), 4.36-4.33 (m, 2H), 4.18-4.15 (m, 2H), 3.78 (s, 3H), 3.26-3.10 (m, 2H), 2.32 (s, 6H) |
| 97 | (2*S*)-3-(4-Benzyloxyphenyl)-2-{4-[2-(pyridin-2-yloxy)ethoxy]-benzoylamino}propionic acid methyl ester; MS: 527 |
| 98 | (2*S*)-3-(4-Benzyloxyphenyl)-2-{4-[2-(quinolin-8-yloxy)ethoxy]-benzoylamino}propionic acid methyl ester; MS: 577 |
| 99 | (2*S*)-3-(4-Benzyloxyphenyl)-2-{4-[2-(4-imidazol-1-yl-phenoxy)ethoxy]benzoylamino}propionic acid methyl ester; MS: 592 |
| 100 | (2*S*)-3-(4-Benzyloxyphenyl)-2-{4-[2-(2-methylbenzothiazol-5-yloxy)ethoxylbenzoylamino}propionic acid methyl ester; MS: 597 |
| 101 | (2*S*)-3-(4-Benzyloxyphenyl)-2-{4-[2-(5,6,7,8-tetrahydronaphthalen-2-yloxy)ethoxy]benzoylamino}propionic acid methyl ester; MS: 580 |
| 102 | (2*S*)-3-(4-Benzyloxyphenyl)-2-{4- [2-(quinolin-7-yloxy) ethoxy]-benzoylamino}propionic acid methyl ester; MS: 577 |
| 103 | (2*S*)-3-(4-Benzyloxyphenyl)-2-{4-[2-(quinolin-2-yloxy) ethoxy]-benzoylamino}propionic acid methyl ester; MS: 577 |
| 104 | (2*S*)-3-(4-Benzyloxyphenyl)-2-{4-[3-(3-methylquinoxalin-2-yloxy)propoxy]benzoylamino}propionic acid methyl ester; MS: 606 |
| 105 | (2*S*)-3-(4-Benzyloxyphenyl)-2-{4-[2-(1-methyl-1*H*-i midazol-2-ylsulfanyl)ethoxy]benzoylamino}propionic acid; MS: 532 |
| 106 | (2*S*) -3- (4-Benzyloxyphenyl) -2-{4- [2- (2-fluorophenylsulfanyl)ethoxy]-benzoylamino}propionic acid methyl ester; ¹H-NMR: 7.66 (d, 2H), 7.50-7.25 (m, 7H), 7.25-7.08 (m, 2H), 7.03 (d, 2H), 6.90 (d, 2H), 6.86 (d, 2H), 6.45 (d, 1H), 5.08-6.98 (m, 3H), 4.17 (t, 2H), 3.76 (s, 3H), 3.28 (t, 2H), 3.25-3.10 (m, 2H); MS: 560 |
| 107 | (2*S*)-2-{4-(2-(4-Bromophenylsulfanyl)ethoxylbenzoylamino}-3-cyclohexylpropionic acid methyl ester; ¹H-NMR: 7.74 (d, 2H), 7.43 (d, 2H), 7.28 (d, 2H), 6.87 (d, 2H), 6.39 (d, 1H), 4.95-4.80 (m, 1H), 4.17 (t, 2H), 3.76 (s, 3H), 3.28 (t, 2H), 1.90-1.50 (m, 5H), 1.50-1.20 (m, 5H), 1.20-0.85 (m, 3H) ; MS: 521 |
| 108 | (2*S*)-3-Cyclohexyl-2-{4-[2-(2-methoxyphenylsulfanyl)ethoxy]-benzoylamino}propionic acid methyl ester; ¹H-NMR: 7.73 (d, 2H), 7.38 (d, 1H), 7.30-7.20 (m, 1H), 7.00-6.83 (m, 4H), 6.39 (d, 1H), 4.95-4.80 (m, 1H), 4.17 (t, 2H), 3.89 (s, 3H), 3.76 (s, 3H), 3.28 (t, 2H), 1.90-1.50 (m, 5H), 1.50-1.20 (m, 5H), 1.20-0.85 (m, 3H); MS: 472 |
| 109 | (2*S*)-3-(4-Benzyloxyphenyl)-2-{4-[2-(2-methoxyphenoxy)ethoxy]-benzoylamino}propionic acid methyl ester; ¹H-NMR: 7.70 (d, 2H), 7.48-7.30 (m, 4H), 7.20 (t, 1H), 7.04 (d, 2H), 6.97 (d, 2H), 6.90 (d, 2H), 6.60-6.50 (m, 4H), 6.48 (d, 1H), 5.10-5.00 (m, 3H), 4.40-4.28 (m, 4H), 3.79 (s, 3H), 3.76 (s, 3H), 3.26-3.12 (m, 2H); MS: 556 |
| 110 | (2*S*)-2-[4-(2*-N-*benzylaminoethoxy)benzoylamino]-3-(4-benzyloxyphenyl)propionic acid methyl ester; ³H-NMR: 7.68 (d, 2H), 7.41-7.30 (m, 10H), 7.05 (d, 2H), 6.90 (d, 4H), 6.51 (d, 1H), 5.10-5.00 (m, 3H), 4.14 (t, 2H), 3.90 (s, 2H), 3.76 (s, 3H), 3.19 (m, 2H) 3.06 (t, 2H) |

The compounds of formula (Iaa) y (Iab) shown in Table 14 were synthesized according to methods A or C, starting from intermediate (Xb) or (XIb):

**TABLE 14**

| Ex. | |
|---|---|
| 111 | ((4-Benzyloxybenzyl)-{4-[2-(3-methylquinoxalin-2-yloxy)ethoxy]benzoyl}amino)acetic acid ethyl ester; ¹H-NMR: 7.94 (d, 1H), 7.80 (d, 1H), 7.63-7.52 (m, 4H), 7.46-7.31 (m, 6H), 7.14 (d, 1H), 7.00-6.96 (m, 4H), 5.07 (s, 2H), 4.86 (t, 2H), 4.73-4.62 (m, 2H), 4.44 (t, 2H), 4.30-4.18 (m, 2H), 4.11-3.89 (m, 2H), 2.64 (s, 3H), 1.26 (m, 3H) |
| 112 | (2*S*)-3-(4-Benzyloxyphenyl)-2-{3-[2-(3-methylquinoxalin-2-yloxy)ethoxy]benzoylamino}propionic acid methyl ester; ¹H-NMR: 7.94 (dd, 1H), 7.81 (d, 1H), 7.60-7.52 (m, 2H), 7.43-7.25 (m, 8H), 7.13 (dd, 1H), 7.04 (d, 2H), 6.90 (d, 2H), 6.57 (d, 1H), 5.08-5.03 (m, 3H), 4.87 (t, 2H), 4.48 (t, 2H), 3.77 (s, 3H), 3.28-3.14 (m, 2H), 2.64 (s, 3H) |
| 113 | 3-((3-Benzyloxybenzyl)-{3-[2-(3-methylquinoxalin-2-yloxy)ethoxy]benzbyl}amino)propionic acid ethyl ester; ¹H-NMR: 7.95 (d, 1H), 7.79 (d, 1H), 7.64-7.52 (m, 2H), 7.41-7.25 (m, 8H), 7.03-6.78 (m, 5H), 5.04 (s, 2H), 4.81 (m, 2H), 4.55-4.00 (m, 6H), 3.72 (m, 2H), 2.72-2.63 (m, 5H), 1.27 (m, 3H) |
| 114 | ((3-Benzyloxybenzyl)-{4-[2-(3-methylquinoxalin-2-yloxy)ethoxy]benzoyl}amino)acetic acid ethyl ester; ¹H-NNBt: 7.94 (d, 1H), 7.80 (d, 1H), 7.61-7.29 (m, 10H), 6.97-6.82 (m, 5H), 5.08 (s, 2H), 4.86 (t, 2H), 4.78-4.66 (m, 2H), 4.44 (t, 2H), 4.30-4.18 (m, 2H), 4.12-3.89 (m, 2H), 2.64 (s, 3H), 1.28 (m, 3H) |
| 115 | 3-((3-Benzyloxybenzyl)-{4-[2-(3-methylquinoxalin-2-yloxy)ethoxy]benzoyl}amino)propionic acid ethyl ester; ¹H-NMR: 7.94 (d, 1H), 7.80 (d, 1H), 7.64-7.52 (m, 2H), 7.45-7.24 (m, 8H), 6.97-6.78 (m, 5H), 5.07 (s, 2H), 4.86 (t, 2H), 4.62 (br s, 2H), 4.43 (t, 2H), 4.20-4.05 (m, 2H), 3.67 (m, 2H), 2.75-2.64 (s, 5H), 1.25 (m, 3H) |
| 116 | ((3-Benzyloxybenzyl) - {3- [2- (3-methylquinoxalin-2-yloxy)ethoxy]benzoyl}amino)acetic acid ethyl ester; ¹H-NMR: 7.94 (d, 1H) , 7.81 (d, 1H) , 7.65-7.50 (m, 2H), 7.43-7.25 (m, 8H), 7.12-6.80 (m, 5H), 5.05 (m, 2H), 4.86-4.79 (m, 2H), 4.60-4.53 (m, 2H), 4.44-4.30 (m, 2H), 4.24-4.15 (m, 2H), 4.14-3.85 (m, 2H), 2.63 (s, 3H), 1.32-1.20 (m, 3H) |
| 117 | (2*S*)-3-(4-Bromophenyl)-2-{4-[2-(3-methylquinoxalin-2-yloxy)ethoxy]benzoylamino}propionic acid methyl ester; ¹H-NMR: 7.94 (dd, 1H) , 7.81 (d, 1H), 7.72 (d, 2H), 7.60-7.52 (m, 2H), 7.40 (d, 2H), 7.02-6.98 (m, 4H), 6.52 (d, 1H), 5.06 (m, 1H), 4.88 (t, 2H), 4.47 (t, 2H), 3.77 (s, 3H), 3.26 (dd, 1H), 3.17 (dd, 1H), 2.64 (s, 3H) |
| 118 | (2*S*)-3-(4-Fluorophenyl)-2-{4-[2-(3-methylquinoxali n-2-yloxy) ethoxy] benzoylamino}propionic acid methyl ester; ¹H-NMR: 7.92 (d, 1H), 7.80 (d, 1H), 7.72 (d, 2H), 7.65-7.52 (m, 2H), 7.11-6.94 (m, 6H), 6.51 (d, 1H), 5.06 (m, 1H), 4.88 (t, 2H), 4.47 (t, 2H), 3.77 (s, 3H), 3.27 (dd, 1H), 3.18 (dd, 1H), 2.64 (s, 3H) |
| 119 | (2*S*)-3'-Cyclohexyl-2-{4-[2-(3-methylquinoxalin-2-yloxy)ethoxy]benzoylamino}propionic acid methyl ester; ¹H-NMR: 7.94 (d, 1H), 7.81-7.77 (m, 3H), 7.64-7.51 (m, 2H), 7.02 (d, 2H), 6.44 (d, 1H), 4.90-4.83 (m, 3H), 4.47 (t, 2H), 3.76 (s, 3H), 2.64 (s, 3H), 1.90-0.95 (m, 13H) |
| 120 | (2*S*)-3-Cyclohexyl-2-{3-[2-(3-methylquinoxalin-2-yloxy)ethoxy]benzoylamino}propionic acid methyl ester; ¹H-NMR: 7.93 (d, 1H), 7.80 (d, 1H), 7.64-7.51 (m, 2H), 7.46 (s, 1H), 7.36 (d, 2H), 7.12 (m, 1H), 6.54 (d, 1H), 4.90-4.83 (m, 3H), 4.47 (t, 2H), 3.76 (s, 3H), 2.63 (s, 3H), 1.90-0.95 (m, 13H) |
| 121 | {Thiophen-3-ylmethyl-{3-[2-(thiophen-2-ylsulfanyl)ethoxy]benzoyl}amino} acetic acid methyl ester; ¹H-NMR: 7.40-7.25 (m, 4H), 7.25-7.05 (m, 2H), 7.05-6.85 (m, 2H), 7.78-4.55 (m, 2H), 4.20-4.04 (m, 4H), 3.78-3.65 (m, 2H), 3.15-3.05 (m, 2H); MS : 448 |
| 122 | {Thiophen-2-yl{3-[2-(thiophen-2-ylsulfanyl)ethoxy] benzoyl}amino}acetic acid methyl ester; ¹H-NMR: 7.40-7.25 (m, 4H), 7.25-7.05 (m, 2H), 7.05-6.90 (m, 2H), 6.05 (d, 1H), 4.17 (t, 2H) , 4.00-3.54 (m, 4H), 3.14 (t, 2H); MS: 434 |
| 123 | (2*S*)-3-(4-Benzyloxyphenyl)-2-{3-[2-(3-methyl-2-oxo-2H-quinoxalin-1-yl)ethoxy]benzoylamino}propionic acid methyl ester; MS: 592. |
| 124 | (2*S*)-3-((4-Benzyloxybenzyl)-{3-[2-(3-methyl-2-oxo-2*H*-quinoxalin-1-yl)ethoxy]benzoyl}amino)propionic acid methyl ester; MS: 606 |

The compounds of formula (Ia) shown in Table 15 were synthesized according to methods N or P, starting from compounds of formula (Ib)

### METHOD N:

To a solution of 1 eq of the compound of formula (Ib), 1.3 eq of HOBT and 1.3 eq of EDC in tetrahydrofurane, the solution being 0.2 M in the compound of formula (Ib), 2 eq of triethylamine and 5 eq of the corresponding alcohol were added. The reaction mixture was stirred at room temperature for 18h, and then water and dichloromethane were added. The organic layer was separated, and the aquous layer was extracted once with dichloromethane. The organic layers were combined, dried over anhydrous sodium sulfate, and filtered. The solvent was distilled off under reduced pressure.

### METHOD P:

1 Eq of the compound of formula (Ib) was dissolved in the corresponding alcohol, and 2 drops of H₂SO₄ conc. were added. The solution was stirred over night at room temperature, and the solvent was distilled off at reduced pressure.

**TABLE 15**

| Ex. | |
|---|---|
| 125 | (2*S*)-3-(4-Benzyloxyphenyl)-2-{4-[2-(3-methylquinoxalin-2-yloxy)ethoxy]benzoylamino}propionic acid ethyl ester; ¹H-NMR: 7.95 (d, 1H), 7.82 (d, 1H), 7.73 (d, 2H), 7.65-7.52 (m, 2H), 7.45-7.30 (m, 5H), 7.05 (d, 2H), 7.00 (d, 2H), 6.89 (d, 2H), 6.52 (d, 1H), 5.03 (m, 3H), 4.88 (t, 2H), 4.47 (t, 2H), 4.22 (q, 2H), 3.20 (m, 2H), 2.65 (s, 3H), 1.29 (t, 3H) |
| 126 | (2*S*)-3-(4-Benzyloxyphenyl)-2-{4-[2-(3-methylquinoxalin-2-yloxy)ethoxy]benzoylamino}propionic acid isopropyl ester; MS: 620 |
| 127 | (2*S*)-3-(4-Benzyloxyphenyl)-2-{4-[2-(3-methylquinoxalin-2-yloxy)ethoxy]benzoylamino}propionic acid propyl ester; MS: 620 |

### EXEMPLE (Ib):

### METHOD Q:

To a solution 0.1 M of 1 eq of the corresponding acid chloride in tetrahydrofurane or dioxane, an aqueous solution of 1 eq of the aminoacidic derivative, and 2 eq of sodium hydroxide was added. The resulting mixture was stirred for 18h at room temperature. Then, HCl 1 N was added dropwise until pH acid was reached, and the solution was extracted twice with ethyl acetate. The combined organic layers were dried over anhydrous sodium sulfate, and filtered. The solvent was distilled off under reduced pressure, and the obtained residue was purified by column chromatography.

### METHOD R:

To a mixture of 1 eq of acid of formula (II), 1.3 eq of HOBT and 1.3 eq of EDC, tetrahydrofurane or dioxane were added, and the resulting solution (0.2 M in the acid of formula (II)) was stirred during 2h at room temperature. Then, an aquous solution of 1 eq of the aminoacidic derivative, and 2 eq of sodium hydroxide was added. The solution was stirred over night at room temperature. Then, HCl 1 N was added dropwise until pH acid was reached, and the solution was extracted twice with ethyl acetate. The combined organic layers were dried over anhydrous sodium sulfate, and filtered. The solvent was distilled off under reduced pressure, and the obtained residue was purified by column chromatography.

The compounds of formula (Ib) shown in Table 16 were synthesized according to methods Q or R, starting from intermediates. (VIb) :

**TABLE 16**

| Ex. | |
|---|---|
| 128 | (2*S*)-3-(4-Benzyloxyphenyl)-2-{4-[2-(5-methyl-2-phenyloxazol-4-yl)ethoxy]benzoylamino}propionic acid; ¹H-NMR: 7.90-6.80 (m, 18H), 5.00 (s, 2H), 4.45 (m, 1H), 4.26 (m, 2H), 3.40 (m, 2H), 2.95 (m, 2H), 2.35 (s, 3H) |
| 129 | (2*S*)-2-(4-Benzyloxybenzoylamino)-3-(4-benzyloxyphe nyl) propionic acid; ¹H-NMR: 7.66 (d, 2H), 7.37-7.31 (m, 10H), 7.08 (d, 2H); 6.95 (d, 2H), 6.86 (d, 2H), 5.07 (s, 2H), 4.99 (s, 2H), 4.93 (t, 1H), 3.23-3.15 (m, 2H) |
| 130 | (2*S*)-3-(4-Benzyloxyphenyl)-2-[4-(2-dibenzylaminoethoxy)benzoylamino]propionic acid; ¹H-NMR: 8.00-6.75 (m, 24H), 4.88 (m, 3H), 4.15 (t, 2H), 3.98 (s, 4H), 3.35-3.14 (m, 2H), 3.08 (t, 2H) |
| 131 | (2*R*)-2-[4-(2-Dibenzylaminoethoxy)benzoylamino]-3-(1*H*-indol-3-yl)propionic acid; ¹H-NMR: 7.72-6.74 (m, 20H), 4.80 (m, 1H), 4.02 (t, 2H), 3.72 (s, 4H), 3.55-3.25 (m, 2H), 2.89 (t, 2H) |
| 132 | 3- (4-*tert*-Butylphenyl)-2-[4-(2-dibenzylaminoethoxy)benzoylaminolpropionic acid; ¹H-NMR: 7.62 (d, 2H), 7.50-7.11 (m, 14H), 6.83 (d,1H), 6.76 (d, 2H), 4.91 (m, 1H), 4.10 (t, 2H), 3.94 (s, 4H), 3.38-3.16 (m, 2H), 3.05 (t, 2H), 1.20 (s, 9H) |
| 133 | 3-(4-Bromophenyl)-2-[4-(2-dibenzylaminoethoxy)benzoylamino]propionic acid; ¹H-NMR: 7.62 (d, 2H), 7.43-7.24 (m, 12H), 7.04 (d, 2H), 6.85 (d, 1H), 6.77 (d, 2H), 4.86 (m, 1H), 4.12 (t, 2H), 3.98 (s, 4H), 3.36-3.13 (m, 2H), 3.07 (t, 2H) |
| 134 | 3-Biphenyl-2-yl-2-[4-(2-dibenzylaminoethoxy)benzoylamino]propionic acid; ¹H-NMR: 7.46-7.17 (m, 21H), 6.72 (d, 2H), 6.36 (d, 1H), 4.66 (m, 1H), 4.07 (t, 2H), 3.84 (s, 4H), 3.44-3.09 (m, 2H), 2.98 (t, 2H) |
| 136 | 3-Biphenyl-4-yl-2-[4-(2-dibenzylaminoethoxy)benzoylamino]propionic acid; ¹H-NMR: 7.63 (d, 2H) , 7.45-7.22 (m, 19H), 6.93 (d, 1H), 6.74 (d, 2H), 4.95 (m, 1H), 4.08 (t, 2H), 3.93 (s, 4H), 3.44-3.23 (m, 2H), 3.03 (t, 2H) |
| 137 | 3-(4-tert-Butylphenyl)-2-[3-(2-dibenzylaminoethoxy)benzoylamino]propionic acid; ¹H-NMR: 7.45-6.82 (m, 19H), 4.90 (m, 1H), 4.19 (t, 2H), 3.96 (s, 4H), 3.36-3.02 (m, 4H), 1.16 (s, 9H) |
| 138 | 3-(4-Bromophenyl)-2-[3-(2-dibenzylaminoethoxy)benzoylamino]propionic acid; 1H-NMR: 7.52-6.85 (m, 19H), 4.98 (m, 1H), 4.20 (t, 2H), 3.99 (s, 4H), 3.51-3.03 (m, 4H) |
| 139 | 3-(3-Bromophenyl)-2-[3-(2-dibenzylaminoethoxy)benzoylamino]propionic acid; ¹H-NMR: 7.60-6.88 (m, 19H), 4.83 (m, 1H), 4.29 (t, 2H), 4.11 (s, 4H), 3.32-3.05 (m, 4H) |
| 140 | 3-Biphenyl-2-yl-2-[3-(2-dibenzylaminoethoxy)benzoylamino]propionic acid; ¹H-NMR: 7.50-6.88 (m, 24H), 4.93 (m, 1H), 4.29 (m, 2H), 4.13 (s, 4H), 3.40-3.20 (m, 4H) |
| 141 | 2-[4-(2-Dibenzylaminoethoxy)benzoylamino]-3-(3-phenoxyphenyl)propionic acid; ¹H-NMR: 8.06-6.74 (m, 24H), 4.90 (m, 1H), 4.10 (t, 2H), 3.89 (s, 2H), 3.76 (s, 2H), 3.40-2.92 (m, 4H) |
| 142 | 3-(5-Bromo-1*H*-indol-3-yl)-2-[4-(2-dibenzylaminoethoxy)benzoylamino]propionic acid; ¹H-NMR: 7.81-7.20 (m, 17H), 6.90 (d, 2H), 4.94 (m, 1H), 4.17 (t, 2H), 3.97 (s, 4H), 3.58-3.31 (m, 2H), 3.11 (t, 2H) |
| 143 | 2-[3-(2-Dibenzylaminoethoxy)benzoylamino]-3-(3-phenoxyphenyl)propionic acid; ¹H-NMR: 7.44-6.88 (m, 24H), 4.87 (m, 1H), 4.16 (t, 2H), 3.94 (s, 4H), 3.34-3.03 (m, 4H) |
| 144 | 3- (5-Bromo-1*H*-indol-3-yl) -2-[3-(2-dibenzylaminoethoxy)benzoylamino]propionic acid; ¹H-NMR: 7.79-7.20 (m, 19H), 4.96 (m, 1H), 4.23 (m, 6H), 3.52-3.24 (m, 4H) |
| 145 | (2S) -2-[3-(3-Benzyloxybenzyloxy)benzoylamino] -3- (4 -benzyloxyphenyl)propionic acid; ¹H-NMR: 7.45-7.21 (m,14H), 7.13-7.07 (m, 4H), 7.01 (d, 1H), 6.96-6.89 (m, 3H) , 6.58 (d, 1H), 5.06-4.99 (m, 7H), 3.31 (dd, 1H), 3.21 (dd, 1H) |

The compounds of formula (Ib) shown in Table 17 were synthesized according to methods J or K, starting from compounds of formula (Ia) :

**TABLE 17**

| **Ex.** | |
|---|---|
| 146 | {[4-(2-Dibenzylaminoethoxy)benzoyl]-(3-phenoxybenz yl)amino}acetic acid; ¹H-NMR: 7.66-6.88 (m, 23H) , 4.81-4.68 (m, 2H), 4.57 (s, 4H) , 4.40 (m, 2H), 4.18-4.00 (m, 2H), 3.63 (m, 2H) |
| 147 | (2*S*)-3-(4-Benzyloxyphenyl)-2-[4-(3-phenylallyloxy)benzoylamino]propionic acid; ¹H-NMR: 7.66 (d, 2H), 7.40-6.84 (m, 17H), 6.71 (d, 1H), 6.36 (dt, 1H), 4.98 (s, 2H), 4.92 (t, 1H), 4.70 (d, 2H), 3.30-3.08 (m, 2H) |
| 148 | (2*S*)-3-(4-Benzyloxyphenyl)-2-[4-(4-phenoxybenzyloxy)benzoylamino]propionic acid; ¹H-NMR: 7.62 (d, 2H), 7.34-6.79 (m, 20H), 4.99 (s, 2H), 4.94 (s, 2H), 4.84 (t, 1H), 3.25-3.02 (m, 2H) |
| 149 | (2*S*)-3-(4-Benzyloxyphenyl)-2-[4-(biphenyl-4-ylmethoxy)benzoylamino]propionic acid; ¹H-NMR: 7.82-6.91 (m, 22H), 5.28 (s, 2H), 5.02 (s, 2H), 4.75 (t, 1H), 3.40-3.10 (m, 2H) |
| 150 | (2*S*)-3-(4-Benzyloxyphenyl)-2-[4-(3-phenoxybenzyloxy)benzoylamino]propionic acid; ¹H-NMR: 7.61 (d, 2H), 7.36-6.77 (m, 20H), 4.97 (s, 2H), 4.91 (s, 2H), 4.76 (t, 1H), 3.25-3.02 (m, 2H) |
| 151 | (2*S*)-2-[4-(3-Benzyloxybenzyloxy) benzoylamino]-3-(4-benzyloxyphenyl)propionic acid; ¹H-NMR: 7.61 (d, 2H), 7.34-6.77 (m, 20H), 5.00 (s, 2H), 4.97 (s, 2H), 4.91 (s, 2H), 4.77 (m, 1H), 3.25-3.04 (m, 2H) |
| 152 | (2*S*)-3-(4-Henzyloxyphenyl)-2-(4-phenethyloxybenzoylamino)propionic acid; ¹H-NMR:,63 (d, 2H), 7.39-7.24 (m, 10H), 7.19 (d, 2H), 6.92-6.86 (m, 4H), 6.47 (d, 1H), 5.01-4.92 (m, 3H), 4.19 (t, 2H), 3.26-3.07 (m, 4H) |
| 153 | (2*S*)-3-(4-Benzyloxyphenyl)-2-[4-(3-phenylpropoxy)benzoylamino]propionic acid; ¹H-NMR: 7.66 (d, 2H), 7.39-7.18 (m, 10H), 7.10 (d, 2H), 6.89-6.86 (m, 4H), 5.00 (s, 2H), 4.94 (t, 1H), 4.97 (t, 2H), 3.30-3.13 (m, 2H), 2.80 (t, 2H), 2.10 (m, 2H) |
| 154 | {(4-Benzyloxybenzyl)-[4-(2-dibenzylaminoethoxy)benzoyl]amino}acetic acid; ¹H-NMR: 7.31-6.40 (m, 23H), 4.90-4.50 (m, 4H), 3.90-3.60 (m, 8H), 2.74 (m, 2H) |
| 155 | {(3-Benzyloxybenzyl)-[3-(2-dibenzylaminoethoxy)benzoyl]amino}acetic acid; ¹H-NMR: 7.60-6.91 (m, 23H), 5.04-5.01 (m, 2H), 4.76-3.68 (m, 10H), 3.40-3.20 (m, 2H) |
| 156 | {[3-(2-Dibenzylaminoethoxy)benzoyl]-(3-phenoxybenzyl)amino}acetic acid; ¹H-NMR: 7.58-6.88 (m, 23H), 4.79-3.71 (m, 10H), 3.32-3.17 (m, 2H) |
| 157 | {[3-(2-Dibenzylaminoethoxy)benzoyl]-(4-phenoxybenzyl)amino}acetic acid; ¹H-NMR: 7.61-6.94 (m, 23H), 4.80-3.71 (m, 10H), 3.35-3.15 (m, 2H) |
| 158 | {(4-*tert*-Butylbenzyl)-[3-(2-dibenzylaminoethoxy)benzoyl]amino}acetic acid; 7.65-6.92 (m, 18H), 4.78-3.38 (m, 12H), 1.35-1.20 (m, 9H) |
| 159 | {[3-(2-Dibenzylaminoethoxy)benzoyl]-(3-benzyloxybenzyl)amino}acetic acid; ¹H-NMR: 7.60-6.76 (m, 23H), 5.03-4.99 (m, 2H), 4.74-3.15 (m, 12H) |
| 160 | (2*S*)-3-(4-Benzyloxyphenyl)-2-{4-[2-(3-methylquinoxalin-2-yloxy)ethoxy]benzoylamino}propionic acid; ¹H-NMR: 7.99-6.86 (m, 17H), 6.57 (d, 1H), 4.99 (m, 3H), 4.86 (t, 2H), 4.44 (t, 2H) , 3.39-3.12 (m, 2H), 2.63 (s, 3H) |
| 161 | (2*S*)-3-(4-Benzyloxyphenyl)-2-{4-[2-(3-bromophenoxy)ethoxy]berizoylamino}propionicacid; ¹H-NMR: 7.62 (d, 2H), 7.33-6.79 (m, 15H), 4.95 (s, 2H), 4.83 (t, 1H), 4.26 (s, 4H), 3.25-3.02 (m, 2H) |
| 162 | 3-{(3-Benzyloxybenzyl)-[4-(2-dibenzylaminoethoxy)benzoyl]amino}propionic acid; MS: 629 |
| 163 | 3 - {(3-Benzyloxybenzyl)-[3- (2-dibenzylaminoethoxy)benzoyl]amino}propionic acid; ¹H-NMR: 7.52-7.16 (m, 17H), 7.05-6.62 (m, 6H), 5.03 (s, 2H), 4.75-4.51 (m, 2H), 4.07-3.85 (m, 2H), 3.75-3.66 (m, 6H), 3.14-2.83 (m, 2H), 2.78-2.33 (m, 2H) |
| 164 | 3-{(4-Benzyloxybenzyl)-[3-(2-dibenzylaminoethoxy)benzoyl]amino}propionic acid; ¹H-NMR: 7.38-7.15 (m, 17H), 7.06-6.71 (m, 6H), 4.95 (s, 2H), 4.66-4.38 (m, 2H), 4.10-3.92 (m, 2H), 3.81-3.44 (m, 6H), 2.95-2.73 (m, 2H), 2.54-2.40 (m, 2H) |
| 165 | 2-[4-(2-Dibenzylaminoethoxy)benzoylamino]-3-(1-methyl-1*H*-indol-3-yl)propionic acid; ¹H-NMR: 7.50-7.47 (m, 3H), 7.38-7.24 (m, 9H), 7.09-6.83 (m, 5H), 6.53 (d, 2H), 4.93 (m, 1H), 3.83-3.72 (m, 6H), 3.39 (m, 5H) , 2.83 (m, 2H) |
| 166 | 3-(5-Benzyloxy-1*H*-indol-3-yl)-2-[4-(2-dibenzylaminoethoxy)benzoylamino]propionic acid; ¹H-NMR: 7.40-7.19 (m, 15H), 6.97 (m, 3H) , 6.80-6.72 (m, 2H), 6.40 (m, 2H), 4.92 (m, 1H), 4.69 (d, 1H), 4.46 (d, 1H), 3.68 (m, 6H), 3.26 (m, 2H), 2.76 (m, 2H) |
| 167 | 3-(5-Benzyloxy-1*H*-indol-3-yl)-2-[3-(2-dibenzylaminoethoxy)benzoylamino]propionic acid; ¹H-NMR: 7.27-7.05 (m, 15H), 6.92-6.85 (m, 4H), 6.72-6.50. (m, 4H), 4.82 (m, 1H), 4.64 (d, 1H), 4.43 (d, 1H), 3.67 (m, 2H), 3.56 (s, 4H), 3_{.}20 (m, 2H), 2.66 (m, 2H) |
| 168 | {(4-Benzyloxybenzyl)-{4-[2-(3-methylquinoxalin-2-yloxy)ethoxy]benzoyl}amino}acetic acid; ¹H-NMR: 7.87 (d, 1H), 7.74 (d, 1H), 7.60-7.25 (m, 10H), 7.06 (d, 1H), 6.90-6.88 (m, 4H), 5.00 (s, 2H), 4.80 (t, 2H), 4.68-4.55 (m, 2H), 4.38 (t, 2H), 4.04-3.79 (m, 2H), 2.58 (s, 3H) |
| 169 | (2*S*)-3-(4-Benzyloxyphenyl)-2-13-[2-(2,6-dimethylphenoxy)ethoxy]benzoylamino}propionic acid; ¹H-NMR: 7.43-7.30 (m, 9H), 7.24-6.91 (m, 7H), 6.58 (d, 1H), 5.07-5.00 (m, 3H), 4.35-4.32 (m, 2H), 4.17-4.14 (m, 2H), 3.30 (dd, 1H), 3.21 (dd, 1H), 2.32 (s, 6H) |
| 170 | 3-{(3-Benzyloxybenzyl)-{4-[2-(3-methylquinoxalin-2-yloxy)ethoxy]benzoyl}amino}propionic acid; MS: 592 |
| 171 | {(3-Benzyloxybenzyl)-{4-[2-(3-methylquinoxalin-2-yloxy)ethbxy]benzoyl}amino}acetic acid; MS: 578 |
| 172 | {(3-Benzyloxybenzyl)-{3-[2-(3-methylquinoxalin-2-yloxy)ethoxy]benzoyl}amino}acetic acid; MS: 578 |
| 173 | 3-{(3-Benzyloxybenzyl)-{3-[2-(3-methylquinoxalin-2-yloxy)ethoxy]benzoyl}amino}propionic acid; MS: 592 |
| 174 | {(3-Benzyloxybenzyl)-[4-(3-benzyloxybenzyloxy)benzoyl]amino}acetic acid; ¹H-NMR: 7.45-7.30 (m, 14H), 7.05-6.81 (m, 8H), 5.08 (s, 2H), 5.07 (s, 2H), 5.05 (s, 2H), 4.67 (br s, 2H), 4.15 (br s, 2H) |
| 175 | 3-{(3-Benzyloxybenzyl)-[4-(3-benzyloxybenzyloxy)benzoyl]amino}propionic acid; ¹H-NMR: 7.46-7.25 (m, 13H), 7.06-6.78 (m, 9H), 5.07 (s, 4H), 5.04 (s, 2H), 4.62 (br s, 2H), 3.66 (m, 2H), 2.73 (m, 2H) |
| 176 | 3-{(4-Benzyloxybenzyl)-[4-(3-benzyloxybenzyloxy)benzoyl]amino}propionic acid; ¹H-NMR: 7.46-7.27 (m, 13H), 7.11-6.93 (m, 9H), 5.07 (s, 2H), 5.06 (s, 2H), 5.04 (s, 2H), 4.59 (br s, 2H), 3.66 (m, 2H), 2.70 (m, 2H) |
| 177 | {(4-Benzyloxybenzyl)-[3-(3-benzyloxybenzyloxy)benzoyl]amino}acetic acid; ¹H-NMR: 7.44-7.26 (m, 13H), 7.11-6.91 (m, 9H), 5.08-4.98 (m, 6H), 4.75-4.52 (m, 2H), 4.16-3.86 (m, 2H) |
| 178 | {(3-Benzyloxybenzyl)-[3-(3-benzyloxybenzyloxy)benzoyl]amino}acetic acid; ¹H-NMR: 7.44-7.26 (m, 13H), 7.07-6.80 (m, 9H), 5.09-4.93 (m, 6H), 4.79-4.57 (m, 2H), 4.17-3.86 (m, 2H) |
| 179 | 3-{(3-Benzyloxybenzyl)-[3-(3-benzyloxybenzyloxy)benzoyl]amino}propionic acid; ¹H-NMR: 7.45-7.23 (m, 13H), 7.03-6.89 (m, 8H), 6.75 (m, 1H), 5.06-4.93 (m, 6H), 4.73-4.50 (m, 2H), 3.69-3.47 (m, 2H), 2.73-2.35 (m, 2H) |
| 180 | (2*S*)-3-(4-Benzyloxyphenyl)-2-{3-[2-(3-methylquinoxalin-2-yloxy)ethoxy]benzoylamino}propionic acid; MS: 578 |
| 181 | 3-[(4-Benzyloxybenzoyl)-(3-benzyloxybenzyl)amino]propionic acid; MS: 496 |
| 182 | 3-{(4-Benzyloxybenzyl)-[3-(3-benzyloxybenzyloxy)benzoyl] amino}propionic acid; ¹H-NMR: 7.41-7.26 (m, 13H), 7.02-6.92 (m, 9H), 5.06-4.98 (m, 6H), 4.69-4.46 (m, 2H), 3.68-3.48 (m, 2H), 2.71-2.43 (m, 2H) |
| 183 | (2*S*)-2-[4-(4-Benzyloxybenzyloxy)benzoylamino]-3-(4-benzyloxyphenyl)propionic acid; ³H-NMR: 7.68 (d, 2H), 7.44-7.30 (m, 12H), 7.10 (d, 2H), 7.00-6.90 (m, 4H), 6.89 (d, 2H), 5.08 (s, 2H), 5.02 (s, 4H), 4.95 (t, 1H), 3.27 (dd, 1H), 3.17 (dd, 1H) |
| 184 | (2*S*)-3-(4-Benzyloxyphenyl)-2-[4-(biphenyl-2-ylmeth xy)benzoylamino]propionic acid; ¹H-NMR: 7.64-7.55 (m, 3H), 7.42-7.33 (m, 13H), 7.10 (d, 2H), 6.89-6.84 (m, 4H), 5.02 (s, 2H), 4.97 (s, 2H), 4.95 (t, 1H), 3.26 (dd, 1H), 3.17 (dd, 1H) |
| 185 | (2*S*)-2- [3-(4-Benzyloxybenzyloxy) benzoylamino]-3-(4-benzyloxyphenyl)propionic acid; ¹H-NMR: 7.46-7.18 (m, 15H), 7.14-7.07 (m, 3H), 6.97 (d, 2H), 6.57 (d, 2H), 5.05-4.97 (m, 5H), 3.30 (dd, 1H), 3.19 (dd, 1H) |
| 186 | (2*S*)-3-(4-Benzyloxyphenyl)-2-[3-(3-phenylallyloxy)benzoylamino]propionic acid; ¹H-NMR: 7.41-7.18 (m, 13H), 7.10-7.04 (m, 3H), 6.85 (d, 2H), 6.71 (d, 1H), 6.38. (dt, 1H) , 4.98 (s, 2H), 4.95-4.87 (m, 1H), 4.69 (dd, 1H), 3.25 (dd, 1H), 3.15 (dd, 1H) |
| 187 | (2*S*)-3-(4-Benzyloxyphenyl)-2-[3-(biphenyl-4-ylmethoxy)benzoylamino]propionic acid; ¹H-NMR: 7.50-6.75 (m, 22H), 4.98 (s, 2H), 4.86 (s, 2H), 4.78 (m, 1H), 3.22-3.00 (m, 2H) |
| 188 | (2*S*)-3-(4-Benzyloxyphenyl)-2-[3-(3-phenoxybenzyloxy)benzoylamino]propionic acid; ¹H-NMR: 7.40-7.20 (m, 11H), 7.12-6.89 (m, 11H), 6.55 (d, 1H), 5.04-5.00 (m, 5H), 3.29 (dd, 1H), 3.20 (dd, 1H) |
| 189 | (2*S*)-3-(4-Benzyloxyphenyl)-2-[3-(3-phenylpropoxy)benzoylamino]propionic acid; ¹H-NMR: 7.40-7.16 (m, 13H), 7.06 (d, 2H), 7.00 (dd, 1H), 6.86 (d, 2H), 4.98 (s, 2H), 4.92 (t, 1H), 3.96 (t, 2H), 3.25 (dd, 1H), 3.14 (dd, 1H), 2.78 (t, 2H), 2.08 (m, 2H) |
| 190 | (2*S*)-2-[3-(4-Butylbenzyloxy)benzoylamino]-3-cyclohexylpropionic acid; MS: 438 |
| 191 | (2*S*)-3-(4-Bromophenyl)-2-{4-[2-(3-methylquinoxalin-2-yloxy)ethoxy]benzoylamino}propionic acid; MS: 551 |
| 192 | (2*S*)-3-(4-Fluorophenyl)-2-{4-[2-(3-methylquinoxalin-2-yloxy)ethoxy]benzoylamino}propionic acid; MS: 490 |
| 193 | (2*S*)-2-[4-(3-Benzyloxybenzyloxy)benzoylamino]-3-(4-bromophenyl)propionic acid; MS: 561 |
| 194 | (2*S*)-2-[4-(3-Benzyloxybenzyloxy)benzoylamino]-3-(4-fluorophenyl)propionic acid; MS: 500 |
| 195 | 3-{[4-(2-Dibenzylaminoethoxy)benzoyl]-(3-phenoxybenzyl)amino}propionic acid; MS: 615 |
| 196 | 3-{[4-(2-Dibenzylaminoethoxy)benzoyl] (4-phenoxybenzyl)amino}propionic acid; MS: 615 |
| 197 | 3-{[3-(2-Dibenzylaminoethoxy)benzoyl]naphthalen-2-ylmethylamino}propionic acid; MS: 573 |
| 198 | 3-{(4-*tert*-Butylbenzyl)-[3-(2-dibenzylaminoethoxy)benzoyl]amino}propionic acid; MS: 579. |
| 199 | 3-{Biphenyl-4-ylmethyl-[3-(2-dibenzylaminoethoxy)benzoyl]amino}propionic acid; MS: 599 |
| 200 | 3-{(4-Bromobenzyl)-[3-(2-dibenzylaminoethoxy)benzo yl]amino}propionic acid; MS: 601 |
| 201 | 3-{[3-(2-Dibenzylaminoethoxy)benzoyl]-(3-phenoxybenzyl)amino}propionic acid; MS: 615 |
| 202 | (2*S*)-3-(4-Benzyloxyphenyl)-2-{4-[2-(pyridin-2-yloxy)ethoxy]benzoylamino}propionic acid; MS: 513 |
| 203 | (2*S*)-3-(4-Benzyloxyphenyl)-2-{4-[2-(quinolin-8-yloxy)ethoxy]benzoylamino}propionic acid; MS: 563 |
| 204 | (2*S*)-3-(4-Benzyloxyphenyl)-2-{4-[2-(4-imidazol-1-yl-phenoxy)ethoxy]benzoylamino}propionic acid; MS: 578 |
| 205 | (2*S*) -3- (4-Benzyloxyphenyl) -2- [4- (2-naphthalen-2-yl -ethoxy)benzoylamino]propionic acid; ¹H-NMR: 7.80-7.76 (m, 3H), 7.69-7.62 (m, 3H), 7.46-7.25 (m, 8H), 7.06 (d, 2H), 6.89-6.83 (m, 4H), 4.97 (s, 2H) , 4.90 (t, 1H), 4.25 (t, 2H) , 3.24-3.20 (m, 3H), 3.11 (dd, 1H) |
| 206 | (2*S*)-3-(4-Benzyloxyphenyl)-2-{4-[2-(2-methylbenzothiazol-5-yloxy)ethoxy]benzoylamino}propionic acid; MS: 583 |
| 207 | (2*S*)-3-(4-Benzyloxyphenyl)-2-{4-[2-(5,7,8-tetrahydronaphthalen-2-yloxy)ethoxy]benzoylamino}propionic acid; MS: 566 |
| 208 | (2*S*)-2-[4-(4-Butylbenzyloxy)benzoylamino]-3-phenyl propionic acid; MS: 432 |
| 209 | (2*S*)-3-(4-Benzyloxyphenyl)-2-[4-(4-butylbenzyloxy)benzoylamino]propionic acid; MS: 538 |
| 210 | (2*S*)-2-[4-(4-Butylbenzyloxy)benzoylamino]-3-cyclohexylpropionic acid; MS: 438 |
| 211 | (2*S*)-3-Cyclohexyl-2-{4-[2-(3-methylquinoxalin-2-yloxy)ethoxy]benzoylamino}propionic acid; MS: 478 |
| 212 | (2*S*)-3-Cyclohexyl-2-{3-[2-(3-methylquinoxalin-2-yloxy)ethoxy]benzoylamino}propionic acid; MS: 478 |
| 213 | (2*S*)-2-[4-(3-Benzyloxybenzyloxy)benzoylamino]-3-cyclohexylpropionic acid; MS: 488 |
| 214 | (2*S*)-2-[3-(3-Benzyloxybenzyloxy) benzoylamino]-3-cyclohexylpropionic acid; MS: 488 |
| 215 | (2*S*)-3-(4-Benzyloxyphenyl)-2-[3-(4-butylbenzyloxy)benzoylamino]propionic acid; MS: 538 |
| 216 | {(3-Benzyloxybenzyl)-[4-(4-butylbenzyloxy)benzoyl] mino}acetic acid; MS: 538 |
| 217 | {(4-Benzyloxybenzyl)-[4-(4-butylbenzyloxy)benzoyl]amino}acetic acid; ¹H-NMR: 7.50-7.10 (m, 13H), 6.97 (d, 4H), 5.06 (s, 2H), 5.03 (s, 2H), 4.63 (br s, 2H), 4.15 (br s, 2H), 2.61(t, 2H), 1.59 (q, 2H), 1.36 (m, 2H), 0.92 (t, 3H) |
| 218 | 3-{(3-Benzyloxybenzyl)-[4-(4-butylbenzyloxy)benzoyl]amino}propionic acid; MS: 552 |
| 219 | 3-{(4-Benzyloxybenzyl)-[4-(4-butylbenzyloxy)benzoyl]amino}propionic acid; MS: 552 |
| 220 | [3-(4-Benzyloxybenzyloxy)benzoylamino]phenylacetic acid; MS: 468 |
| 221 | (2*S*)-2-[3-(4-Benzyloxybenzyloxy)benzoylaminol-3-phenylpropionic acid; MS: 482 |
| 222 | (2*S*)-2-[3-(4-Benzyloxybenzyloxy) benzoylamino]-3-cyclohexylpropionic acid; MS: 488 |
| 223 | {(3-Benzyloxybenzyl)-[3-(4-butylbenzyloxy)benzoyl] mino}acetic acid; MS: 538 |
| 224 | 3-{(3-Benzyloxybenzyl)-[3-(4-butylbenzyloxy)benzoyl]amino}propionic acid; MS: 552 |
| 225 | (2*S*) -2-{4-[2-(3-Methylquinoxalin-2-yloxy)ethoxy]benzoylamino}-3-phenylpropionic acid; MS: 472 |
| 226 | [3-(3-Benzyloxybenzyloxy)benzoylamino]phenylacetic acid; MS: 468 |
| 227 | (2*S*)-2-[3-(3-Benzyloxybenzyloxy)benzoylamino]-3-phenylpropionic acid; MS: 482 |
| 228 | {(3-Benzyloxybenzyl)-[3-(4-benzyloxybenzyloxy)benzoyl]amino}acetic acid; MS: 588 |
| 229 | 3-{(3-Benzyloxybenzyl)-[3-(4-benzyloxybenzyloxy)benzoyl]amino}propionic acid; MS: 602 |
| 230 | (2*S*)-3-(4-Benzyloxyphenyl)-2-{4-[2-(quinolin-2-yloxy)ethoxylbenzoylamino}propionic acid; MS: 563 |
| 231 | (2*S*)-3-(4-Benzyloxyphenyl)-2-{4-[2-(quinolin-7-yloxy) ethoxy]benzoylamino}propionic acid; MS: 563 |
| 232 | [4-(4-Butylbenzyloxy)benzoylamino]phenylacetic acid; MS: 418 |
| 233 | (2*S*)-3-(4-Benzyloxyphenyl)-2-[4-(4-butoxybenzyloxy)benzoylamino]propionic acid; MS: 554 |
| 234 | {[4-(3-Benzyloxybenzyloxy)benzoyl]-(4-benzyloxyphenyl)amino}acetic acid; MS: 574 |
| 235 | {[4-(3-Benzyloxybenzyloxy)benzoyl]-(3-benzyloxyphenyl)amino}acetic acid; MS: 574 |
| 236 | (2*S*)-3-(4-Benzyloxyphenyl)-2-[4-(2-pyridin-2-yl-ethoxy)benzoylamino]propionic acid; MS: 497 |
| 237 | (2*S*) -2- [4- (4-Butoxybenzyloxy) benzoylamino]-3-cyclohexylpropionic acid; MS: 454 |
| 238 | (2*S*)-3-(4-Benzyloxyphenyl)-2-[4-(2-bromobenzyloxy)benzoylamino]propionic acid; MS: 561 |
| 239 | (2*S*)-3-(4-Henzyloxyphenyl)-2-[4-(3-bromobenzyloxy)benzoylamino]propionic acid; MS: 561 |
| 240 | (2*S*)-3-(4-Benzyloxyphenyl)-2-[4-(2-chlorobenzyloxy)benzoylamino]propionic acid; MS: 516 |
| 241 | (2*S*)-3-(4-Benzyloxyphenyl)-2-[4-(3-chlorobenzyloxy)benzoylamino]propionic acid; MS: 516 |
| 242 | (2*S*)-3-(4-Benzyloxyphenyl)-2-[4-(2-fluorobenzyloxy)benzoylamino]propionic acid; MS: 500 |
| 243 | (2*S*)-3-(4-Benzyloxyphenyl)-2-[4-(2-methylbenzyloxy)benzoylamino]propionic acid; MS: 496 |
| 244 | (2*S*)-3-(4-Benzyloxyphenyl)-2-[4-(3-methylbenzyloxy)benzoylamino]propionic acid; MS: 496 |
| 245 | (2*S*)-3-(4-Benzyloxyphenyl)-2-[4- 3-trifluoromethylbenzyloxy)benzoylamino] propionic acid; MS: 550 |
| 246 | (2*S*)-3-(4-Benzyloxyphenyl)-2-[4-(2-trifluoromethylbenzyloxy)benzoylamino] propionic acid; MS: 550 |
| 247 | (2*S*)-2-(4-(3-Bromobenzyloxy)benzoylamino]-3-cyclohexylpropionic acid; MS: 461 |
| 248 | (2*S*)-3-(4-Benzyloxyphenyl)-2-[4-(2-methoxybenzyloxy)benzoylamino]propionic acid; MS: 512 |
| 249 | (2*S*)-3-(4-Benzyloxyphenyl)-2-{4-[(benzylphenylcarbamoyl)methoxy]-benzoylamino}propionic acid; ¹H-NMR: 7.60 (d, 2H), 7.38-6.99 (m, 17H), 6.87 (d, 2H), 6.75 (d, 2H), 6.63 (d, 1H), 5.10-4.90 (m, 5H), 4.41 (s, 2H), 3.32-3.10 (m, 2H) |
| 250 | (2*S*)-3-(4-Benzyloxyphenyl)-2-{4-[(dibenzylcarbamoy1)methoxy]-benzoylamino}propionic acid; ¹H-NMR: 7.64 (d, 2H), 7.41-6.84 (m, 21H), 6.64 (d, 1H), 5.03-4.91 (m, 3H), 4.81 (s, 2H), 4.61 (s, 2H), 4.50 (s, 2H), 3.33-3.12 (m, 2H) |
| 251 | (2*S*)-3-(4-Benzyloxyphenyl)-2-{4-[2-(10,11-dihydrodibenzo[b,f]azepin-5-yl)-2-oxoethoxy] benzoylamino}propionic acid; ¹H-NMR: 7.60 (d, 2H), 7.38-7.06 (m, 15H), 6.87 (d, 2H), 6.79 (d, 2H), 6.60 (d, 1H), 5.04-4.93 (m, 3H), 4.77 (d, 1H), 4.46 (d, 1H), 3.37-3.20 (m, 4H), 2.89-2.74 (m, 2H) |
| 252 | (2*S*) -2-{4- [2- (Benzoylbenzylamino) ethoxy] benzoylamino}-3-(4-benzyloxyphenyl)propionic acid; ¹H-NMR: 7.65 (d, 2H), 7.38-6.61 (m, 22H), 4.98 (m, 3H), 4.69-3.63 (m, 6H), 3.34-3.13 (m, 2H) |
| 253 | (2*S*)-3-(4-Benzyloxyphenyl)-2-(4-{2-[benzyl(pyridine-3-carbonyl) amino] ethoxy}benzoylamino) propionic acid; ¹H-NMR: 8.70-6.74 (m, 23H) , 5.01-4.89 (m, 3H), 4.88-3.61 (m, 6H), 3.35-3.14 (m, 2H) |
| 254 | [{4-[2-(Benzoylbenzylamino)ethoxy]benzoyl}-(4-benzyloxybenzyl)amino] acetic acid; 1H-NMR: 7.79-6.74 (m, 23H), 5.06 (s, 2H), 4.89-3.61 (m, 10H) |
| 255 | [(4-Benzyloxybenzyl)-(4-{2-[benzyl(pyridine-3-carbonyl)amino]ethoxy}benzoyl) amino] acetic acid; ¹H-NMR:: 8.64 (d, 1H), 7.79-6.80 (m, 21H), 5.06 (s, 2H), 4.87-3.65 (m, 10H) |
| 256 | {Thiophen-3-ylmethyl-{3-[2-(thiophen-2-ylsulfanyl)ethoxy]benzoyl}amino}acetic acid; MS: 434 |
| 257 | {Thiophen-2-yl-{3-[2-(thiophen-2-ylsulfanyl)ethoxy]benzoylamino}acetic acid; MS: 420. |
| 258 | {3-[(Benzylphenylcarbamoyl)methoxy]benzoylamino}thiophen-3-yl-acetic acid; MS: 501 |
| 259 | 3-(4-Benzyloxyphenyl)-2-{3-[(benzylphenethylcarbamoyl)methoxy]-benzoylamino}propionic acid; ¹H-NMR: 7.41-7.00 (m, 21H), 6.90-6.75 (m, 2H), 4.85-4.75 (m, 3H), 4.67-4.55 (m, 2H) , 4.45-4.25 (m, 2H), 3.60-3.35 (m, 2H); 3.35-3.05 (m, 2H), 2.60-2.05 (m, 2H); MS: 643 |
| 260 | 3-(4-Benzyloxyphenyl)-2-{4-[(phenylpyridin-2-yl-carbamoyl)methoxy]-benzoylamino}propionic acid; ¹H-NMR: 9.92 (d, 1H), 7.62-7.40 (m, 6H), 7.35 (d, 2H), 7.30-7.10 (m, 7H), 7.12 (d, 1H), 6.97 (d, 2H), 6.90 (2H) , 6.75 (d, 2H), 5.27 (s, 2H), 4.89 (s 2H), 4.76 (t, 1H), 3.11 (dd, 1H), 3.01 (dd, 1H) |
| 261 | 3-(4-Benzyloxyphenyl)-2-{4-[(cyclohexylphenylcarbamoyl)methoxy]benzoylamino}propionic acid; MS: 607 |
| 262 | 3-(4-Benzyloxyphenyl)-2-{4-[(*tert*-butylcyclohexylcarbamoyl)methoxy]-benzoylamino}propionic acid; MS: 587 |
| 263 | 3-(4-Benzyloxyphenyl)-2-(4-{[(2-fluorophenyl)thiophen-2-ylmethylcarbamoyl]methoxy}benzoylamino)propionic acid; MS: 639 |
| 264 | (2*S*)-3-(4-Benzyloxyphenyl)-2-{3-[2-(3-methyl-2-oxo-2*H*-quinoxalin-1-yl)ethoxylbenzoylamino)propionic acid; MS: 578 |
| 265 | (2*S*)-3-((4-Benzyloxybenzyl)-{3-[2-(3-methyl-2-oxo-2*H*-quinoxalin-1-yl)ethoxy]benzoyl}amino)propionic acid; MS: 592 |

### EXAMPLES (Ic) and (Id)

The compounds of formula (Ic) and (Id) shown in Table 18 were synthesized either according to any of methods A to C, starting from compounds of formula (Ib) and the aminic derivatives HNR₂R₃ oR HNR₂OR₁:

**TABLE 18**

| Ex. | |
|---|---|
| 266 | *N*-[(1*S*)-2-(4-Benzyloxyphenyl)-1-dimethylcarbamoylethyl]-4-phenetyloxybenzamide; ¹H-NMR: 7.75 (d, 2H), 7.44-6.88 (m, 16H), 5.30 (m, 1H), 5.05 (s, 2H), 4.22 (t, 2H), 3.25-2.90 (m, 4H), 2.88 (s, 3H), 2.67 (s, 3H) |
| 267 | *N*-[(1*S*)-2-(4-Benzyloxyphenyl)-1-dimethylcarbamoylethyl]-4-[2-(3-methylquinoxalin-2-yloxy) ethoxy]benzamide; 7.96-7.29 (m, 9H), 7.14-7.09 (m, 4H), 7.00 (d, 2H), 6.89 (d, 2H), ¹H-NMR: 5.30 (m, 1H), 5.05 (s, 2H), 4.88 (t, 2H), 4.47 (t, 2H), 3.30=2.95 (m, 2H), 2.88 (s, 3H), 2.68 (s, 3H), 2.65 (s, 3H) |
| 268 | *N*-[(1*S*)-2-(4-Benzyloxyphenyl)-1-hydroxycarbamoylethyl]-4-[2-(3-methylquinoxalin-2-yloxy)ethoxy]benzamide; MS: 593 |
| 269 | *N*-[(1*S*)-2-(4-Benzyloxyphenyl)-1-methoxycarbamoylethyl]-4-[2-(3-methylquinoxalin-2-yloxy)ethoxy]benzamide;MS: 607 |
| 270 | *N*-[(1*S*)-2-(4-Benzyloxyphenyl)-1-(methoxymethycarbamoyl)ethyl]-4-[2-(3-methylquinoxalin-2-yloxy)etho xy]benzamide; MS: 621 |

### Assay of binding to the PPARγ2

The cDNA encoding for the open reading frame of the hPPARγ2 is amplified by PCR *(polymerase chain reaction)* and inserted in the plasmid pGEX-4T-2. This construction (pGEX-hPPARγ) is introduced into *E*. *coli* where it is overexpressed and semipurified as a fusion protein with glutathione S-transferase (GST) (Elbrecht et al., J. Biol. Chem. 1999, 274, 7913-7922).

The binding of the compounds to the GST-hPPARγ2 s is determined by modifications in the method described by Lehmann et al. (J. Biol. Chem. 1995, 270, 12953-12957). The receptors (2.5 µg) were incubated in 96-well plates in the presence or in the absence of the products with [³H]BRL-49853 (100 nM) for 3 h at 4°C, in a final volume of 200 µL of buffer Tris-HCl 10 mM pH:8.0, containing KCl 50 mM and DTT 10 mM. Non-specific binding was determined in the presence of BRL-49853 100 µM. The reaction mixture was transferred to a Multiscreen Durapore (Millipore) microplate containing glutathione-Sepharose 4B in every well. The reaction mixture was left to incubate with the resin during 10 min, and then centrifuged at 735 g during 2 min. To dissociate the receptor bound to the resin, reduced glutathione 10 mM is added and incubated during 10 min. The receptor was eluted by centrifugation. Then, 800 µL of scintillation liquid were added to the elution and the contained radioactivity was quantified by liquid scintillation spectroscopy (Microbeta Wallac, Perkin Elmer).

### LBD-hPPARs transactivation assay

COS-7 cells were cultivated in 24-well plates and transfected with the pFACMV plasmids that encode the chimeric proteins containing the GAL4 DNA binding domain fused to the PPARγ LBD. The reporter plasmid for the foregoing constructions was pFR-Luc, which contains five repetitions of the GAL4-response element in front of a promoter that controls the transcription of the luciferase gene. Lipofectamine was used as a transfection agent.

The plasmids of the chimeric receptors and the reporter gene were inserted in the cells by transitory transfection in COS-7 cells in culture. When the products were added to the culture for 48 h, the luciferase activity showed the effect of the PPAR activity modulation on the transcription of the reporter construction (Wright et al., J. Biol. Chem. 2000, 275, 1873).

### Cloning of human PPARα, PPARδ and PPARγ2

The human PPARs cDNAs were amplified through RT-PCR. For hPPARα, RNA was obtained from HepG2 cells treated with linoleic acid; for h PPARδ, RNA was obtained from untreated HepG2 cells; for hPPARy2, RNA was obtained from human white adipose tissue. Each amplified fragment was cloned into pBluescript (Stratagene^{®}) and sequenced. One clone for each construction was selected and used as template for further subcloning and PCR amplifications.

### GST-fused protein construction

To generate these chimeric proteins, the complete cDNA of the four human PPARs were cloned into pGEX4T2 (Amersham Biosciences). The fragment was obtained from the pBluescript-cDNAs clones digested with endonucleases. To assess the plasmid identity and to ensure the in-phase cloning of the proteins, pGEXs constructions were sequenced. GST-hPPARy2, GST-hPPARα or GST-h PPARδ fusion proteins were generated in Escherichia coli (BL21 strain DE3). Cells were cultured in LB medium to a density of A600= 1.6 odu, and induced for overexpression by addition of isopropyl-l-thio-β-D-galactopyranoside (IPTG)-induced cultures to a final concentration of 0.5 mM. The IPTG-induced cultures were grown at room temperature o/n, before cells were harvested by centrifugation at 5000 g for 15 min. After sonication, the GST-fusion proteins were purified from the cell pellet using glutathione-Sepharose beads, following the procedure recommended by the manufacturer (Amersham Pharmacia Biotech). Excess of gluthatione was removed o/n by dyalisis at 4°C. Receptor purity was visualized by SDS-PAGE and protein content was determined by Bradford method. Receptor aliquots were stored at -80°C until use.

### GST-hPPARα and GST-hPPARδ binding

Using 96-well culture plates, PPARα or PPARδ (5 µg) were diluted to a total volume of 100 µL with buffer consisting of 50 mM HEPES (pH:7.0), 50 mM KCl, 5 mM EDTA and10 mM DTT, in the presence of [3H]-GW2433 (100 and 50 nM for PPARα and PPARδ, respectively). Nonspecific binding was estimated in parallel incubations containing 50 µM of GW-2433. Plates were incubated for 2 h at room temperature. Free radioligand was separated from receptor-bound ligand by size exclusion chromatography using. Sephadex G-25 in 96-wells spin plates, using the Multiscreen Column loader (Millipore). Eluted radioactivity was quantitated by liquid scintillation counting in a Microbeta counter (Perkin Elmer).

In Table 19, affinity and functional activity data of some of the compounds of the present invention are shown.

**TABLE 19**

| Ex. | Affinity PPARγ ⁽¹⁾ | Functional activity PPARγ | Affinity PPARα ⁽¹⁾ | Affinity PPARδ ⁽¹⁾ |
|---|---|---|---|---|
| 20 | +++ | Partial agonist | + | + |
| 21 | +++ | Partial agonist | + | + |
| 27 | +++ | Antagonist | + | + |
| 95 | +++ | Agonist | + | + |
| 98 | +++ | Antagonist | + | + |
| 129 | +++ | Partial agonist | + | + |
| 131 | ++ | Partial agonist | + | + |
| 136 | ++ | Antagonist | + | ++ |
| 141 | ++ | Antagonist | + | ++ |
| 142 | ++ | Antagonist | + | ++ |
| 145 | ++ | Antagonist | + | + |
| 146 | ++ | Antagonist | + | + |
| 153 | ++ | Partial agonist | + | + |
| 160 | + | Partial agonist | + | + |
| 161 | ++ | Antagonist | + | + |
| 162 | +++ | Antagonist | + | + |
| 163 | +++ | Antagonist | + | + |
| 164 | ++ | Antagonist | + | + |
| 170 | ++ | Antagonist | + | + |
| 176 | +++ | Antagonist | ++ | + |
| 180 | +++ | Partial agonist | ++ | + |
| 183 | +++ | Partial agonist | + | + |
| 184 | +++ | Antagonist | + | - |
| 185 | +++ | Partial agonist | + | + |
| 187 | +++ | Agonist | + | + |
| 188 | +++ | Partial agonist | + | + |
| 192 | + | Partial agonist | + | + |
| 210 | +++ | Agonist | + | + |
| 218 | +++ | Antagonist | + | + |
| 237 | +++ | Partial agonist | + | + |
| 238 | +++ | Antagonist | + | + |
| 243 | +++ | Antagonist | + | + |
| 267 | ++ | Partial agonist | + | + |

| | | | | |
|---|---|---|---|---|
| ⁽¹⁾ +++ : Ki < 1000 nM, ++: 1000 nM< Ki <3000 nM, + : Ki >3000 nM | | | | |

## Claims

1. A compound of formula (I), 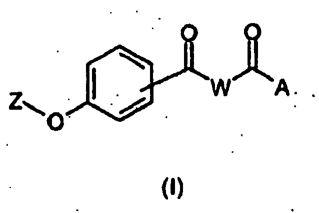 its stereoisomers and mixtures thereof, its polymorphs and mixtures thereof, and the pharmaceutically acceptable solvates and addition salts of all of them, wherein the central benzene ring may be substituted in *meta*- or *para-*position and,
-A is a radical selected from the group consisting of -OR1, -NR2OR1 and -NR2R3; wherein R1, R2 and R3 independently represent -H or (C₁-C₄) -alkyl;
-W- is a biradical selected from the group: -NH-CH(E)-, and -K(D)-CH₂-CH₂-; wherein B is a radical of the -G-I-J-K type and D is a radical of the -G-I'-J-K type where:
-G- is a bond or a - (CH2)₁₋₄- biradical;
-I- is a biradical of a cycle selected from the following groups:
a) cyclopropane, cyclobutane, cyclopentane, cyclohexane and cyclohexene, all optionally substituted by one or several radicals independently selected from -OH, oxo (-O), -CHO, -SH, -NO_{2,} -CN, -F, -Cl, -Br, (C₁C₄) -alkanoyl, (C₁C₄)-alkokycarbonyl, (C₁-C₄) -alkanoyloxy, (C₁-C₄) -alkylsulfinyl, (C₂-C₄) -alkylsulfanyl, (C₁-C₄)-alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -F, and (C₁-C₄)-alkoxyl optionally substituted by one or several -OH or -F;
b) a five- or aix-membered aromatic heterocycle containing from one to three heteroatoms selected from O, S and N, this heterocycle being optionally substituted by one or several radicals independently selected from -OH, oxo (=O), -CHO, -SH, -NO₂, -CN, -P, -Cl, -Br, (C₁-C₄) alkanoyl, (C₁-C₄) -alkoxycarbonyl, (C₁-C₄) -alkanoyloxy, (C₁-C₄) -alkylsulfinyl, (C₁-C₄)-alkylsulfanyl, (C₁-C₄)-alkylsulfonyl, (C₁-C₄) -alkyloxy-SO₂-, -(C₁-C₄) -alkyl-SO₂O-, -NR2R3, -CONR2R3, (C₁-C₄) -alkyl optionally substituted by one or several -OH or -P, and (C₁-C₄)-alkoxyl optionally substituted by one or several -OH or -F;
c) benzene or benzene substituted by one or several radicals independently selected from -OH, -CHO, -SH, -NO₂, -CN, -F, -Cl, -Br, (C₁-C₄) -alkanoyl, (C₁-C₄) -alkoxycarbonyl, (C₁-C₄)-alkanoyloxy, (C₁-C₄)-alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄)-alkyl optionally substituted by one or several -OH or -F, and (C₁-C₄) -alkoxyl optionally substituted by one or several -OH or -F; and
d) a bicyclic system consisting of a benzene fused with a five- or six-membered ring optionally containing from one to three heteroatoms elected from O, S and N, this bicyclic system being optionally substituted by one or several radicals independently selected from -OH, oxo (=O), -CHO, -SH, -NO₂, -CN, -F, -Cl, -Br, (C₁-C₄) -alkanoyl, (C₂-C₄) -alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substitute by one or several -OH or -F, and (C₁-C₄) -alkoxyl optionally substituted by one or several -OH or -F;
-J- is a bond or a biradical selected from the following groups:
a) -(CH₂)₁₋₄-alkylidene;
b) -O-, and
c) -O- (C₁-C₄)-alkyl-;
-K is a radical selected from the following groups:
a) -H;
b) (C₁-C₄)-alkyl;
c) a radical from a cycle selected from the following: cyclopropane, cyclobutane, cyclopentane, cyclohexane and cyclohexene, all of them optionally substituted by one or several radical independently selected from -OH, oxo (=O), -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄)-alkyl optionally substituted by one or several -OH or -F, and (C₁-C₄)-alkoxyl optionally substitute by one or several -OH or -F;
d) a radical from a five- or six-membered heterocycle containing from one to three heteroatoms selected from o, s, and N, being this heterocycle optionally substituted by one or several radicals independently selected from -OH, oxo (=O)_{,} -CHO, -SH, "NO₂. -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -F, and (C₁-C₄) -alkoxyl optionally substituted by one or several -OH or -F; and
e) phenyl or phenyl optionally substituted by one or several radicals independently selected from -OH, -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄) -alkylsulfanyl (C₁-C₄) -alkylsulfonyl, (C₁-C₄) -alkyloxy-SO₂-, (C₁-C₄) -alkyl-SO₂O-, -NR2R3, -CONR2R3, (C₂-C₄) -alkyl optionally substituted, by one or several -OH or -F, and (C₁-C₄) -alkoxyl optionally substituted by one or several -OH or -F;
-I'- is a biradical of a cycle selected from the following groups :
a) cyclopropane, cyclobutane, cyclopentane, cyclohexane and cyclohexene, all optionally substituted by one or several radicals independently selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl- SO₂O-, -NR2R3, -CONR2R3, (C₁-C₄) -alkyl optionally substituted by one or several -OH or -P, and (C₁-C₄) -alkoxyl optionally substituted by one or several -OH or -F;
b) a five- or six-membered aromatic heterocycle containing from one to three heteroatoms selected from O, S and N, being this heterocycle optionally substituted by one or several radicals independently selected from -OH, oxo (=O), -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄)-alkylsulfinyl, (C₁-C₄) -alkylsulfanyl, (C₁-C₄) -alkylsulfonyl, (C₁-C₄) -alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
c) benzene substituted by one or several radicals independently selected from -OH, -CHO, -SH, -NO₂, -CN, -F, -Cl, -Br, (C₁-C₄) -alkanoyl, (C₁-C₄)-alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -P, (C₁-C₄) -alkoxyl optionally substituted by one or several -OH or -F; and
d) a bicyclic system consisting of a benzene fused with a five- or six-membered ring optionally containing from one to three heteroatoms selected from O, S and N, being this bicyclic system optionally substituted by one or several radicals independently selected from -OH, oxo (=O), -CHO, -SH, -NO₃, -CN, -P, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄)-alkylsulfanyl, (C₁-C₄) -alkylsulfonyl, (C₁-C₄) -alkyloxy-SO₂-, (C₁-C₄) - alkyl - SO₂O-, -NR2R3, -CONR2R3, (C₁-C₄) -alkyl optionally substituted by one or several -OH or -F, and (C₁-C₄) -alkoxyl optionally substituted by one or several -OH or -F;
-Z is a radical selected from the following groups:
a) -Q-I-J-T wherein
-Q- is a biradical - -(CH₂)₁₋₃-;
-I- is as defined above ;
-J- is as defined above; and
-T is a radical selected from the following groups:
a.a) -H;
a.b) (C₁-C₄) -alkyl;
a.c) a radical from a cycle selected from the following: cyclopropane, cyclobutane, cyclopentane, cyclohexane and cyclohexene, all of them optionally substitute by one or several radicals independently selected from -OH, oxo (=O), -CHO, -SH, -NO₂, -CN, -P, -Cl, -Br, (C₁-C₄)-alkanoyl, (C₁-C₄) -alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -P, and (C₁-C₄)-alkoxyl optionally substituted by one or several -OH or -F;
a.d) a radical from a five- or six-membered heterocycle containing from one to three heteroatoms selected from O, S and N, this heterocycle, being optionally substituted by one or several radical independently selected from -OH, oxo (=O), -CHO, -SH, -NO₂, -CN, -F, -Cl, -Br, (C₁-C₄)-alkandyl, (C₁-C₄) -alkoxycarbonyl, (C₁-C₄) -alkanoyloxy, (C₁-C₄) -alkylsulfinyl, (C₁-C₄)-alkylsulfanyl, (C₁-C₄)-alkylsulfonyl, (C₁-C₄) -alkyloxy-SO₂-, (C₁-C₄) -alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
a.e) phenyl or phenyl optionally substituted by one or several radicals independently selected from -OH, -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) - alkylsulfinyl, (C₁-C₄) -alkylsulfanyl, (C₁-C₄)-alkylsulfonyl, (C₁-C₄) -alkyloxy-SO₂-, (C₁-C₄)-alkyl-SO₂O-; -NR2R3, -CONR2R3, (C₁-C₄) -alkyl optionally substituted by one or several -OH or -F, and (C₁-C₄) -alkoxyl optionally, substituted by one or several -OH or -F; and
a.f) a radical from a bicyclic system consisting of a benzene fused with a five-or six-membered ring optionally containing from one to three heteroatoms selected from O, S and N, being this bicyclic system optionally substituted by one or several radicals independently selected from -OH, oxo (=O) -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄) -alkylsulfanyl, (C₁-C₄) -alkylsulfonyl, (C₁-C₄) -alkyloxy-SO₂-, (C₁-C₄)-alkyl-SO₂O-, -NR2R3, =CONR2R3, <C₁-C₄) -alkyl optionally substituted by one or several -OH or -F, and (C₁-C₄)-alkoxyl optionally substituted by one or several -OH or -F;
b) (CH₂) ₛ-X-P-I-J-T wherein
s is 2 or 3;
-X- is selected from the group consisting of -O-, -S-, -SO-, -SO₂- and -NR4-, being R4 a radical selected from the group:
b.a) -H;
b.b) (C₁-C₁₀) -alkyl;
b.c) cycloalkyl, cycloalkyl-CO-, cycloalkyl- (C₁-C₃) -alkyl and cycloalkyl- (C₁-C₃) -alkanoyl, wherein the cycloalkyl is a five- or six-membered ring optionally substituted by one or several radicals selected from -OH, oxo (=O), -CHO, -SH, -NO₂, -CN, -F, -Cl, -Br, (C₁-C₄) -alkanoyl, (C₁-C₄) -alkoxycarbonyl, (C₁-C₄) alkanoyloxy, (C₁-C₄) -alkylsulfinyl, (C₁-C₄)-alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -P, and - (C₁-C₄) -alkoxyl optionally substituted by one or several OH or F;
b.d) phenyl, phenyl-CO-, phenyl- (C₁-C₃) -alkyl and phenyl- (C₁-C₃) -alkanoyl, being this aromatic ring optionally substituted by one or several radicals selected from -OH, -CHO, -SH, -NO₂, -CN, -F, -Cl, -Br, (C₁-C₄)-alkanoyl, (C₁-C₄) -alkoxycarbonyl, (C₁-C₄) -alkanoyloxy, (C₁-C₄) -alkylsulfinyl, (C₁-C₄)-alkylsulfanyl, (C₁-C₄) -alkylsulfonyl, (C₁-C₄) -alkyloxy-SO₂-, (C₁-C₄) -alkyl-SO₂O-, -NR2R3, -CONR2R3, (C₁-C₄) -alkyl optionally substituted by one or several -OH or -F, and (C₁-C₄)-alkoxyl optionally substituted by one or several -OH or -F; and
b.e) a heterocycle, heterocycle-CO, heterocycle-(C₁-C₃) -alkyl and heterocycle-(C₁-C₃) -alkanoyl, wherein the heterocycle is a five- or six-membered ring containing from one to three heteroatoms selected from O, S and N, being this heterocycle optionally substituted by one or several radicals selected from -OH, oxo (=O), -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl; (C₁-C₄) -alkylsulfanyl, (C₁-C₄) -alkylsulfonyl, (C₁-C₄ -alkyloxy-SO₂-, (C₁-C₄) -alkyl-SO₂O-, -NR2R3, -CONR2R3, (C₁-C₄)-alkyl optionally substituted by one or several -OH or -F, and (C₁-C₄) -alkoxyl optionally substituted by one or several -OH or -F;
-P- is a bond or a - (CH₂) ₁₋₄- biradical;
-I- is as defined above;
-J- is as defined above; and
-T is a radical as defined above;
c) -(CH₂)ᵤ-CO-NR5-P-I-J-T wherein
u is 1 or 2;
-R5 is a radical selected from the group:
c.a) -H;
c.b) (C₁-C₁₀) -alkyl;
c.c) cycloalkyl and cycloalkyl- (C₁-C₃) -alkyl, wherein the cycloalkyl is a five- or six-membered ring optionally substituted by one or several radicals selected from -OH, oxo (=O), -CHO, -SH, -NO₂, -CN, -F, -Cl, -Br, (C₁-C₄) -alkanoyl, (C₁-C₄) -alkoxycarbonyl, (C₁-C₄) -alkanoyloxy, (C₁-C₄) -alkylsulfinyl, (C₁-C₄) -alkylsulfanyl, (C₁₋C₄)-alkylsulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
c.d) phenyl and phenyl-(C₁-C₃) -alkyl, being this aromatic ring, optionally substituted by one or several radicals selected from -OH, -CHO, -SH, -NO₂, -CN, -P, -Cl, -Br, (C₁-C₄)-alkanoyl, (C₁-C₄)-alkoxycarbonyl, (C₁-C₄) -alkanoyloxy, (C₁-C₄) -alkylsulfinyl; (C₁-C₄)-alkylsulfanyl, (C₁-C₄)-alkylsulfonyl, (C₁-C₄)-alkyloxy-SO₂-, (C₁-C₄)-alkyl-SO₂O-, -NR2R3, -CONR2R3, (C₁-C₄₎-alkyl optionally substitute by one or several -OH or -F, and (C₁-C₄)-alkoxyl optionally substituted by one or several -OH or -F; and
c.e) a heterocycle and heterocycle-(C₁-C₄)-alkyl, wherein the heterocycle is a five- or six-membered ring containing from one to three heteroatoms selected from, O, S and N, being this heterocyclo optionally substituted by one or several radicals selected from -OH, oxo (=O), -CHO, -SH, -NO₂, -CN, -F, -Cl, -Br, (C₁-C₄)-alkanoyl, (C₁-C₄)-alkoxycarbonyl, (C₁-C₄) -alkanoyloxy, (C₁-C₄)-alkylsulfinyl, (C₁-C₄)-alkylsulfanyl, (C₁-C₄)-alkyloulfonyl, (C₁-C₄)-alkyloxy-SO₂-, (C₁-C₄)-alkyl-SO₂O-, -NR2R3, -CONR2R3, (C₁-C₄)-alkyl optionally substituted by one or several -OH or -F, and (C₁-C₄)-alkoxyl optionally substituted by one or several -OH or -F;
-P- is as defined above;
-I- is as defined above;
-J- is as defined above; and
-T is as defined above;
with the proviso that compound of formula (I) is neither of 2-(4-benzyloxybenzoylamino)-3-phenylpropionic acid, 2-[4-(4-methoxybenzyloxy)benzoylamino]-3-phenylpropionic acid, 2-[4-(4-bromobenzyloxy)benzoylamino]-3-phenylpropionic acid, , cyclopentyl-[4-(2-methylquinolin-4-ylmethoxy)benzoylamino]acetic acid methyl ester, [4-(2-methylquinolin-4-ylmethoxy)benzoylamino](tetrahydropyran-4-yl)acetic acid methyl ester or 2-(4-benzyloxybenzoylainino)-3-biphenyl-4-ylpropionic acid or 2-(4-benzyloxybenzoylamino)-3-(4'-trifluoromethoxybiphenyl-4-yl)propionic acid.

2. The compound according to claim 1, wherein W is -NH-CH(E)-.

3. The compound according to claim 2, wherein -Z is a radical of the -Q-I-J-T type.

4. The compound according to claim 2, wherein -Z is a radical of the -(CH₂)ₛ-X-P-I-J-T type.

5. The compound according to claim 4, wherein -X- is -O-.

6. The compound according to claim 4, wherein s is 2 and -X- is -NR4-.

7. The compound of claim 1, wherein W is -N(E)-CH₂-CH₂-.

8. The compound according to claim 7, wherein -Z is a radical of the -Q-I-J-T type.

9. The compound according to claim 7, wherein -Z is a radical of the -(CH₂)ₛ-X-P-I-J-T type.

10. The compound according to claim 9, wherein -X- is -O-.

11. The compound according to claim 9, wherein s is 2 and -X-is -NR4-.

12. The compound according to claim 1, wherein -A is an -OR1 type radical.

13. The compound according to claim 1 selected from the group consisting of:
(2*S*)-3-(4-benzyloxyphenyl)-2-[4-(4-butoxybenzyloxy)benzoylamino]propionic acid methyl ester;
(2*S*)-3-(4-benzyloxyphenyl)-2-[4-(3-bromobenzyloxy)benzoylamino]propionic acid methyl ester;
(2*S*)-3-(4-benzyloxyphenyl)-2-[4-(2-chlorobenzyloxy)benzoylamino]propionic acid methyl ester;
(2*S*)-3-(4-benzyloxyphenyl)-2-[4-(2-fluorobenzyloxy)benzoylamino]propionic acid methyl ester;
(2*S*)-3-(4-benzyloxyphenyl)-2-[4-(3-methylbenzyloxy)benzoylamino]propionic acid methyl ester;
(2*S*)-3-(4-benzyloxyphenyl)-2-4-(3-trifluoromethylbenzyloxy)benzoylamino]propionic acid methyl ester;
(2*S*)-3-(4-benzyloxyphenyl)-2-[4-(2-methoxybenzyloxy)benzoylamino]propionic acid methyl ester;
(2*S*) -3- (4-benzyloxyphenyl) -2- [4- (2-methylbenzyloxy)benzoylamino]propionic acid methyl ester;
(2*S*)-3-(4-benzyloxyphenyl)-2-[4-(2-trifluoromethylbenzyloxy)benzoylamino]propionic acid methyl ester;
(2*S*)-3-(4-benzyloxyphenyl)-2-[4-(2-*o-*tolylethoxy)benzoylamino]propionic acid methyl ester;
(2*S*)-3-(4-benzyloxyphenyl)-2-{4-[3-(4-propoxyphenoxy)propoxy]benzoylamino}propionic acid methyl ester;
(2*S*)-3-(4-benzyloxyphenyl)-2-[4-(3-methoxybenzyloxy)benzoylamino]propionic acid methyl ester;
(2*S*)-3-(4-benzyloxyphenyl)-2-[4-(2-ethoxybenzyloxy)benzoylamino]propionic acid methyl ester;
(2*S*)-3-(4-benzyloxyphenyl)-2-[4-(4-butylbenzyloxy)benzoylamino]propionic acid methyl ester;
(2*S*)-2-[4-(4-butylbenzyloxy)benzoylamino]-3-cyclohexylpropionic acid methyl ester;
(2*S*)-2-{4-[2-(3-methylquinoxalin-2yloxy)ethoxy]benzoylamino}-3-phenylpropionic acid methyl ester;
(2*S*)-3-(4-benzyloxyphenyl)-2-[4-(2-pyridin-2-ylethoxy)benzoylamino]propionic acid methyl ester;
(2*S*)-3-(4-benzyloxyphenyl)-2-{4-[2-(3-methylquinoxalin-2-yloxy)ethoxy]benzoylamino}propionic acid methyl ester;
(2*S*)-3-(4-benzyloxyphenyl)-2-{4-[2-(pyridin-2-yloxy)ethoxy]benzoylamino}propionic acid methyl ester;
(2*S*)-3-(4-benzyloxyphenyl)-2-{4-[2-(quinolin-8-yloxy)ethoxy]benzoylamino}propionic acid methyl ester;
(2*S*)-3-(4-benzyloxyphenyl)-2-{4-[2-(quinolin-7-yloxy)ethoxy]benzoylamino}propionic acid methyl ester;
(2*S*)-3-(4-benzyloxyphenyl)-2-{4-[2-(quinolin-2-yloxy)ethoxy]benzoylamino}propionic acid methyl ester;
(2*S*)-3-(4-benzyloxyphenyl)-2-{4-[3-(3-methylquinoxalin-2-yloxy)propoxy]benzoylamino}propionic acid methyl ester;
(2*S*)-3-(4-bromophenyl)-2-{4-[2-(3-methylquinoxalin-2-yloxy)ethoxy]benzoylamino}propionic acid methyl ester;
(2*S*)-3-(4-fluorophenyl)-2-{4-[2-(3-methylquinoxalin-2-yloxy)ethoxy]benzoylamino}propionic acid methyl ester;
(2*S*)-3-(4-benzyloxyphenyl)-2-{4-[2-(3-methylquinoxalin-2-yloxy)ethoxy]benzoylamino}propionic acid ethyl ester;
(2*S*)-3-(4-benzyloxyphenyl)-2-{4-[2-(3-methylquinoxalin-2-yloxy)ethoxy]benzoylamino}propionic acid isopropyl ester;
(2*S*)-3-(4-benzyloxyphenyl)-2-{4-[2-(3-methylquinoxalin-2-yloxy)ethoxy]benzoylamino}propionic acid propyl ester;
(2*S*)-2-(4-benzyloxybenzoylamino)-3-(4-benzyloxyphenyl)propion ic acid;
(2*S*)-2-[4-(3-benzyloxybenzyloxy)benzoylamino]-3-(4-benzyloxyphenyl)propionic acid;
3-{(3-benzyloxybenzyl)-[4-(2-dibenzylaminoethoxy)benzoy)amino}propionic acid;
3-((3-benzyloxybenzyl)-{3-[2-(3-methylquinoxalin-2-yloxy)ethoxy]benzoyl}amino)propionic acid;
3-{(3-benzyloxybenzyl)-[4-(3-benzyloxybenzyloxy)benzoyl]amino}propionic acid;
2-[4-(4-benzyloxybenzyloxy)benzoylamino]-3-(4-benzyloxyphenyl)propionic acid;
(2*S*)-2-[3-(4-benzyloxybenzyloxy)benzoylamino]-3-(4-benzyloxyphenyl)propionic acid;
3-(4-benzyloxyphenyl)-2-[3-(biphenyl-4-ylmethoxy)benzoylamino]propionic acid;
2-[4-(3-benzyloxybenzyloxy)benzoylamino]-3-(4-bromophenyl)propionic acid;
3-(4-benzyloxyphenyl)-2-[4-(4-butylbenzyloxy)benzoylamino]propionic acid;
2-[-4-(4-butylbenzyloxy)benzoylamino]-3-cyclohexylpropionic acid;
{(3-benzyloxybenzyl)-[4-(4-butylbenzyloxy)benzoyl)amino}acetic acid;
3-{(3-benzyloxybenzyl)-[4-(4-butylbenzyloxy)benzoyl]amino}propionic acid;
3-(4-benzyloxyphenyl)-2-[4-(2-bromobenzyloxy)benzoylamino]propionic acid;
3-(4-benzyloxyphenyl)-2-[4-(2-chlorobenzyloxy)benzoylamino] propionic acid;
3-(4-benzyloxyphenyl)-2-[4-(2-methylbenzyloxy)benzoylamino]propionic acid;
3-(4-benzyloxyphenyl)-2-[4-(3-trifluoromethylbenzyloxy)benzoylamino]propionic acid; and
3-(4-benzyloxyphenyl)-2-[4-(2-trifluoromethylbenzyloxy)benzoylamino]propionic acid.

14. A pharmaceutical composition comprising, as an active ingredient, a therapeutically effective amount of the compound according to any one of the claims 1 to 13 together with appropriate amounts of pharmaceutically acceptable excipients.

15. Use of the compound as defined in any one of claims 1 to 13 for the manufacture of a medicament for the prophylactic and/or curative treatment of diseases in an animal including a human.

16. Use of the compound as defined in any one of claims 1 to 13 for the manufacture of a medicament for the prophylactic and/or curative treatment of PPARγ mediated diseases in an animal including a human.

17. Use of the compound as defined in any one of claims 1 to 13 for the manufacture of a medicament for the prophylactic and/or curative treatment of PPARγ / PPARδ mediated diseases in an animal including a human..

18. Use of the compound as defined in any one of claims 1 to 13 for the manufacture of a medicament for the prophylactic and/or curative treatment of a condition associated with a metabolic disease in an animal including a human.

19. Use according to claim 18, wherein the metabolic disease is non-insulin-dependent diabetes mellitus (NIDDM).

20. Use according to claim 18, wherein the metabolic disease is obesity.

21. Use according to claim 18, wherein the metabolic disease is selected from hypercholesterolaemia, and other lipid-mediated pathologies.

22. Use of the compound as defined in any one of claims 1 to 13 for the manufacture of a medicament for the prophylactic and/or curative treatment of a cardiovascular disease associated with metabolic syndrome in an animal including a human.

23. Use of the compound as defined in any one of claims 1 to 13 for the manufacture of a medicament for the prophylactic and/or curative treatment of inflammation or an inflammatory process in general in an animal including a human.

24. Use according to claim 23, wherein the inflammatory process is selected from rheumatoid arthritis, and atherosclerosis.

25. Use according to claim 23, wherein the inflammatory process is selected from psoriasis, and intestinal inflammatory disease.

26. Use of the compound as defined in any one of claims 1 to 13 for the manufacture of a medicament for the prophylactic and/or curative treatment of a bone disease, particularly osteoporosis, in an animal including a human.

27. Use of the compound as defined in any one of claims 1 to 13 for the manufacture of a medicament for the prophylactic and/or curative treatment of cancer in an animal including a human.

28. Use of the compound as defined in any one of claims 1 to 13 for the manufacture of a medicament for the prophylactic and/or curative treatment of skin wound healing or cutaneous disorders associated with an anomalous differentiation of epidermic cells, particularly the formation of keloids, in an animal including a human.

29. Use according to any of the claims 15 to 28, wherein the medicament is administered orally, parenterally or topically.

## Patentansprüche

1. Verbindung der Formel (I) 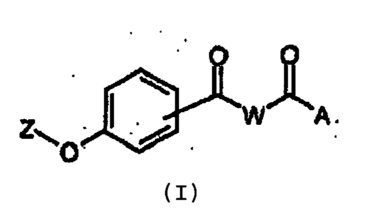 ihre Stereoisomere und deren Mischungen, ihre polymorphen Formen und deren Mischungen sowie die pharmazeutisch unbedenklichen Solvate und Additionssalze sämtlicher der genannten Formen, wobei der zentrale Benzolring in *meta*- oder para-Stellung substituiert sein kann und
-A ein Rest ist, der aus der Gruppe ausgewählt ist, die aus -OR1, NR2OR1 und NR2R3 besteht; wobei R1, R2, und R3 unabhängig voneinander für -H oder -(C₁-C₄)-Alkyl stehen;
-W- ein zweibindiger Rest ist, der aus der folgenden Gruppe gewählt ist: -NH-CH(E)- und -N(D)-CH₂-CH₂-; wobei E ein Rest der Art -G-I-J-K ist und D ein Rest der Art -G-I'-J-K ist, wobei:
-G- eine Bindung oder ein zweibindiger -(CH₂)₁₋₄-Rest ist;
-I- ein zweibindiger Rest einer zyklischen Verbindung ist, die aus den folgenden Gruppen gewählt ist:
a) Cyclopropan, Cyclobutan, Cyclopentan, Cyclohexan und Cyclohexen, von denen sämtliche mit einem oder mehreren Resten substituiert sein können, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
b) einem fünf- oder sechsgliedrigen aromatischen Heterozyklus, der ein bis drei Heteroatome enthält, welche aus O, S und N gewählt sind, wobei dieser Heterozyklus mit einem oder mehreren Resten substituiert sein kann, die unabhängig voneinander aus -OH, Oxo (=O), -CHO, -SH, -NO₂, -CN, -F, -Cl, -Br, (C₁-C₄)-Alkanoyl, (C₁-C₄) -Alkoxycarbonyl, (C₁-C₄) -Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
c) Benzol oder Benzol, das mit einem oder mehreren Resten substituiert ist, die unabhängig voneinander aus -OH, -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
d) einem bizyklischen System, das aus einem Benzol besteht, welches mit einem fünf- oder sechsgliedrigen Ring zusammengefügt ist, der möglicherweise ein bis drei Heteroatome enthält, welche aus O, S und N gewählt sind, wobei dieses bizyklische System mit einem oder mehreren Resten substituiert sein kann, die unabhängig voneinander aus -OH, -Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
-J- eine Bindung oder ein zweibindiger Rest ist, der aus den folgenden Gruppen gewählt ist:
a) (CH₂)₁₋₄-Alkyliden;
b) -O-, und
c) -O-(C₁-C₄)-Alkyl-;
-K- ein Rest ist, der aus den folgenden Gruppen gewählt ist:
a) -H;
b) (C₁-C₄) -Alkyl;
c) einem Rest einer zyklischen Verbindung, die aus den folgenden gewählt ist: Cyclopropan, Cyclobutan, Cyclopentan, Cyclohexan und Cyclohexen, von denen sämtliche mit einem oder mehreren Resten substituiert sein können, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
d) einem fünf- oder sechsgliedrigen Heterozyklus, der ein bis drei Heteroatome enthält, welche aus O, S und N gewählt sind, wobei dieser Heterozyklus mit einem oder mehreren Resten substituiert sein kann, die unabhängig voneinander aus -OH, Oxo (=O) , -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
e) Phenyl oder Phenyl, das mit einem oder mehreren Resten substituiert sein kann, die unabhängig voneinander aus -OH, -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
-I'- ein zweibindiger Rest einer zyklischen Verbindung ist, die aus den folgenden Gruppen gewählt ist:
a) Cyclopropan, Cyclobutan, Cyclopentan, Cyclohexan und Cyclohexen, von denen sämtliche mit einem oder mehreren Resten substituiert sein können, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄) -Alkyloxy-SO₂-, (C₁-C₄)-Alkyl-SO₂-O-, NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
b) einem fünf- oder sechsgliedrigen aromatischen Heterozyklus, der ein bis drei Heteroatome enthält, welche aus O, S und N gewählt sind, wobei dieser Heterozyklus mit einem oder mehreren Resten substituiert sein kann, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
c) Benzol, das mit einem oder mehreren Resten substituiert ist, die unabhängig voneinander aus -OH, -CHO, -SH, -NO₂, -CN, -F, -Cl, -Br, (C₁-C₄) -Alkanoyl, (C₁-C₄) -Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
d) einem bizyklischen System, das aus einem Benzol besteht, welches mit einem fünf- oder sechsgliedrigen Ring zusammengefügt ist, der möglicherweise ein bis drei Heteroatome enthält, welche aus O, S und N gewählt sind, wobei dieses bizyklische System mit einem oder mehreren Resten substituiert sein kann, die unabhängig voneinander aus -OH, -Oxo (=O), -CHO, -SH, -NO₂, -CN, -F, -Cl, -Br, (C₁-C₄)-Alkanoyl , (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
-Z ein Rest ist, der aus den folgenden Gruppen gewählt ist:
a) -G-I-J-T, wobei
-Q- ein zweibindiger -(CH₂)₁₋₃-Rest ist;
-I- der obigen Begriffsbestimmung entspricht;
-J- der obigen Begriffsbestimmung entspricht; und
-T ein Rest ist, der aus den folgenden Gruppen gewählt ist:
a.a) -H;
a.b) (C₁-C₄)-Alkyl;
a.c) einem Rest einer zyklischen Verbindung, die aus den folgenden gewählt ist: Cyclopropan, Cyclobutan, Cyclopentan, Cyclohexan und Cyclohexen, von denen sämtliche mit einem oder mehreren Resten substituiert sein können, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O- , -NR2R3, -CONR2R3, (C₁-C₄)-Alkyl, das möglicherweise mit einem oder mehreren -OH oder -F substituiert ist, und C₁-C₄)-Alkoxyl, das möglicherweise mit einem oder mehreren -OH oder -F substituiert ist, gewählt sind;
a.d) einem Rest eines fünf- oder sechsgliedrigen Heterozyklus, der ein bis drei Heteroatome enthält, welche aus O, S und N gewählt sind, wobei dieser Heterozyklus mit einem oder mehreren Resten substituiert sein kann, die unabhängig voneinander aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O- , -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
a.e) Phenyl oder Phenyl, das mit einem oder mehreren Resten substituiert sein kann, die unabhängig voneinander aus -OH, -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄) -Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
a.f) einem Rest eines bizyklischen Systems, das aus einem Benzol besteht, welches mit einem fünf- oder sechsgliedrigen Ring zusammengefügt ist, der möglicherweise ein bis drei Heteroatome enthält, welche aus O, S und N gewählt sind, wobei dieses bizyklische System mit einem oder mehreren Resten substituiert sein kann, die unabhängig voneinander aus -OH, -Oxo (=O), -CHO, -SH, -NO₂, -CN, -F, -Cl, -Br, (C₁-C₄)-Alkanoyl , (C₁-C₄) -Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
b) -(CH₂)ₛ-X-P-I-J-T, wobei
s gleich 2 oder 3 ist;
-x- aus der Gruppe gewählt ist, die aus -O-, -S-, -SO-, -SO₂- und -NR4- besteht, wobei R4 ein Rest ist, der aus der folgenden Gruppe gewählt ist:
b.a) -H;
b.b) (C₁-C₁₀) -Alkyl;
b.c) Cycloalkyl, Cycloalkyl-CO-, Cycloalkyl-(C₁-C₃)-alkyl und Cycloalkyl-(C₁-C₃)-alkanoyl , wobei das Cycloalkyl ein fünf- oder sechsgliedriger Ring ist, der mit einem oder mehreren Resten substituiert sein kann, die aus -OH, Oxo (=O), -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₂-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
b.d) Phenyl, Phenyl-CO-, Phenyl-(C₁-C₃)-alkyl und Phenyl-(C₁-C₃)-alkanoyl, wobei dieser aromatische Ring mit einem oder mehreren Resten substituiert sein kann, die aus -OH, -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
b.e) einem Heterozyklus, Heterozyklus-CO, Heterozyklus (C₁-C₃)-alkyl und Heterozyklus-(C₁-C₃)-alkanoyl, wobei der Heterozyklus ein fünf- oder sechsgliedriger Ring ist, der ein bis drei Heteroatome enthält, welche aus O, S und N gewählt sind, wobei dieser Heterozyklus mit einem oder mehreren Resten substituiert sein kann, die aus -OH, Oxo (=O), -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
-P- eine Bindung oder ein zweibindiger-(CH₂)₁₋₄-Rest ist;
-I- der obigen Begriffsbestimmung entspricht; -J- der obigen Begriffsbestimmung entspricht; und
-T ein Rest ist, welcher der obigen Begriffsbestimmung entspricht;
c) -(CH₂)ᵤ, -CO-NR5-P-I-J-T, wobei
u gleich 1 oder 2 ist;
-R5 ein Rest ist, der aus der folgenden Gruppe gewählt ist:
c.a) -H;
c.b) (C₁-C₁₀) -Alkyl;
c.c) Cycloalkyl und Cycloalkyl-(C₁-C₃)-alkyl, wobei das Cycloalkyl ein fünf- oder sechsgliedriger Ring ist, der mit einem oder mehreren Resten substituiert sein kann, die aus -OH, Oxo (=O), -CHO, -SH, -NO₂, -CN, -F, -Cl, -Br, (C₁-C₄)-Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
c.d) Phenyl und Phenyl-(C₁-C₃)-alkyl, wobei dieser aromatische Ring mit einem oder mehreren Resten substituiert sein kann, die aus -OH, -CHO, -SH, -NO₂, -CN, -F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O-, -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
c.e) einem Heterozyklus und Heterozyklus (C₁-C₃)-alkyl, wobei dieser Heterozyklus ein fünf- oder sechsgliedriger Ring ist, der ein bis drei Heteroatome enthält, welche aus O, S und N gewählt sind, wobei dieser Heterozyklus mit einem oder mehreren Resten substituiert sein kann, die aus -OH, Oxo (=O), -CHO, -SH, -NO₂, -CN, - F, -Cl, -Br, (C₁-C₄) -Alkanoyl, (C₁-C₄)-Alkoxycarbonyl, (C₁-C₄)-Alkanoyloxy, (C₁-C₄)-Alkylsulfinyl, (C₁-C₄)-Alkylsulfanyl, (C₁-C₄)-Alkylsulfonyl, (C₁-C₄)-Alkyloxy-SO₂-, (C₁-C₄)-Alkyl-SO₂-O- , -NR2R3, -CONR2R3, (C₁-C₄)-Alkyl, das möglicherweise mit einem oder mehreren -OH oder -F substituiert ist, und (C₁-C₄)-Alkoxyl, das möglicherweise mit einem oder mehreren -OH oder -F substituiert ist, gewählt sind;
-P- der obigen Begriffsbestimmung entspricht;
-I- der obigen Begriffsbestimmung entspricht;
-J- der obigen Begriffsbestimmung entspricht; und
-T der obigen Begriffsbestimmung entspricht;
mit der Maßgabe, dass es sich bei der Verbindung der Formel (I) weder um 2-(4-Benzyloxybenzoylamino)-3-phenylpropionsäure, noch um 2-[4-(4-Methoxybenzyloxy)benzoylamino]-3-phenylpropionsäure, 2-[4-(4-Brombenzyloxy)benzoylamino]-3-phenylpropionsäure, Cyclopentyl-[4-(2-methylchinolin-4-ylmethoxy)benzoylamino]essigsäuremethylester, [4-(2-Methylchinolin-4-ylmethoxy)benzoylamino](tetrahydropyran-4-yl)essigsäuremethylester oder um 2-(4-Benzyloxybenzoylamino)-3-biphenyl-4-ylpropionsäure oder um (4'-Trifluormethoxybiphenyl-4-yl)propionsäure handelt.

2. Verbindung nach Anspruch 1, wobei W gleich NH-CH(E)- ist.

3. Verbindung nach Anspruch 2, wobei -Z ein Rest der Art -Q-I-J-T ist.

4. Verbindung nach Anspruch 2, wobei -Z ein Rest der Art -(CH₂)ₛ-X-P-I-J-T ist.

5. Verbindung nach Anspruch 4, wobei -X- gleich -0- ist.

6. Verbindung nach Anspruch 4, wobei s gleich 2 ist und -X-gleich -NR4- ist.

7. Verbindung nach Anspruch 1, wobei W gleich -N(E)-CH₂-CH₂- ist.

8. Verbindung nach Anspruch 7, wobei -Z ein Rest der Art -Q-I-J-T ist.

9. Verbindung nach Anspruch 7, wobei -Z ein Rest der Art -(CH₂)ₛ-X-P-I-J-T ist.

10. Verbindung nach Anspruch 9, wobei -X- gleich -0- ist.

11. Verbindung nach Anspruch 9, wobei s gleich 2 ist und -X-gleich -NR4- ist.

12. Verbindung nach Anspruch 1, wobei -A ein Rest der Art -OR1 ist.

13. Verbindung nach Anspruch 1, ausgewählt aus der Gruppe bestehend aus:
(2*S*)-3-(4-Benzyloxyphenyl)-2-[4-(4-butoxybenzyloxy)benzoylamino]propionsäuremethylester;
(2*S*)-3-(4-Benzyloxyphenyl)-2-C4-(3-brombenzyloxy)benzoylamino]propionsäuremethylester;
(2*S*)-3-(4-Benzyloxyphenyl)-2-[4-(2-chlorbenzyloxy)benzoylamino]propionsäuremethylester;
(2*S*)-3-(4-Benzyloxyphenyl)-2-[4-(2-fluorbenzyloxy)benzoylamino]propionsäuremethylester;
(2*S*)-3-(4-Benzyloxyphenyl)-2-[4-(3-methylbenzyloxy)benzoylaminopropionsäuremethylester;
(2*S*)-3-(4-Benzyloxyphenyl)-2-[4-(3-trifluormethylbenzyloxy)benzoylamino]propionsäuremethylester;
(2*S*)-3-(4-Benzyloxyphenyl)-2-[4-(2-methoxybenzyloxy)benzoylamino]propionsäuremethylester;
(2*S*)-3-(4-Benzyloxyphenyl)-2-[4-(2-methylbenzyloxy)benzoylamino]propionsäuremethylester;
(2*S*)-3-(4-Benzyloxypbenyl)-2-[4-(2-trifluormethylbenzyloxy)benzoylamino]propionsäuremethylester;
(2*S*)-3-(4-Benzyloxyphenyl)-2-[4-(2-o-tolylethoxy)benzoylamino]propionsäuremethylester;
(2*S*)-3-(4-Benzyloxyphenyl)-2-{4-[3-(4-propoxyphenoxy)propoxy]benzoylamino}propionsäuremethylester
(2*S*)-3-(4-Benzyloxyphenyl)-2-[4-(3-methoxybenzyloxy)benzoylamino]propionsäuremethylester;
(2S)-3-(4-Benzyloxyphenyl)-2-[4-(2-ethoxybenzyloxy)benzoylamino]propionsäuremethylester;
(2*S*)-3-(4-Benzyloxyphenyl)-2-[4-(4-butylbenzyloxy)benzoylamino]propionsäuremethylester;
(2*S*)-2-[4-(4-Butylbenzyloxy)benzoylamino]-3-cyclohexylpropionsäuremethylester;
(2*S*)-2-{4-[2-(3-Methylchinoxalin-2yloxy)ethoxy]benzoylamino}-3-phenylpropionsäuremethylester;
(2*S*)-3-(4-Benzyloxyphenyl)-2-[4-(2-pyridin-2-ylethoxy)benzoylamino]propionsäuremethylester;
(2*S*)-3-(4-Benzyloxyphenyl)-2-[4-(2-(3-methylchinoxalin-2-yloxy)ethoxylbenzoylamino)propionsäuremethylester;
(2*S*)-3-(4-Benzyloxyphenyl)-2-{4-[2-(pyridin-2-yloxy)ethoxy]benzoylamino}propionsäuremethylester;
(2*S*)-3-(4-Benzyloxyphenyl)-2-{4-[2-(chinolin-8-yloxy)ethoxy]benzoylamino}propionsäuremethylester;
(2*S*)-3-(4-Benzyloxyphenyl)-2-{4-[2-(chinolin-7-yloxy)ethoxy]benzoylamino}propionsäuremethylester;
(2*S*)-3-(4-Benzyloxyphenyl)-2-{4-[2-(chinolin-2-yloxy)ethoxy]benzoylamino}propionsäuremethylester;
(2*S*)-3-(4-Benzyloxyphenyl)-2-{4-[3-(3-methylchinoxalin-2-yloxy)propoxy]benzoylamino}propionsäuremethylester;
(2*S*)-3-(4-Bromphenyl)-2-{4-[2-(3-methylchinoxalin-2-yloxy)ethoxythenzoylamino]propionsäuremethylester;
(2*S*)-3-(4-Fluorphenyl)-2-{4-[2-(3-methylchinoxalin-2-yloxy)ethoxy]benzoylamino}propionsäuremethylester;
(2*S*)-3-(4-Benzyloxyphenyl)-2-{4-[2-(3-methylchinoxalin-2-yloxy)ethoxy]benzoylamino}propionsäureethylester;
(2*S*)-3-(4-Benzyloxyphenyl)-2-{4-[2-(3-methylchinoxalin-2-yloxy)ethoxy]benzoylamino}propionsäureisopropylester;
(2*S*)-3-(4-Benzyloxyphenyl)-2-{4-[2-(3-methylchinoxalin-2-yloxy)ethoxy]benzoylamino}propionsäurepropylester;
(2*S*)-2-(4-Benzyloxybenzoylamino)-3-(4-benzyloxyphenyl)propionsäure;
(2*S*)-2-[4-(3-Benzyloxybenzyloxy)benzoylamino]-3-(4-benzyloxyphenyl)propionsäure;
3-{(3-Benzyloxybenzyl)-[4-(2-dibenzylaminoethoxy)benzoyl]amino}propionsäure;
3-{(3-Benzyloxybenzyl){3-[2-(3-methylchinoxalin-2-yloxy)ethoxy]benzoyl}amino)propionsäure;
3-{(3-Benzyloxybenzyl)-[4-(3-benzyloxybenzyloxy)benzoyl]amino}propionsäure;
2-[4-(4-Benzyloxybenzyloxy)benzoylamino]-3-(4-benzyloxyphenyl)propionsäure;
(2*S*)-2-[3-(4-Benzyloxybenzyloxy)benzoylamino]-3-(4-benzyloxyphenyl)propionsäure;
3-(4-Benzyloxyphenyl)-2-[3-(biphenyl-4-ylmethoxy)benzoylaminopropionsäure;
2-[4-(3-Benzyloxybenzyloxy)benzoylamino]-3-(4-bromphenyl)propionsäure;
3-(4-Benzyloxyphenyl)-2-[4-(4-butylbenzyloxy)benzoylamino]propionsäure;
2-[4-(4-Butylbenzyloxy)benzoylamino]-3-cyclohexylpropionsäure;
{(3-Benzyloxybenzyl)-[4-(4-butylbenzyloxy)benzoyl]amino}essigsäure;
3-{(3-Benzyloxybenzyl)-[4-(4-butylbenzyloxy)benzoyl]amino}propionsäure;
3-(4-Benzyloxyphenyl)-2-[4-(2-brombenzyloxy)benzoylamino]propionsäure;
3-(4-Benzyloxyphenyl)-2-[4-(2-chlorbenzyloxy)benzoylamino]propionsäure;
3-(4-Benzyloxyphenyl)-2-[4-(2-methylbenzyloxy)benzoylamino]propionsäure;
3-(4-Benzyloxyphenyl)-2-[4-(3-trifluormethylbenzyloxy)benzoylamino]propionsäure; und
3-(4-Benzyloxyphenyl)-2-[4-(2-trifluormethylbenzyloxy)benzoylamino]propionsäure.

14. Pharmazeutische Zusammensetzung, die als Wirkstoff eine therapeutisch wirksame Menge der Verbindung nach einem beliebigen der Ansprüche 1 bis 13 sowie zweckmäßige Mengen an pharmazeutisch unbedenklichen Trägerstoffen enthält.

15. Verwendung der Verbindung gemäß der Begriffsbestimmung in einem beliebigen der Ansprüche 1 bis 13 zur Herstellung eines Arzneimittels zur prophylaktischen und/oder heilenden Behandlung von Krankheiten bei einem Tier einschließlich eines Menschen.

16. Verwendung der Verbindung gemäß der Begriffsbestimmung in einem beliebigen der Ansprüche 1 bis 13 zur Herstellung eines Arzneimittels zur prophylaktischen und/oder heilenden Behandlung von PPARγ-vermittelten Krankheiten bei einem Tier einschließlich eines Menschen.

17. Verwendung der Verbindung gemäß der Begriffsbestimmung in einem beliebigen der Ansprüche 1 bis 13 zur Herstellung eines Arzneimittels zur prophylaktischen und/oder heilenden Behandlung von PPARγ/PPARδ-vermittelten Krankheiten bei einem Tier einschließlich eines Menschen.

18. Verwendung der Verbindung gemäß der Begriffsbestimmung in einem beliebigen der Ansprüche 1 bis 13 zur Herstellung eines Arzneimittels zur prophylaktischen und/oder heilenden Behandlung eines Zustands, der mit einer Stoffwechselkrankheit in Verbindung steht, bei einem Tier einschließlich eines Menschen.

19. Verwendung nach Anspruch 18, wobei es sich bei der Stoffwechselkrankheit um Formen des Diabetes mellitus handelt, bei denen keine Insulingabe indiziert ist (NIDDM).

20. Verwendung nach Anspruch 18, wobei es sich bei der Stoffwechselkrankheit um Fettleibigkeit handelt.

21. Verwendung nach Anspruch 18, wobei die Stoffwechselkrankheit aus Hypercholesterinämie und anderen lipidvermittelten Erkrankungen gewählt ist.

22. Verwendung der Verbindung gemäß der Begriffsbestimmung in einem beliebigen der Ansprüche 1 bis 13 zur Herstellung eines Arzneimittels zur prophylaktischen und/oder heilenden Behandlung einer Herz-Kreislauf-Krankheit, die mit dem metabolischen Syndrom in Verbindung steht, bei einem Tier einschließlich eines Menschen.

23. Verwendung der Verbindung gemäß der Begriffsbestimmung in einem beliebigen der Ansprüche 1 bis 13 zur Herstellung eines Arzneimittels zur prophylaktischen und/oder heilenden Behandlung einer Entzündung oder, allgemeiner gesagt, eines entzündlichen Vorgangs bei einem Tier einschließlich eines Menschen.

24. Verwendung nach Anspruch 23, wobei der entzündliche Vorgang aus rheumatoider Arthritis und Arteriosklerose gewählt ist.

25. Verwendung nach Anspruch 23, wobei der entzündliche Vorgang aus Schuppenflechte und chronisch-entzündlicher Darmerkrankung gewählt ist.

26. Verwendung der Verbindung gemäß der Begriffsbestimmung in einem beliebigen der Ansprüche 1 bis 13 zur Herstellung eines Arzneimittels zur prophylaktischen und/oder heilenden Behandlung einer Knochenkrankheit, insbesondere von Osteoporose, bei einem Tier einschließlich eines Menschen.

27. Verwendung der Verbindung gemäß der Begriffsbestimmung in einem beliebigen der Ansprüche 1 bis 13 zur Herstellung eines Arzneimittels zur prophylaktischen und/oder heilenden Behandlung von Krebs bei einem Tier einschließlich eines Menschen.

28. Verwendung der Verbindung gemäß der Begriffsbestimmung in einem beliebigen der Ansprüche 1 bis 13 zur Herstellung eines Arzneimittels zur prophylaktischen und/oder heilenden Behandlung von Störungen der Hautwundheilung oder kutanen Störungen, die mit einer anormalen Ausdifferenzierung von Zellen der Oberhaut in Verbindung stehen, insbesondere der Bildung von Keloiden, bei einem Tier einschließlich eines Menschen.

29. Verwendung gemäß einem beliebigen der Ansprüche 15 bis 28, wobei das Arzneimittel oral, parenteral oder topisch verabreicht wird.

## Revendications

1. Composé de formule (I), 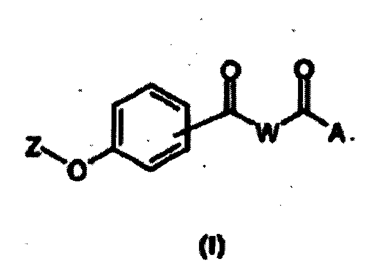 ses stéréo-isomères et leurs mélanges, ses polymorphes et leurs mélanges et les solvates et sels d'addition pharmaceutiquement acceptables de tous ceux-ci, dans lequel le cycle benzène central peut être substitué en position méta ou para et,
-A est un radical choisi dans le groupe consistant en -OR1, -NR2OR1 et -NR2R3 ;
où R1, R2 et R3 représentent indépendamment -H ou un groupe alkyle (en C₁ à C₄) ;
-W- est un biradical choisi dans le groupe : -NH-CH(E)- et -N(D)-CH₂-CH₂- ; où E est un radical du type -G-I-J-K et D est un radical du type -G-I'-J-K où :
-G- est une liaison ou un biradical -(CH₂)₁₋₄- ;
-I- est un biradical d'un cycle choisi parmi les groupes suivants :
a) cyclopropane, cyclobutane, cyclopentane, cyclohexane et cyclohexène, tous étant éventuellement substitués par un ou plusieurs radicaux choisis indépendamment parmi -OH, oxo (=O), -CHO, -SH, -NO₂, -CN, -F, -Cl, -Br, les groupes alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs groupes -OH ou -F, et un groupe alcoxyle (en C₁ à C₄) éventuellement substitué par un ou plusieurs -OH ou -F ;
b) un hétérocycle aromatique de cinq ou six chaînons contenant de un à trois hétéroatomes choisis parmi O, S et N, cet hétérocycle étant éventuellement substitué par un ou plusieurs radicaux choisis indépendamment parmi -OH, oxo (=O), -CHO, -SH, -NO₂, -CN, -F, -Cl, -Br, un groupe alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs groupes -OH ou -F, et un groupe alcoxyle (en C₁ à C₄) éventuellement substitué par un ou plusieurs -OH ou -F ;
c) benzène ou benzène substitué par un ou plusieurs radicaux choisis indépendamment parmi -OH, -CHO, -SH, -NO₂, -CN, -F, -C₁, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O NR2R3, -CONR2R3, alkyle (en C₁ à C₄) éventuellement substitué par un ou plusieurs -OH ou -F et un groupe alcoxyle (en C₁ à C₄) éventuellement substitué par un ou plusieurs -OH ou -F ; et
d) un système bicyclique consistant en un groupe benzène fusionné à un cycle de cinq ou six chaînons contenant éventuellement de un à trois hétéroatomes choisis parmi O, S et N, ce système bicyclique étant éventuellement substitué par un ou plusieurs radicaux choisis indépendamment parmi -OH, oxo (=O), -CHO, -SH, -NO₂ -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F et alcoxyle (en C₁ à C₄) éventuellement substitué par un ou plusieurs -OH ou -F ;
-J- est une liaison ou un biradical choisi parmi les groupes suivants :
a) -(CH₂)₁₋₄-alkylidène ;
b) -O- et
c) -O-alkyle (en C₁ à C₄) ;
-K est un radical choisi parmi les groupes suivants :
a) -H ;
b) alkyle (en C₁ à C₄) ;
c) un radical d'un cycle choisi parmi les suivants : cyclopropane, cyclobutane, cyclopentane, cyclohexane et cyclohexène, tous étant éventuellement substitués par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F et alcoxy (en C₁ à C₄) éventuellement substitué par un ou plusieurs -OH ou -F ;
d) un radical d'un hétérocycle de cinq ou six chaînons contenant de un à trois hétéroatomes choisis parmi O, S et N, cet hétérocycle étant éventuellement substitué par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂, alkyle (en C₁ à C₄)-SO₂O, NR2R3, -CONR2R3, alkyle (en C₁ à C₄) éventuellement substitué par un ou plusieurs -OH ou -F et alcoxyle (en C₁ à C₄) éventuellement substitué par un ou plusieurs -OH ou -F ; et
e) phényle ou phényle éventuellement substitué par un ou plusieurs radicaux choisis indépendamment parmi -OH, -CHO, -SH, -NO₂ -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F et un groupe alcoxyle (en C₁ à C₄) éventuellement substitué par un ou plusieurs -OH ou -F ;
-I'- est un biradical d'un cycle choisi parmi les groupes suivants :
a) cyclopropane, cyclobutane, cyclopentane, cyclohexane et cyclohexène, tous étant éventuellement substitués par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F et alcoxyle (en C₁ à C₄) éventuellement substitué par un ou plusieurs -OH ou -F ;
b) un hétérocycle aromatique de cinq ou six chaînons contenant de un à trois hétéroatomes choisis parmi O, S et N, cet hétérocycle étant éventuellement substitué par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanoyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
c) benzène substitué par un ou plusieurs radicaux choisis indépendamment parmi -OH, -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O- -NR2R3, -CONR2R3, alkyle (en C₁ à C₄) éventuellement substitué par un ou plusieurs OH ou -F, alcoxyle (en C₁ à C₄) éventuellement substitué par un ou plusieurs -OH ou -F et
d) un système bicyclique consistant en un benzène fusionné avec un cycle de cinq ou six chaînons contenant éventuellement de un à trois hétéroatomes choisis parmi O, S et N, ce système bicyclique étant éventuellement substitué par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O- NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
-Z est un radical choisi parmi les groupes suivants :
a) -Q-I-J-T, où
-Q- est un biradical -(CH₂)₁₋₃- ;
-I- est tel que défini ci-dessus ;
-J- est tel que défini ci-dessus ; et
-T est un radical choisi parmi les groupes suivants :
a.a) -H ;
a.b) alkyle (en C₁ à C₄) ;
a.c) un radical d'un cycle choisi parmi les suivants : cyclopropane, cyclobutane, cyclopentane, cyclohexane et cyclohexène, tous étant éventuellement substitués par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
a.d) un radical d'un hétérocycle de cinq ou six chaînons contenant de un à trois hétéroatomes choisis parmi O, S et N, cet hétérocycle étant éventuellement substitué par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
a.e) phényle ou phényle éventuellement substitué par un ou plusieurs radicaux choisis indépendamment parmi -OH, -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ; et
a.f) un radical d'un système bicyclique consistant en un benzène fusionné à un cycle de cinq ou six chaînons contenant éventuellement de un à trois hétéroatomes choisis parmi O, S et N, ce système bicyclique étant éventuellement substitué par un ou plusieurs radicaux choisis indépendamment parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou
-F, et alcoxyle (en C₁ à C₄) éventuellement substitué par un ou plusieurs -OH ou -F ;
b) -(CH₂)ₛ-X-P-I-J-T où
s vaut 2 ou 3 ;
-X- est choisi dans le groupe comprenant -O-, -S-, -SO-, -SO₂- et -NR4-, R4 étant un radical choisi dans le groupe :
b.a) -H ;
b.b) un groupe alkyle (en C₁ à C₁₀),
b.c) cycloalkyle, cycloalkyle-CO-, cycloalkyle-alkyle (en C₁ à C₃), et cycloalkyle-alcanoyle (en C₁ à C₃), le groupe cycloalkyle étant un cycle de cinq à six chaînons éventuellement substitué par un ou plusieurs radicaux choisis parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂- alkyle (en C₁ à C₄)-SO₂O-, -NR2R3, -CONR2R3, alkyle (en C₁ à C₄) éventuellement substitué par un ou plusieurs -OH ou -F, et alcoxyle (en C₁ à C₄) éventuellement substitué par un ou plusieurs OH ou F ;
b.d) un groupe phényle, phényl-CO-, phényl-alkyle (en C₁ à C₃) et phényle-alcanoyle (en C₁ à C₃), ce cycle aromatique étant éventuellement substitué par un ou plusieurs radicaux choisis parmi -OH, -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à ₄)-SO₂- alkyle (en C₁ à C₄)-SO₂O, -NR2R3, -CONR2R3,alkyle (en C₁ à C₄) éventuellement subsitué par un ou plusieurs -OH ou -F, et alcoxyle (en C₁ à C₄) éventuellement substitué par un ou plusieurs -OH ou -F ; et
b.e) un hétérocycle, hétérocycle-CO, hétérocycle-alkyle (en C₁ à C₃) et hétérocycle-alcanoyle (en C₁ à C₃), l'hétérocycle étant un cycle de cinq ou six chaînons contenant de un à trois hétéroatomes choisis parmi O, S et N, cet hétérocycle étant éventuellement substitué par un ou plusieurs radicaux choisis parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
-P- est une liaison ou un biradical -(CH₂)₁₋₄ ;
-I- est tel que défini ci-dessus ;
-J- est tel que défini ci-dessus ; et
-T est un radical tel que défini ci-dessus ;
c) -(CH₂)ᵤ-CO-NR5-P-I-J-T, où
u vaut 1 ou 2 ;
-R5 est un radical choisi dans le groupe :
c.a) -H,
c.b) alkyle (en C₁ à C₁₀) ;
c.c) cycloalkyle et cycloalkyle-alkyle (en C₁ à C₃), où le groupe cycloalkyle est un cycle de cinq à six chaînons éventuellement substitués par un ou plusieurs radicaux choisis parmi -OH, oxo (=O), -CHO, -SH, -NO₂ -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ;
c.d) phényle et phényle-alkyle (en C₁ à C₃), ce cycle aromatique étant éventuellement substitué par un ou plusieurs radicaux choisis parmi -OH, -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ à C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ; et
c.e) un hétérocycle et un hétérocycle-alkyle (en C₁ à C₃), l'hétérocycle étant un cycle de cinq ou six chaînons contenant de un à trois hétéroatomes choisis parmi O, S et N, cet hétérocycle étant éventuellement substitué par un ou plusieurs radicaux choisis parmi -OH, oxo (=O), -CHO, -SH, -NO₂, -CN, -F, -Cl, -Br, alcanoyle (en C₁ à C₄), alcoxycarbonyle (en C₁ à C₄), alcanoyloxy (en C₁ à C₄), alkylsulfinyle (en C₁ à C₄), alkylsulfanyle (en C₁ C₄), alkylsulfonyle (en C₁ à C₄), alkyloxy (en C₁ à C₄)-SO₂-, alkyle (en C₁ à C₄)-SO₂O-, -NR2R3, -CONR2R3, alkyle (en C₁ à C₄) éventuellement substitué par un ou plusieurs -OH ou -F, et alcoxyle (en C₁ à C₄) éventuellement substitué par un ou plusieurs -OH ou -F ; et
-P- est tel que défini ci-dessus ;
-I- est tel que défini ci-dessus ;
-J- est tel que défini ci-dessus ; et
-T est tel que défini ci-dessus ;
à condition que le composé de formule (I) ne soit ni l'acide 2-(4-benzyloxybenzoylamino)-3-phénylpropionique, l'acide 2-[4-(4-méthoxybenzyloxy)benzoylamino]-3-phénylpropionique, l'ester méthylique de l'acide 2-[4-(4-bromobenzyloxy)benzoylamino]-3-phénylpropionique, l'ester méthylique de l'acide cyclopentyl-[4-(2-méthylquinolin-4-yl méthoxy)benzoylamino]acétique méthylester, l'ester méthylique de l'acide [4-(2-méthylquinolin-4-yl méthoxy)benzoylamino](tétrahydropyran-4-yl)acétique ou l'acide 2-(4-benzyloxybenzoylamino)-3-biphényl-4-yl propionique ou l'acide 2-(4-benzyloxybenzoylamino)-3-(4'-trifluorométhoxybiphényl-4-yl)propionique.

2. Composé selon la revendication 1, dans lequel W est -NH-CH(E)- .

3. Composé selon la revendication 2, dans lequel -Z est un radical du type -Q-I-J-T.

4. Composé selon la revendication 2, dans lequel -Z est un radical du type -(CH₂)ₛ-X-P-I-J-T.

5. Composé selon la revendication 4, dans lequel -X- est -O-.

6. Composé selon la revendication 4, dans lequel s vaut 2 et -X- est -NR4-.

7. Composé selon la revendication 1, dans lequel W est -N(E)-CH₂-CH₂-.

8. Composé selon la revendication 7, dans lequel -Z est un radical du type -Q-I-J-T.

9. Composé selon la revendication 7, dans lequel -Z est un radical du type -(CH₂)ₛ-X-P-I-J-T.

10. Composé selon la revendication 9, dans lequel -X- est -O-.

11. Composé selon la revendication 9, dans lequel s vaut 2 et -X- est -NR4-.

12. Composé selon la revendication 1, dans lequel -A est un radical de type -OR1.

13. Composé selon la revendication 1, choisi dans le groupe comprenant :
l'ester méthylique de l'acide (2*S*)-3-(4-benzyloxyphényl)-2-[4-(4-butoxybenzyloxy)benzoylamino] propionique ;
l'ester méthylique de l'acide (2*S*)-3-(4-benzyloxyphényl)-2-[4-(3-bromobenzyloxy)benzoylamino] propionique ;
l'ester méthylique de l'acide (2*S*)-3-(4-benzyloxyphényl)-2-[4-(2-chlorobenzyloxy)benzoylamino] propionique ;
l'ester méthylique de l'acide (2*S*)-3-(4-benzyloxyphényl)-2-[4-(2-fluorobenzyloxy)benzoylamino] propionique ;
l'ester méthylique de l'acide (2*S*)-3-(4-benzyloxyphényl)-2-[4-(3-méthylbenzyloxy)benzoylamino] propionique ;
l'ester méthylique de l'acide (2*S*)-3-(4-benzyloxyphényl)-2-[4-(3-trifluorométhylbenzyloxy)benzoylamino] propionique;
l'ester méthylique de l'acide (2*S*)-3-(4-benzyloxyphényl)-2-[4-(2-méthoxybenzyloxy)benzoylamino] propionique ;
l'ester méthylique de l'acide (2*S*)-3-(4-benzyloxyphényl)-2-[4-(2-méthylbenzyloxy)benzoylamino] propionique ;
l'ester méthylique de l'acide (2*S*)-3-(4-benzyloxyphényl)-2-[4-(2-trifluorométhylbenzyloxy)benzoylamino] propionique ;
l'ester méthylique de l'acide (2*S*)-3-(4-benzyloxyphényl)-2-[4-(2-o-tolyléthoxy)benzoylamino]propionique ;
l'ester méthylique de l'acide (2*S*)-3-(4-benzyloxyphényl)-2-{4-[3-(4-propoxyphénoxy)propoxy] benzoylamino}propionique ;
l'ester méthylique de l'acide (2*S*)-3-(4-benzyloxyphényl)-2-[4-(3-méthoxybenzyloxy)benzoylamino] propionique ;
l'ester méthylique de l'acide (2*S*)-3-(4-benzyloxyphényl)-2-[4-(2-éthoxybenzyloxy)benzoylamino] propionique ;
l'ester méthylique de l'acide (2*S*)-3-(4-benzyloxyphényl)-2-[4-(4-butylbenzyloxy)benzoylamino] propionique ;
l'ester méthylique de l'acide (2*S*)-2-[4-(4-butylbenzyloxy)benzoylamino]-3-cyclohexylpropionique ;
l'ester méthylique de l'acide (2*S*)-2-{4-[2-(3-méthylquinoxalin-2yl oxy)éthoxy]benzoylamino}-3-phénylpropionique;
l'ester méthylique de l'acide (2*S*)-3-(4-benzyloxyphényl)-2-[4-(2-pyridin-2-yl éthoxy)benzoyl-amino] propionique ;
l'ester méthylique de l'acide (2*S*)-3-(4-benzyloxyphényl)-2-{4-[2-(3-méthylquinoxalin-2-yl oxy) éthoxy]benzoylamino}propionique ;
l'ester méthylique de l'acide (2*S*)-3-(4-benzyloxyphényl)-2-{4-[2-(pyridin-2-yl oxy)éthoxy]benzoylamino} propionique ;
l'acide (2*S*)-3-(4-benzyloxyphényl)-2-{4-[2-(quinolin-8-yloxy)éthoxy]benzoylamino} propionique ;
l'ester méthylique de l'acide (2*S*)-3-(4-benzyloxyphényl)-2-{4-[2-(quinolin-7-yl oxy)éthoxy]benzoylamino} propionique ;
l'ester méthylique de l'acide (2*S*)-3-(4-benzyloxyphényl)-2-{4-[2-(quinolin-2-yl oxy)éthoxy]benzoylamino} propionique ;
l'ester méthylique de l'acide (2*S*)-3-(4-benzyloxyphényl)-2-{4-[3-(3-méthylquinoxalin-2-yloxy)propoxyl] benzoylamino}propionique;
l'ester méthylique de l'acide (2*S*)-3-(4-bromophényl)-2-{4-[2-(3-méthylquinoxalin-2-yl oxy)éthoxy] benzoylamino}propionique;
l'ester méthylique de l'acide (2*S*)-3-(4-fluorophényl)-2-{4-[2-(3-méthylquinoxalin-2-yl oxy)éthoxy] benzoylamino}propionique ;
l'ester éthylique de l'acide (2*S*)-3-(4-benzyloxyphényl)-2-{4-[2-(3-méthylquinoxalin-2-yl oxy)éthoxy] benzoylamino}propionique ;
l'ester isopropylique de l'acide (2*S*)-3-(4-benzyloxyphényl)-2-{4-[2-(3-méthylquinoxalin-2-yl oxy)éthoxy] benzoylamino}propionique;
l'ester propylique de l'acide (2*S*)-3-(4-benzyloxyphényl)-2-{4-[2-(3-méthylquinoxalin-2-yl oxy)éthoxy] benzoylamino}propionique;
l'acide (2*S*)-2-(4-benzyloxybenzoylamino)-3-(4-benzyloxyphényl)propionique ;
l'acide (2*S*)-2-[4-(3-benzyloxybenzyloxy)benzoylamino]-3-(4-benzyloxyphényl) propionique ;
l'acide 3-{(3-benzyloxybenzyl)-[4-(2-dibenzylaminoéthoxy)benzoyl]amino} propionique ;
l'acide 3-((3-benzyloxybenzyl)-{3-[2-(3-méthylquinoxalin-2-yloxy)éthoxy]benzoyl} amino}propionique ;
l'acide 3-{(3-benzyloxybenzyl)-[4-(3-benzyloxybenzyloxy)benzoyl]amino} propionique ;
l'acide 2-[4-(4-benzyloxybenzyloxy)benzoylamino]-3-(4-benzyloxyphényl)propionique ;
l'acide (2*S*)-2-[3-(4-benzyloxybenzyloxy)benzoylamino]-3-(4-benzyloxyphényl) propionique ;
l'acide 3-(4-benzyloxyphényl)-2-[3-(biphényl-4-ylméthoxy)benzoylamino] propionique ;
l'acide 2-[4-(3-benzyloxybenzyloxy)benzoylamino]-3-(4-bromophényl)propionique ;
l'acide 3-(4-benzyloxyphényl)-2-[4-(4-butylbenzyloxy)benzoylamino]propionique ;
l'acide 2-[4-(4-butylbenzyloxy)benzoylamino]-3-cyclohexylpropionique ;
l'acide {(3-benzyloxybenzyl)-[4-(4-butylbenzyloxy)benzoyl]amino}acétique ;
l'acide 3-{(3-benzyloxybenzyl)-[4-(4-butylbenzyloxy)benzoyl]amino}propionique ;
l'acide 3-(4-benzyloxyphényl)-2-[4-(2-bromobenzyloxy)benzoylamino]propionique ;
l'acide 3-(4-benzyloxyphényl)-2-[4-(2-chlorobenzyloxy)benzoylamino]propionique ;
l'acide 3-(4-benzyloxyphényl)-2-[4-(2-méthylbenzyloxy)benzoylamino]propionique ;
l'acide 3-(4-benzyloxyphényl)-2-[4-(3-trifluorométhylbenzyloxy)benzoylamino] propionique ; et
l'acide 3-(4-benzyloxyphényl)-2-[4-(2-trifluorométhylbenzyloxy)benzoylamino] propionique.

14. Composition pharmaceutique comprenant, comme ingrédient actif, une quantité thérapeutiquement efficace du composé selon l'une quelconque des revendications 1 à 13, conjointement à des quantités appropriées d'excipients pharmaceutiquement acceptables.

15. Utilisation du composé selon l'une quelconque des revendications 1 à 13, pour la fabrication d'un médicament pour le traitement prophylactique et/ou curatif de maladies chez un animal comprenant un être humain.

16. Utilisation du composé selon l'une quelconque des revendications 1 à 13, pour la fabrication d'un médicament pour le traitement prophylactique et/ou curatif de maladies induites par PPARγ chez un animal, comprenant un être humain.

17. Utilisation du composé selon l'une quelconque des revendications 1 à 13, pour la fabrication d'un médicament pour le traitement prophylactique et/ou curatif de maladies induites par PPARγ / PPARδ chez un animal, comprenant un être humain.

18. Utilisation du composé selon l'une quelconque des revendications 1 à 13, pour la fabrication d'un médicament pour le traitement prophylactique et/ou curatif d'un état associé à une maladie métabolique chez un animal, comprenant un être humain.

19. Utilisation selon la revendication 18, dans laquelle la maladie métabolique est le diabète sucré non insulinodépendant (NIDDM).

20. Utilisation selon la revendication 18, dans laquelle la maladie métabolique est l'obésité.

21. Utilisation selon la revendication 18, dans laquelle la maladie métabolique est choisie parmi l'hypercholestérolémie et autres pathologies induites par les lipides.

22. Utilisation du composé selon l'une quelconque des revendications 1 à 13, pour la fabrication d'un médicament pour le traitement prophylactique et/ou curatif d'une maladie cardiovasculaire associée au syndrome métabolique chez un animal comprenant un être humain.

23. Utilisation du composé selon l'une quelconque des revendications 1 à 13, pour la fabrication d'un médicament pour le traitement prophylactique et/ou curatif d'une inflammation ou d'un processus inflammatoire en général, chez un animal comprenant un être humain.

24. Utilisation selon la revendication 23, dans laquelle le processus inflammatoire est choisi parmi la polyarthrite rhumatoïde et l'athérosclérose.

25. Utilisation selon la revendication 23, dans laquelle le processus inflammatoire est choisi parmi le psoriasis et une maladie inflammatoire intestinale.

26. Utilisation du composé selon l'une quelconque des revendications 1 à 13, pour la fabrication d'un médicament pour le traitement prophylactique et/ou curatif d'une maladie osseuse, en particulier l'ostéoporose, chez un animal comprenant un être humain.

27. Utilisation du composé selon l'une quelconque des revendications 1 à 13, pour la fabrication d'un médicament pour le traitement prophylactique et/ou curatif du cancer chez un animal comprenant un être humain.

28. Utilisation du composé selon l'une quelconque des revendications 1 à 13, pour la fabrication d'un médicament pour le traitement prophylactique et/ou curatif de la cicatrisation d'une plaie cutanée ou de troubles cutanés associés à une différenciation anormale de cellules épidermiques, en particulier, la formation de chéloïdes, chez un animal comprenant un être humain.

29. Utilisation selon l'une quelconque des revendications 15 à 28, dans laquelle le médicament est administré par voie orale, parentérale ou topique.
